# EUROPEAN PATENT APPLICATION

(11) **EP 4 682 224 A1**
(43) Date of publication of application: **21.01.2026**
(21) Application number: 25187419.4
(22) Date of filing: 03.07.2025
(51) Int. Cl.: C09K 11/06, C07D 251/24, H10K 50/11, H10K 85/60

(54) **HETEROCYCLIC COMPOUND, ORGANIC LIGHT EMITTING DEVICE COMPRISING THE SAME AND COMPOSITION FOR ORGANIC MATERIAL LAYER**

(30) Priority: 17.07.2024 KR 20240094249; 22.05.2025 KR 20250066869
(71) Applicant: LT Materials Co., Ltd., Yongin-si, Gyeonggi-do 17118 (KR)
(72) Inventor: JEONG, Cheol Hun, 17118 Yongin-si (KR); PARK, Geon Yu, 17118 Yongin-si (KR); NO, Young Seok, 17118 Yongin-si (KR); KIM, Dong Jun, 17118 Yongin-si (KR); CHOI, Dae Hyuk, 17118 Yongin-si (KR)
(74) Representative: Meissner Bolte Partnerschaft mbB

(57) **Abstract**

The present disclosure relates to a heterocyclic compound represented by Chemical Formula 1, an organic light emitting device including the same and a composition for an organic material layer.

Each substituent of Chemical Formula 1 has the same definition as described in the description of the disclosure.

## Description

### Technical Field

This application claims priority to Korean Patent Application No. 10-2024-0094249, filed on July 17, 2024, and Korean Patent Application No. 10-2025-0066869, filed May 22, 2025, the disclosures of which are incorporated herein by reference in their entirety.

The present disclosure relates to a heterocyclic compound, an organic light emitting device including the same, and a composition for an organic material layer.

### Description of the Related Art

An organic light emitting device is one type of self-emissive display devices, and has advantages of having a wide viewing angle and a high response speed as well as having an excellent contrast.

The organic light emitting device has a structure of disposing an organic thin film between two electrodes. When a voltage is applied to the organic light emitting device having such a structure, electrons and holes injected from the two electrodes bind and pair in the organic thin film, and then light is emitted as these annihilate. The organic thin film may be formed in a single layer or a multilayer as necessary.

A material of the organic thin film may have a light emitting function as necessary. For example, as a material of the organic thin film, compounds each capable of forming a light emitting layer themselves alone may be used, or compounds each capable of serving as a host or a dopant of a host-dopant-based light emitting layer may also be used. In addition thereto, compounds capable of performing roles of hole injection, hole transport, electron blocking, hole blocking, electron transport, electron injection and the like may also be used as a material of the organic thin film.

Development of an organic thin film material has been continuously required for enhancing performance, lifetime or efficiency of an organic light emitting device.

### Prior Art Documents

### Patent Documents

(Patent Document 1) US Patent No. 4,356,429

### SUMMARY

The present disclosure is directed to providing a heterocyclic compound, an organic light emitting device including the same, and a composition for an organic material layer.

One embodiment of the present application provides a heterocyclic compound represented by the following Chemical Formula 1.

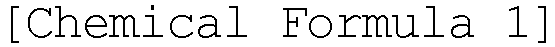

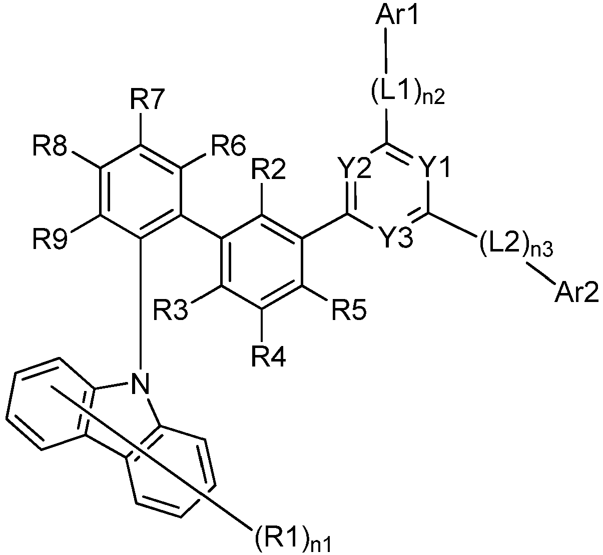

In Chemical Formula 1,
Y1 to Y3 are the same as or different from each other, and each independently CH; or N,
at least one of Y1 to Y3 is N,
Ar1 and Ar2 are the same as or different from each other, and each independently a substituted or unsubstituted C6 to C60 aryl group; or a substituted or unsubstituted C2 to C60 heteroaryl group,
L1 and L2 are the same as or different from each other, and each independently a single bond; a substituted or unsubstituted C6 to C60 arylene group; or a substituted or unsubstituted C2 to C60 heteroarylene group,
R1s are the same as or different from each other, and each independently hydrogen; deuterium; a halogen; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
R2 to R9 are the same as or different from each other, and each independently hydrogen; deuterium; a C1 to C60 alkyl group unsubstituted or substituted with deuterium; a C3 to C60 cycloalkyl group unsubstituted or substituted with deuterium; a C2 to C60 heterocycloalkyl group unsubstituted or substituted with deuterium; a C6 to C60 aryl group unsubstituted or substituted with deuterium; or a C2 to C60 heteroaryl group unsubstituted or substituted with deuterium,
at least one of R2 to R9 is deuterium or a C6 to C60 aryl group substituted with deuterium,
n1 is an integer of 0 to 8,
n2 and n3 are the same as or different from each other, and each independently an integer of 0 to 4, and
when n1, n2 and n3 are 2 or greater, R1s, L1s and L2s are the same as or different from each other.

In addition, one embodiment of the present application provides an organic light emitting device, wherein an organic material layer including the heterocyclic compound represented by Chemical Formula 1 further includes a heterocyclic compound represented by the following Chemical Formula 2 or Chemical Formula 3.

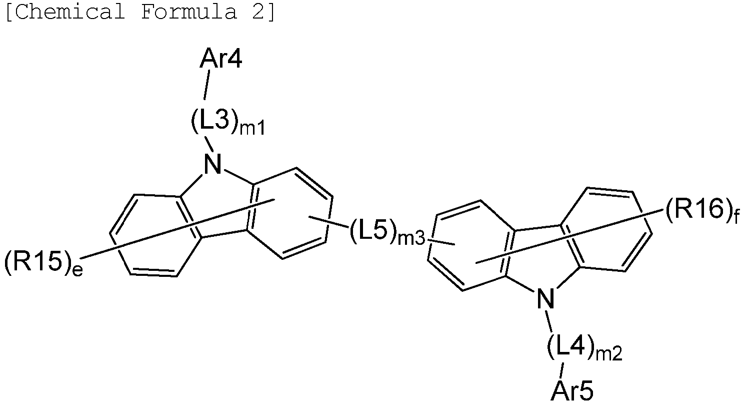

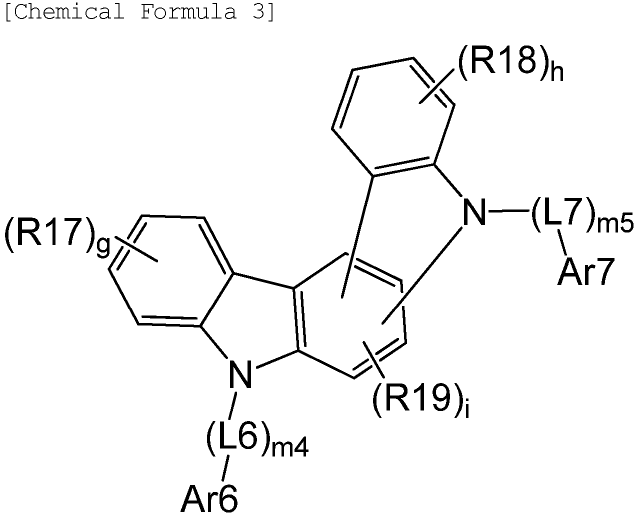

In Chemical Formula 2 and Chemical Formula 3,
L3 to L7 are the same as or different from each other, and each independently a single bond; a substituted or unsubstituted C6 to C60 arylene group; or a substituted or unsubstituted C2 to C60 heteroarylene group,
Ar4 to Ar7 are the same as or different from each other, and each independently a substituted or unsubstituted C6 to C60 aryl group; or a substituted or unsubstituted C2 to C60 heteroaryl group,
R15 to R19 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
m1 to m5 are the same as or different from each other, and each independently an integer of 0 to 3,
e and f are the same as or different from each other, and each independently an integer of 0 to 7,
g and h are the same as or different from each other, and each independently an integer of 0 to 4,
i is an integer of 0 to 2, and
when m1 to m5, e, f, g, h, and i are 2 or greater, L3s to L7s and R15s to R19s are the same as or different from each other.

In addition, another embodiment of the present application provides a composition for an organic material layer, the composition including: the heterocyclic compound represented by Chemical Formula 1; and the heterocyclic compound represented by Chemical Formula 2 or Chemical Formula 3.

The heterocyclic compound according to one embodiment can be used as an organic material layer material of an organic light emitting device. The compound is capable of performing roles of a hole injection layer material, a hole transport layer material, a light emitting layer material, an electron transport layer material, an electron injection layer material and the like in an organic light emitting device. Particularly, the compound can be used as a light emitting layer material of an organic light emitting device. The compound can be used alone as a light emitting material, or can be used as a host material or a dopant material of a light emitting layer.

When the heterocyclic compound represented by Chemical Formula 1 is used in an organic material layer, it is possible to lower a driving voltage of an organic light emitting device, and improve light emission efficiency and lifetime properties thereof.

### BRIEF DESCRIPTION OF THE DRAWINGS

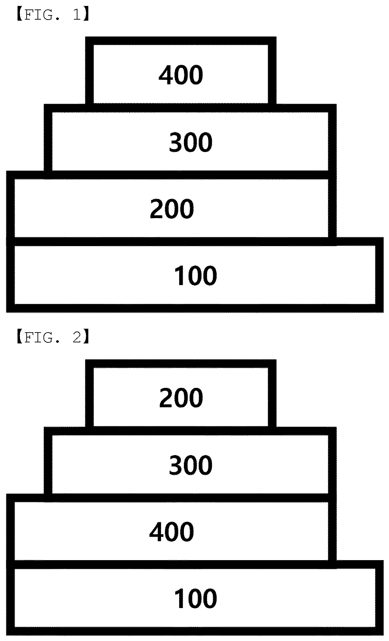

FIGS. 1 to 3 are diagrams each schematically illustrating a lamination structure of an organic light emitting device according to one embodiment of the present disclosure.

### DESCRIPTION OF SPECIFIC EMBODIMENTS

Hereinafter, the present disclosure will be described in more detail.

In the present specification, a description of a certain part "including" certain components means that it may further include other components, and does not exclude other components unless particularly stated on the contrary.

In the present specification, a term "substitution" means that a hydrogen atom bonding to a carbon atom of a compound is changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 linear or branched alkyl group; a C2 to C60 linear or branched alkenyl group; a C2 to C60 linear or branched alkynyl group; a C3 to C60 monocyclic or polycyclic cycloalkyl group; a C2 to C60 monocyclic or polycyclic heterocycloalkyl group; a C6 to C60 monocyclic or polycyclic aryl group; a C2 to C60 monocyclic or polycyclic heteroaryl group; - SiRR'R"; -P(=O)RR'; a C1 to C20 alkylamine group; a C6 to C60 monocyclic or polycyclic arylamine group; and a C2 to C60 monocyclic or polycyclic heteroarylamine group or being unsubstituted, or being substituted with a substituent in which two or more substituents selected from among the substituents exemplified above are linked or being unsubstituted.

In the present specification, "unsubstituted or substituted with deuterium" means that at least one substituted position is substituted with deuterium. All substituted positions are not necessarily substituted with deuterium, and it is sufficient that at least one of the substituted positions is substituted with deuterium, and the position not substituted with deuterium is unsubstituted.

R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In the present specification, the halogen may be fluorine, chlorine, bromine or iodine.

In the present specification, the alkyl group includes a linear or branched form having 1 to 60 carbon atoms, and may be further substituted with other substituents. The number of carbon atoms of the alkyl group may be from 1 to 60, specifically from 1 to 40 and more specifically from 1 to 20. Specific examples of the alkyl group may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 4-methylhexyl group, a 5-methylhexyl group and the like, but are not limited thereto.

In the present specification, the alkenyl group includes a linear or branched form having 2 to 60 carbon atoms, and may be further substituted with other substituents. The number of carbon atoms of the alkenyl group may be from 2 to 60, specifically from 2 to 40 and more specifically from 2 to 20. Specific examples of the alkenyl group may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes a linear or branched form having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples of the alkoxy group may include a methoxy group, an ethoxy group, an n-propoxy group, an isopropoxy group, an n-butoxy group, an isobutoxy group, a tert-butoxy group, a sec-butoxy group, an n-pentyloxy group, a neopentyloxy group, an isopentyloxy group, an n-hexyloxy group, a 3,3-dimethylbutyloxy group, a 2-ethylbutyloxy group, an n-octyloxy group, an n-nonyloxy group, an n-decyloxy group, a benzyloxy group, a p-methylbenzyloxy group and the like, but are not limited thereto.

In the present specification, the cycloalkyl group includes a monocyclic or polycyclic group having 3 to 60 carbon atoms, and may be further substituted with other substituents. Herein, the polycyclic group means a group in which the cycloalkyl group is directly linked to or fused with another cyclic group. Herein, the another cyclic group may be a cycloalkyl group, but may also be different types of cyclic groups such as a heterocycloalkyl group, an aryl group and a heteroaryl group. The number of carbon atoms of the cycloalkyl group may be from 3 to 60, specifically from 3 to 40 and more specifically from 5 to 20. Specific examples of the cycloalkyl group may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes a monocyclic or polycyclic group having 2 to 60 carbon atoms, and may be further substituted with other substituents. Herein, the polycyclic group means a group in which the heterocycloalkyl group is directly linked to or fused with another cyclic group. Herein, the another cyclic group may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes a monocyclic or polycyclic group having 6 to 60 carbon atoms, and may be further substituted with other substituents. Herein, the polycyclic group means a group in which the aryl group is directly linked to or fused with another cyclic group. Herein, the another cyclic group may be an aryl group, but may also be different types of cyclic groups such as a cycloalkyl group, a heterocycloalkyl group and a heteroaryl group. The aryl group may include a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be applied. Examples of the phosphine oxide group may include a diphenylphosphine oxide group, a dinaphthylphosphine oxide group and the like, but are not limited thereto.

In the present specification, the silyl group is a substituent including Si and having the Si atom directly linked as a radical, and is represented by -SiR101R102R103. R101 to R103 are the same as or different from each other, and may be each independently a substituent formed with at least one of hydrogen; deuterium; a halogen; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

When the fluorenyl group is substituted, the following structural formulae and the like may be included, however, the structure is not limited thereto.

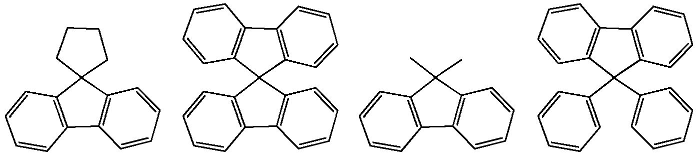

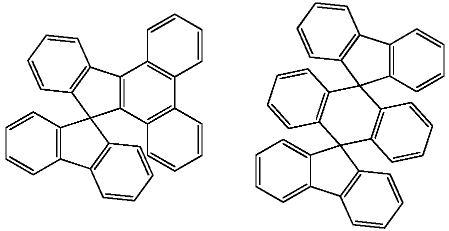

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

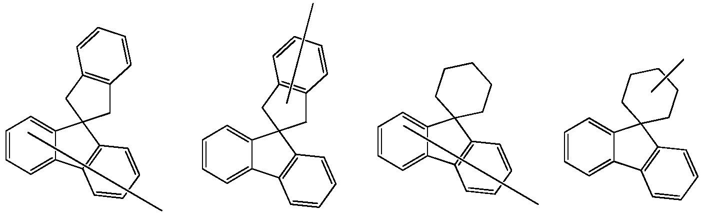

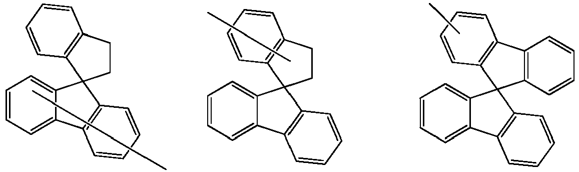

In the present specification, the heteroaryl group includes S, O, Se, N or Si as a heteroatom, includes a monocyclic or polycyclic group having 2 to 60 carbon atoms, and may be further substituted with other substituents. Herein, the polycyclic group means a group in which the heteroaryl group is directly linked to or fused with another cyclic group. Herein, the another cyclic group may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophenyl group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolinyl group, a naphthyridyl group, an acridinyl group, a phenanthridinyl group, an imidazopyridinyl group, a diazanaphthalenyl group, a triazaindenyl group, a 2-indolyl group, an indolizinyl group, a benzothiazolyl group, a benzoxazolyl group, a benzimidazolyl group, a benzothiophenyl group, a benzofuranyl group, a dibenzothiophenyl group, a dibenzofuranyl group, a carbazolyl group, a benzocarbazolyl group, a dibenzocarbazolyl group, a phenazinyl group, a dibenzosilole group, a spirobi(dibenzosilole) group, a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepinyl group, a 9,10-dihydroacridinyl group, a phenanthrazinyl group, a phenothiazinyl group, a phthalazinyl group, a naphthylidinyl group, a phenanthrolinyl group, a benzo[c] [1,2,5]thiadiazolyl group, a 5,10-dihydrodibenzo[b,e] [1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms is not particularly limited, but preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting at ortho positions in a benzene ring, and two substituents substituting at the same carbon in an aliphatic ring may be interpreted as groups "adjacent" to each other.

In the present disclosure, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present disclosure, a "case of a substituent being not indicated in a chemical formula or compound structure" may mean that positions to which substituents may come are all hydrogen or deuterium. In other words, since deuterium is an isotope of hydrogen, some hydrogen atoms may be deuterium that is an isotope, and herein, a content of the deuterium may be from 0% to 100%.

In one embodiment of the present disclosure, in a "case of a substituent being not indicated in a chemical formula or compound structure", hydrogen and deuterium may be used interchangeably in compounds when deuterium is not explicitly excluded such as "a deuterium content being 0%", "a hydrogen content being 100%" or "substituents being all hydrogen".

In one embodiment of the present disclosure, deuterium is one of isotopes of hydrogen, is an element having deuteron formed with one proton and one neutron as a nucleus, and may be expressed as hydrogen-2, and the elemental symbol thereof may also be written as D or ²H.

In one embodiment of the present disclosure, an isotope means an atom with the same atomic number (Z) but with a different mass number (A), and may also be interpreted as an element with the same number of protons but with a different number of neutrons.

In one embodiment of the present disclosure, a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 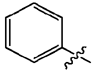 may mean that the total number of substituents that the phenyl group may have is 5 (T1 in the formula), and the number of deuterium atoms among these is 1 (T2 in the formula). In other words, having a deuterium content of 20% in a phenyl group may be represented by the following structural formulae.

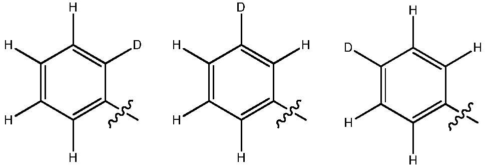

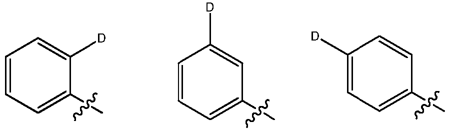

In addition, in one embodiment of the present disclosure, "a phenyl group having a deuterium content of 0%" may mean a phenyl group that does not include a deuterium atom, that is, a phenyl group that has 5 hydrogen atoms.

In the present disclosure, the C6 to C60 aromatic hydrocarbon ring means a compound including an aromatic ring formed with C6 to C60 carbons and hydrogens. Examples thereof may include benzene, biphenyl, terphenyl, triphenylene, naphthalene, anthracene, phenalene, phenanthrene, fluorene, pyrene, chrysene, perylene, azulene and the like, but are not limited thereto, and include all aromatic hydrocarbon ring compounds known in the art and satisfying the above-mentioned number of carbon atoms.

One embodiment of the present application provides a heterocyclic compound represented by the following Chemical Formula 1.

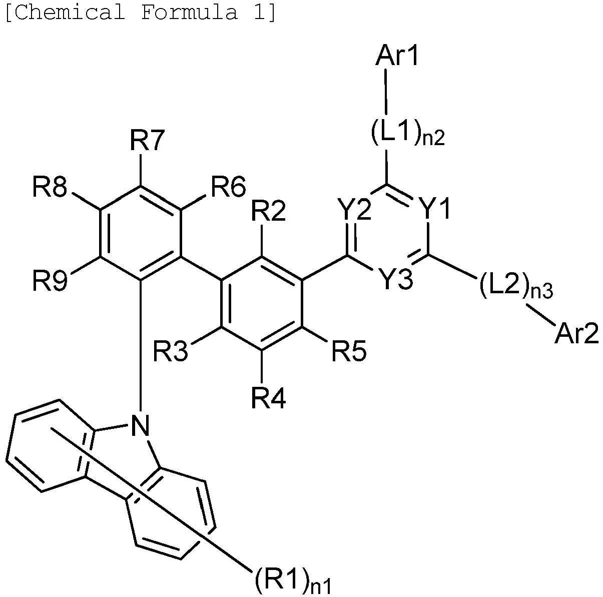

In Chemical Formula 1,
Y1 to Y3 are the same as or different from each other, and each independently CH; or N,
at least one of Y1 to Y3 is N,
Ar1 and Ar2 are the same as or different from each other, and each independently a substituted or unsubstituted C6 to C60 aryl group; or a substituted or unsubstituted C2 to C60 heteroaryl group,
L1 and L2 are the same as or different from each other, and each independently a single bond; a substituted or unsubstituted C6 to C60 arylene group; or a substituted or unsubstituted C2 to C60 heteroarylene group,
R1s are the same as or different from each other, and each independently hydrogen; deuterium; a halogen; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
R2 to R9 are the same as or different from each other, and each independently hydrogen; deuterium; a C1 to C60 alkyl group unsubstituted or substituted with deuterium; a C3 to C60 cycloalkyl group unsubstituted or substituted with deuterium; a C2 to C60 heterocycloalkyl group unsubstituted or substituted with deuterium; a C6 to C60 aryl group unsubstituted or substituted with deuterium; or a C2 to C60 heteroaryl group unsubstituted or substituted with deuterium,
at least one of R2 to R9 is deuterium or a C6 to C60 aryl group substituted with deuterium,
n1 is an integer of 0 to 8,
n2 and n3 are the same as or different from each other, and each independently an integer of 0 to 4, and
when n1, n2 and n3 are 2 or greater, R1s, L1s and L2s are the same as or different from each other.

In one embodiment of the present application, Y1 is N, and Y2 and Y3 may be CH.

In another embodiment, Y1 and Y2 are N, and Y3 may be CH.

In another embodiment, Y1 and Y3 are N, and Y2 may be CH.

In another embodiment, Y1 is CH, and Y2 and Y3 may be N.

In another embodiment, Y1 and Y2 are CH, and Y3 may be N.

In another embodiment, Y1 and Y3 are CH, and Y2 may be N.

In another embodiment, Y1 to Y3 may be N.

In one embodiment of the present application, Ar1 and Ar2 are the same as or different from each other, and may be each independently a substituted or unsubstituted C6 to C40 aryl group or a substituted or unsubstituted C2 to C40 heteroaryl group.

In another embodiment, Ar1 and Ar2 are the same as or different from each other, and may be each independently a substituted or unsubstituted C6 to C30 aryl group or a substituted or unsubstituted C2 to C30 heteroaryl group.

In another embodiment, Ar1 and Ar2 are the same as or different from each other, and may be each independently a substituted or unsubstituted C6 to C20 aryl group or a substituted or unsubstituted C2 to C20 heteroaryl group.

In another embodiment, Ar1 and Ar2 are the same as or different from each other, and may be each independently a substituted or unsubstituted C6 to C10 aryl group or a substituted or unsubstituted C2 to C10 heteroaryl group.

In another embodiment, Ar1 and Ar2 are the same as or different from each other, and may be each independently a substituted or unsubstituted phenyl group; a substituted or unsubstituted biphenyl group; a substituted or unsubstituted naphthyl group; a substituted or unsubstituted terphenyl group; a substituted or unsubstituted fluorenyl group; a substituted or unsubstituted carbazoyl group; a substituted or unsubstituted triphenyl group; a substituted or unsubstituted dibenzofuranyl group; or a substituted or unsubstituted dibenzothiophenyl group.

In another embodiment, Ar1 and Ar2 are the same as or different from each other, and each independently a substituted or unsubstituted phenyl group; a substituted or unsubstituted biphenyl group; a substituted or unsubstituted terphenyl group; a substituted or unsubstituted carbazoyl group; a substituted or unsubstituted dibenzofuranyl group; or a substituted or unsubstituted dibenzothiophenyl group.

In another embodiment, Ar1 and Ar2 are the same as or different from each other, and may not include deuterium.

In one embodiment of the present application, L1 and L2 are the same as or different from each other, and may be each independently a single bond; a substituted or unsubstituted C6 to C40 arylene group; or a substituted or unsubstituted C2 to C40 heteroarylene group.

In another embodiment, L1 and L2 are the same as or different from each other, and may be each independently a single bond; a substituted or unsubstituted C6 to C30 arylene group; or a substituted or unsubstituted C2 to C30 heteroarylene group.

In another embodiment, L1 and L2 are the same as or different from each other, and may be each independently a single bond; a substituted or unsubstituted C6 to C20 arylene group; or a substituted or unsubstituted C2 to C20 heteroarylene group.

In another embodiment, L1 and L2 are the same as or different from each other, and may be each independently a single bond; a substituted or unsubstituted C6 to C10 arylene group; or a substituted or unsubstituted C2 to C10 heteroarylene group.

In another embodiment, L1 and L2 are the same as or different from each other, and may be each independently a single bond; a substituted or unsubstituted phenylene group; a substituted or unsubstituted biphenylene group; a substituted or unsubstituted naphthylene group; a substituted or unsubstituted terphenylene group; a substituted or unsubstituted fluorenylene group; a substituted or unsubstituted triphenylene group; a substituted or unsubstituted dibenzofuranylene group; a substituted or unsubstituted carbazoylene group; or a substituted or unsubstituted dibenzothiophenylene group.

In another embodiment, L1 and L2 are the same as or different from each other, and may be each independently a single bond; a substituted or unsubstituted phenylene group; a substituted or unsubstituted biphenylene group; a substituted or unsubstituted terphenylene group; a substituted or unsubstituted dibenzofuranylene group; or a substituted or unsubstituted dibenzothiophenylene group.

In another embodiment, L1 and L2 are the same as or different from each other, and may each not include deuterium.

In one embodiment of the present application, the portion corresponding to 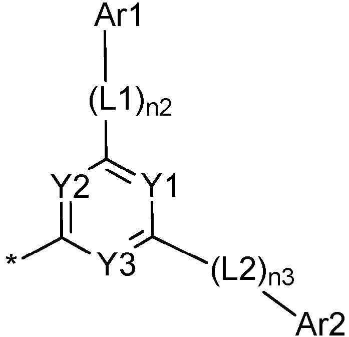 of Chemical Formula 1 may have a deuterium content of 100% or less based on the total number of hydrogen atoms and deuterium atoms, and may have a deuterium content of, for example, 100% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, 4% or less, 3% or less, 2% or less or 1% or less.
* means a site linked to Chemical Formula 1, and
Y1 to Y3, Ar1, Ar2, L1, L2, n2 and n3 have the same definitions as in Chemical Formula 1.

In another embodiment, the portion corresponding to 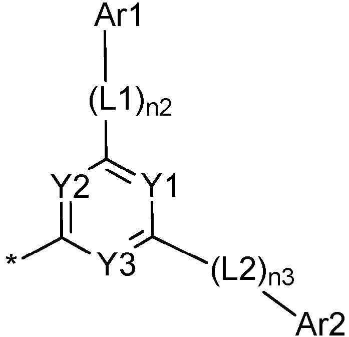 of Chemical Formula 1 may have a deuterium content of 0%.

In one embodiment of the present application, R1s are the same as or different from each other, and may be each independently hydrogen; deuterium; a halogen; a cyano group; a substituted or unsubstituted C1 to C40 alkyl group; a substituted or unsubstituted C3 to C40 cycloalkyl group; a substituted or unsubstituted C2 to C40 heterocycloalkyl group; a substituted or unsubstituted C6 to C40 aryl group; or a substituted or unsubstituted C2 to C40 heteroaryl group.

In another embodiment, R1s are the same as or different from each other, and may be each independently hydrogen; deuterium; a halogen; a cyano group; a substituted or unsubstituted C1 to C30 alkyl group; a substituted or unsubstituted C3 to C30 cycloalkyl group; a substituted or unsubstituted C2 to C30 heterocycloalkyl group; a substituted or unsubstituted C6 to C30 aryl group; or a substituted or unsubstituted C2 to C30 heteroaryl group.

In another embodiment, R1s are the same as or different from each other, and may be each independently hydrogen; deuterium; a halogen; a cyano group; a substituted or unsubstituted C1 to C20 alkyl group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In another embodiment, R1s are the same as or different from each other, and may be each independently hydrogen; deuterium; a halogen; a cyano group; a substituted or unsubstituted C1 to C10 alkyl group; a substituted or unsubstituted C3 to C10 cycloalkyl group; a substituted or unsubstituted C2 to C10 heterocycloalkyl group; a substituted or unsubstituted C6 to C10 aryl group; or a substituted or unsubstituted C2 to C10 heteroaryl group.

In another embodiment, R1s are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, R1s are the same as or different from each other, and may be each independently hydrogen; deuterium; a substituted or unsubstituted C1 to C40 alkyl group; a substituted or unsubstituted C6 to C40 aryl group; or a substituted or unsubstituted C2 to C40 heteroaryl group.

In another embodiment, R1s are the same as or different from each other, and may be each independently hydrogen; deuterium; a substituted or unsubstituted C1 to C30 alkyl group; a substituted or unsubstituted C6 to C30 aryl group; or a substituted or unsubstituted C2 to C30 heteroaryl group.

In another embodiment, R1s are the same as or different from each other, and may be each independently hydrogen; deuterium; a substituted or unsubstituted C1 to C20 alkyl group; a substituted or unsubstituted C6 to C20 aryl group; or a substituted or unsubstituted C2 to C20 heteroaryl group.

In another embodiment, R1s are the same as or different from each other, and may be each independently hydrogen; deuterium; a substituted or unsubstituted C1 to C10 alkyl group; a substituted or unsubstituted C6 to C10 aryl group; or a substituted or unsubstituted C2 to C10 heteroaryl group.

In another embodiment, R1s are the same as or different from each other, and may be each independently hydrogen; deuterium; a substituted or unsubstituted methyl group; a substituted or unsubstituted butyl group; a substituted or unsubstituted tert-butyl group; a substituted or unsubstituted phenyl group; a substituted or unsubstituted biphenyl group; a substituted or unsubstituted carbazoyl group; a substituted or unsubstituted dibenzofuranyl group; or a substituted or unsubstituted dibenzothiophenyl group.

In one embodiment of the present application, R2 to R9 are the same as or different from each other, and may be each independently hydrogen; deuterium; a C1 to C40 alkyl group unsubstituted or substituted with deuterium; a C3 to C40 cycloalkyl group unsubstituted or substituted with deuterium; a C2 to C40 heterocycloalkyl group unsubstituted or substituted with deuterium; a C6 to C40 aryl group unsubstituted or substituted with deuterium; or a C2 to C40 heteroaryl group unsubstituted or substituted with deuterium.

In another embodiment, R2 to R9 are the same as or different from each other, and may be each independently hydrogen; deuterium; a C1 to C30 alkyl group unsubstituted or substituted with deuterium; a C3 to C30 cycloalkyl group unsubstituted or substituted with deuterium; a C2 to C30 heterocycloalkyl group unsubstituted or substituted with deuterium; a C6 to C30 aryl group unsubstituted or substituted with deuterium; or a C2 to C30 heteroaryl group unsubstituted or substituted with deuterium.

In another embodiment, R2 to R9 are the same as or different from each other, and may be each independently hydrogen; deuterium; a C1 to C20 alkyl group unsubstituted or substituted with deuterium; a C3 to C20 cycloalkyl group unsubstituted or substituted with deuterium; a C2 to C20 heterocycloalkyl group unsubstituted or substituted with deuterium; a C6 to C20 aryl group unsubstituted or substituted with deuterium; or a C2 to C20 heteroaryl group unsubstituted or substituted with deuterium.

In another embodiment, R2 to R9 are the same as or different from each other, and may be each independently hydrogen; deuterium; a C1 to C10 alkyl group unsubstituted or substituted with deuterium; a C3 to C10 cycloalkyl group unsubstituted or substituted with deuterium; a C2 to C10 heterocycloalkyl group unsubstituted or substituted with deuterium; a C6 to C10 aryl group unsubstituted or substituted with deuterium; or a C2 to C10 heteroaryl group unsubstituted or substituted with deuterium.

In another embodiment, R2 to R9 are the same as or different from each other, and may be each independently hydrogen; deuterium; or a phenyl group unsubstituted or substituted with deuterium.

In one embodiment of the present disclosure, at least one of R2 to R9 may be deuterium; or a C6 to C60 aryl group substituted with deuterium.

In another embodiment, at least two of R2 to R9 may be deuterium; or a C6 to C60 aryl group substituted with deuterium.

In another embodiment, at least three of R2 to R9 may be deuterium; or a C6 to C60 aryl group substituted with deuterium.

In another embodiment, at least four of R2 to R9 may be deuterium; or a C6 to C60 aryl group substituted with deuterium.

In another embodiment, at least five of R2 to R9 may be deuterium; or a C6 to C60 aryl group substituted with deuterium.

In another embodiment, at least six of R2 to R9 may be deuterium; or a C6 to C60 aryl group substituted with deuterium.

In another embodiment, at least seven of R2 to R9 may be deuterium; or a C6 to C60 aryl group substituted with deuterium.

In another embodiment, R2 to R9 may be deuterium; or a C6 to C60 aryl group substituted with deuterium.

In one embodiment of the present disclosure, at least one of R2 to R9 may be deuterium; or a C6 to C40 aryl group substituted with deuterium.

In another embodiment, at least two of R2 to R9 may be deuterium; or a C6 to C40 aryl group substituted with deuterium.

In another embodiment, at least three of R2 to R9 may be deuterium; or a C6 to C40 aryl group substituted with deuterium.

In another embodiment, at least four of R2 to R9 may be deuterium; or a C6 to C40 aryl group substituted with deuterium.

In another embodiment, at least five of R2 to R9 may be deuterium; or a C6 to C40 aryl group substituted with deuterium.

In another embodiment, at least six of R2 to R9 may be deuterium; or a C6 to C40 aryl group substituted with deuterium.

In another embodiment, at least seven of R2 to R9 may be deuterium; or a C6 to C40 aryl group substituted with deuterium.

In another embodiment, R2 to R9 may be deuterium; or a C6 to C40 aryl group substituted with deuterium.

In one embodiment of the present disclosure, at least one of R2 to R9 may be deuterium; or a C6 to C30 aryl group substituted with deuterium.

In another embodiment, at least two of R2 to R9 may be deuterium; or a C6 to C30 aryl group substituted with deuterium.

In another embodiment, at least three of R2 to R9 may be deuterium; or a C6 to C30 aryl group substituted with deuterium.

In another embodiment, at least four of R2 to R9 may be deuterium; or a C6 to C30 aryl group substituted with deuterium.

In another embodiment, at least five of R2 to R9 may be deuterium; or a C6 to C30 aryl group substituted with deuterium.

In another embodiment, at least six of R2 to R9 may be deuterium; or a C6 to C30 aryl group substituted with deuterium.

In another embodiment, at least seven of R2 to R9 may be deuterium; or a C6 to C30 aryl group substituted with deuterium.

In another embodiment, R2 to R9 may be deuterium; or a C6 to C30 aryl group substituted with deuterium.

In one embodiment of the present disclosure, at least one of R2 to R9 may be deuterium; or a C6 to C20 aryl group substituted with deuterium.

In another embodiment, at least two of R2 to R9 may be deuterium; or a C6 to C20 aryl group substituted with deuterium.

In another embodiment, at least three of R2 to R9 may be deuterium; or a C6 to C20 aryl group substituted with deuterium.

In another embodiment, at least four of R2 to R9 may be deuterium; or a C6 to C20 aryl group substituted with deuterium.

In another embodiment, at least five of R2 to R9 may be deuterium; or a C6 to C20 aryl group substituted with deuterium.

In another embodiment, at least six of R2 to R9 may be deuterium; or a C6 to C20 aryl group substituted with deuterium.

In another embodiment, at least seven of R2 to R9 may be deuterium; or a C6 to C20 aryl group substituted with deuterium.

In another embodiment, R2 to R9 may be deuterium; or a C6 to C20 aryl group substituted with deuterium.

In one embodiment of the present disclosure, at least one of R2 to R9 may be deuterium; or a C6 to C10 aryl group substituted with deuterium.

In another embodiment, at least two of R2 to R9 may be deuterium; or a C6 to C10 aryl group substituted with deuterium.

In another embodiment, at least three of R2 to R9 may be deuterium; or a C6 to C10 aryl group substituted with deuterium.

In another embodiment, at least four of R2 to R9 may be deuterium; or a C6 to C10 aryl group substituted with deuterium.

In another embodiment, at least five of R2 to R9 may be deuterium; or a C6 to C10 aryl group substituted with deuterium.

In another embodiment, at least six of R2 to R9 may be deuterium; or a C6 to C10 aryl group substituted with deuterium.

In another embodiment, at least seven of R2 to R9 may be deuterium; or a C6 to C10 aryl group substituted with deuterium.

In another embodiment, R2 to R9 may be deuterium; or a C6 to C10 aryl group substituted with deuterium.

In one embodiment of the present disclosure, at least one of R2 to R9 may be deuterium; or a phenyl group substituted with deuterium.

In another embodiment, at least two of R2 to R9 may be deuterium; or a phenyl group substituted with deuterium.

In another embodiment, at least three of R2 to R9 may be deuterium; or a phenyl group substituted with deuterium.

In another embodiment, at least four of R2 to R9 may be deuterium; or a phenyl group substituted with deuterium.

In another embodiment, at least five of R2 to R9 may be deuterium; or a phenyl group substituted with deuterium.

In another embodiment, at least six of R2 to R9 may be deuterium; or a phenyl group substituted with deuterium.

In another embodiment, at least seven of R2 to R9 may be deuterium; or a phenyl group substituted with deuterium.

In another embodiment, R2 to R9 may be deuterium; or a phenyl group substituted with deuterium.

In one embodiment of the present disclosure, at least one of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least two of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least three of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least four of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least five of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least six of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, at least seven of R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In another embodiment, R2 to R9 may be deuterium; a phenyl group substituted with one or more deuterium atoms; a phenyl group substituted with two or more deuterium atoms; a phenyl group substituted with three or more deuterium atoms; a phenyl group substituted with four or more deuterium atoms; or a phenyl group all substituted with deuterium atoms.

In one embodiment of the present disclosure, at least one of R2 to R9 may be deuterium.

In another embodiment, at least two of R2 to R9 may be deuterium.

In another embodiment, at least three of R2 to R9 may be deuterium.

In another embodiment, at least four of R2 to R9 may be deuterium.

In another embodiment, at least five of R2 to R9 may be deuterium.

In another embodiment, at least six of R2 to R9 may be deuterium.

In another embodiment, at least seven of R2 to R9 may be deuterium.

In another embodiment, R2 to R9 may be deuterium.

In one embodiment of the present application, the portion corresponding to 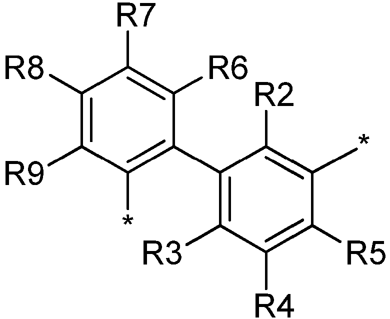 of Chemical Formula 1 may have a deuterium content of 1% to 100% based on the total number of hydrogen atoms and deuterium atoms, and may have a deuterium content of, for example, 1% or greater, 10% or greater, 12.75% or greater, 20% or greater, 25% or greater, 30% or greater, 37.5% or greater, 40% or greater or 50% or greater, and 100% or less, 95% or less, 90% or less, 87.5% or less, 85% or less, 80% or less, 75% or less, 70% or less, 65% or less, 62% or less, 60% or less or 55% or less.
* means a site linked to Chemical Formula 1, and
R2 to R9 have the same definitions as in Chemical Formula 1.

In one embodiment of the present application, Ar1 and Ar2 of Chemical Formula 1 may be represented by any one of the following Chemical Formula 1-1 to Chemical Formula 1-3.

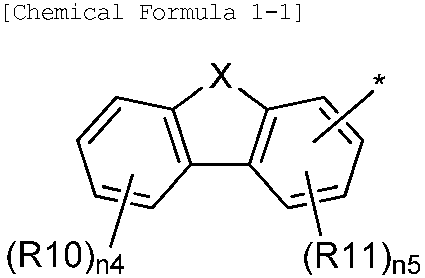

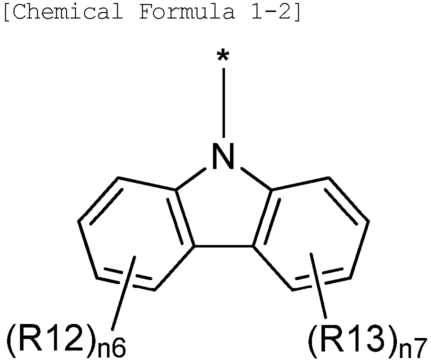

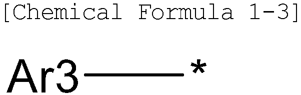

In Chemical Formula 1-1 to Chemical Formula 1-3,
X is O or S,
Ar3 is a substituted or unsubstituted C6 to C60 aryl group,
R10 to R13 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring; or a substituted or unsubstituted C2 to C60 heteroring,
* is a site linked to Chemical Formula 1,
n4, n6 and n7 are the same as or different from each other, and each independently an integer of 0 to 4,
n5 is an integer of 0 to 3, and
when n4 to n7 are 2 or greater, R10s to R13s are the same as or different from each other.

In one embodiment of the present application, X may be O.

In another embodiment, X may be S.

In one embodiment of the present application, R10 to R13 are the same as or different from each other, and each independently selected from the group consisting of hydrogen; deuterium; halogen; a cyano group; a substituted or unsubstituted C1 to C40 alkyl group; a substituted or unsubstituted C2 to C40 alkenyl group; a substituted or unsubstituted C2 to C40 alkynyl group; a substituted or unsubstituted C1 to C40 alkoxy group; a substituted or unsubstituted C3 to C40 cycloalkyl group; a substituted or unsubstituted C2 to C40 heterocycloalkyl group; a substituted or unsubstituted C6 to C40 aryl group; and a substituted or unsubstituted C2 to C40 heteroaryl group, or two or more groups adjacent to each other may bond to each other to form a substituted or unsubstituted C6 to C40 aromatic hydrocarbon ring; or a substituted or unsubstituted C2 to C40 heteroring.

In another embodiment, R10 to R13 are the same as or different from each other, and each independently selected from the group consisting of hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; and a substituted or unsubstituted C2 to C30 heteroaryl group, or two or more groups adjacent to each other may bond to each other to form a substituted or unsubstituted C6 to C30 aromatic hydrocarbon ring; or a substituted or unsubstituted C2 to C30 heteroring.

In another embodiment, R10 to R13 are the same as or different from each other, and each independently selected from the group consisting of hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; and a substituted or unsubstituted C2 to C20 heteroaryl group, or two or more groups adjacent to each other may bond to each other to form a substituted or unsubstituted C6 to C20 aromatic hydrocarbon ring; or a substituted or unsubstituted C2 to C20 heteroring.

In another embodiment, R10 to R13 are the same as or different from each other, and may be each independently hydrogen; deuterium; a substituted or unsubstituted C6 to C20 aryl group; or a substituted or unsubstituted C2 to C20 heteroaryl group.

In another embodiment, R10 to R13 are the same as or different from each other, and may be each independently hydrogen; deuterium; a substituted or unsubstituted C6 to C10 aryl group; or a substituted or unsubstituted C2 to C10 heteroaryl group.

In another embodiment, R10 to R13 are the same as or different from each other, and may be each independently hydrogen; deuterium; or a substituted or unsubstituted C6 to C10 aryl group.

In another embodiment, R10 to R13 are the same as or different from each other, and may be each independently hydrogen; or a substituted or unsubstituted C6 to C10 aryl group.

In another embodiment, R10 to R13 are the same as or different from each other, and may be each independently hydrogen; a substituted or unsubstituted phenyl group; a substituted or unsubstituted biphenyl group; a substituted or unsubstituted naphthyl group; a substituted or unsubstituted terphenyl group; a substituted or unsubstituted fluorenyl group; or a substituted or unsubstituted triphenyl group.

In another embodiment, R10 to R13 are the same as or different from each other, and may be each independently hydrogen; a substituted or unsubstituted phenyl group; a substituted or unsubstituted biphenyl group; or a substituted or unsubstituted naphthyl group.

In another embodiment, R10 to R13 are the same as or different from each other, and may be each independently hydrogen; a substituted or unsubstituted phenyl group; or a substituted or unsubstituted biphenyl group.

In one embodiment of the present application, Ar3 may be a substituted or unsubstituted C6 to C40 aryl group.

In another embodiment, Ar3 may be a substituted or unsubstituted C6 to C30 aryl group.

In another embodiment, Ar3 may be a substituted or unsubstituted C6 to C20 aryl group.

In another embodiment, Ar3 may be a substituted or unsubstituted C6 to C10 aryl group.

In another embodiment, Ar3 may be a substituted or unsubstituted phenyl group; a substituted or unsubstituted biphenyl group; a substituted or unsubstituted naphthyl group; a substituted or unsubstituted terphenyl group; a substituted or unsubstituted fluorenyl group; or a substituted or unsubstituted triphenyl group.

In another embodiment, Ar3 may be a substituted or unsubstituted phenyl group; a substituted or unsubstituted biphenyl group; or a substituted or unsubstituted terphenyl group.

In one embodiment of the present application, when Ar1 or Ar2 of Chemical Formula 1 is represented by Chemical Formula 1-2, L1 and L2 of Chemical Formula 1 are the same as or different from each other, and may be each independently a substituted or unsubstituted C6 to C60 arylene group; or a substituted or unsubstituted C2 to C60 heteroarylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted C6 to C40 arylene group; or a substituted or unsubstituted C2 to C40 heteroarylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted C6 to C30 arylene group; or a substituted or unsubstituted C2 to C30 heteroarylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted C6 to C20 arylene group; or a substituted or unsubstituted C2 to C20 heteroarylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted C6 to C10 arylene group; or a substituted or unsubstituted C2 to C10 heteroarylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted phenylene group; a substituted or unsubstituted biphenylene group; a substituted or unsubstituted naphthylene group; a substituted or unsubstituted terphenylene group; a substituted or unsubstituted fluorenylene group; or a substituted or unsubstituted triphenylene group.

In another embodiment, when Ar1 or Ar2 of Chemical Formula 1 is represented by Chemical Formula 1-2, L1 and L2 of Chemical Formula 1 are the same as or different from each other, and may be each independently a substituted or unsubstituted phenylene group; or a substituted or unsubstituted biphenylene group.

In one embodiment of the present application, the heterocyclic compound represented by Chemical Formula 1 may have a deuterium content of 1% to 100% based on the total number of hydrogen atoms and deuterium atoms.

In one embodiment of the present application, the heterocyclic compound represented by Chemical Formula 1 may have a deuterium content of 10% to 100% based on the total number of hydrogen atoms and deuterium atoms.

In one embodiment of the present application, the heterocyclic compound represented by Chemical Formula 1 may have a deuterium content of 20% to 90% based on the total number of hydrogen atoms and deuterium atoms.

In one embodiment of the present application, the heterocyclic compound represented by Chemical Formula 1 may have a deuterium content of 30% to 80% based on the total number of hydrogen atoms and deuterium atoms.

In one embodiment of the present application, the heterocyclic compound represented by Chemical Formula 1 may have a deuterium content of 40% to 70% based on the total number of hydrogen atoms and deuterium atoms.

In one embodiment of the present application, the heterocyclic compound represented by Chemical Formula 1 may have a deuterium content of 40% to 60% based on the total number of hydrogen atoms and deuterium atoms.

In one embodiment of the present application, the heterocyclic compound represented by Chemical Formula 1 may have a deuterium content of 40% to 50% based on the total number of hydrogen atoms and deuterium atoms.

In one embodiment of the present specification, Chemical Formula 1 may be represented by any one of the following compounds.

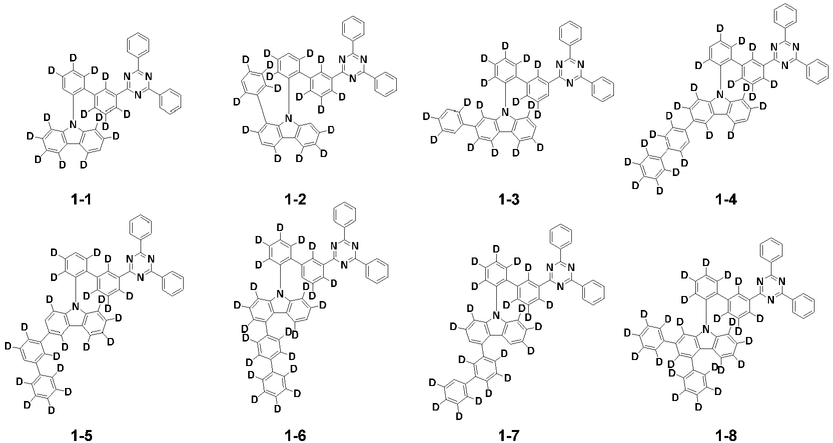

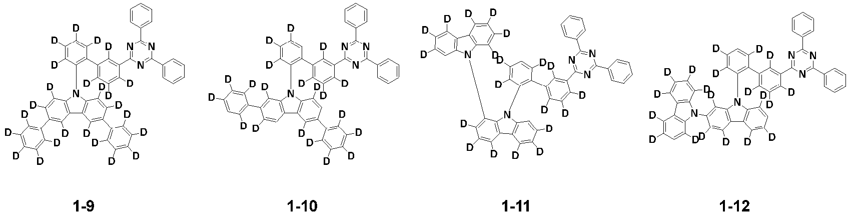

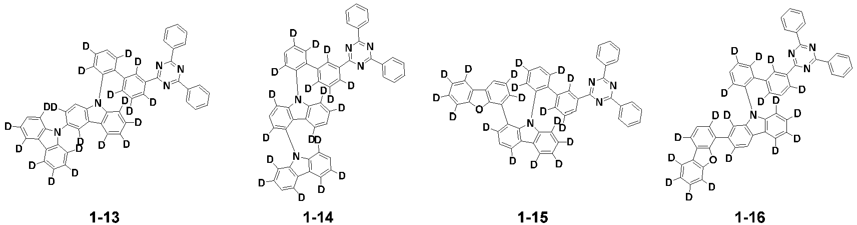

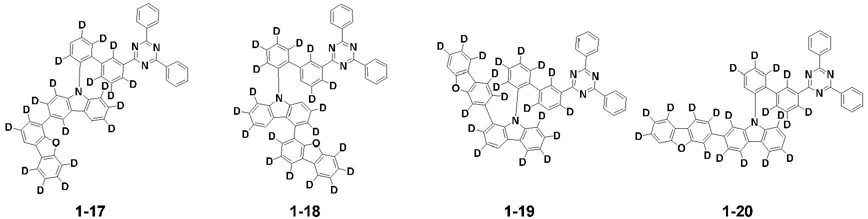

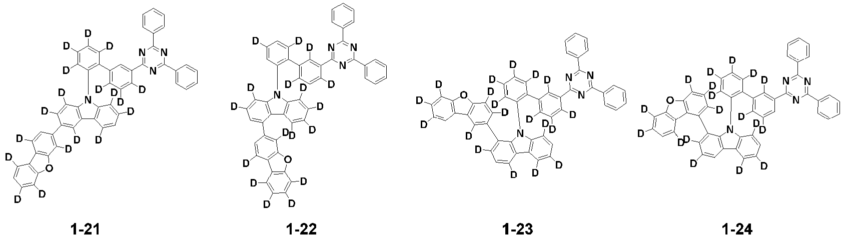

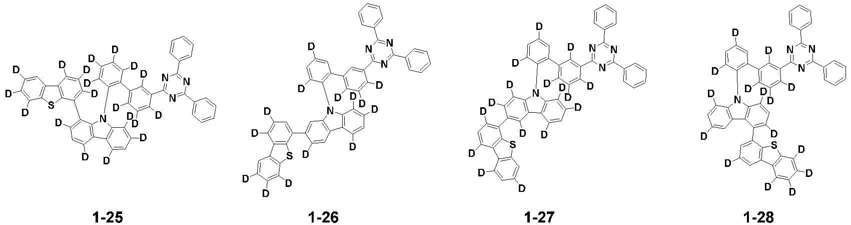

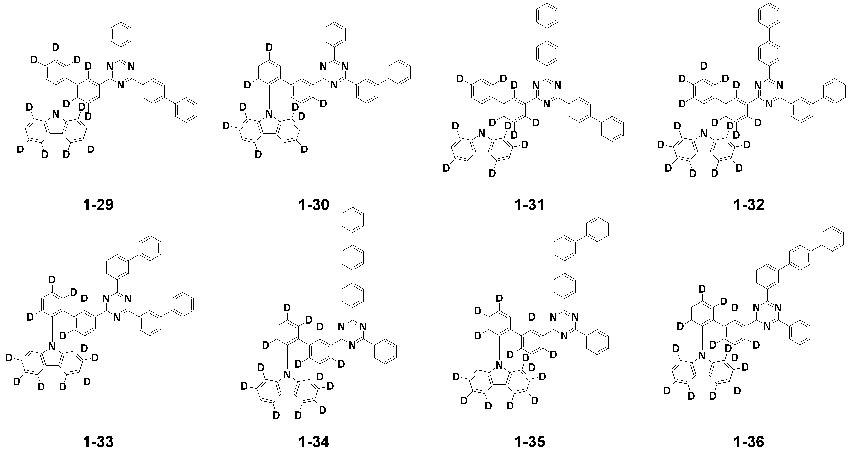

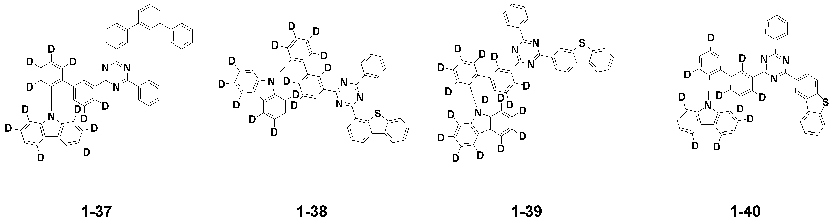

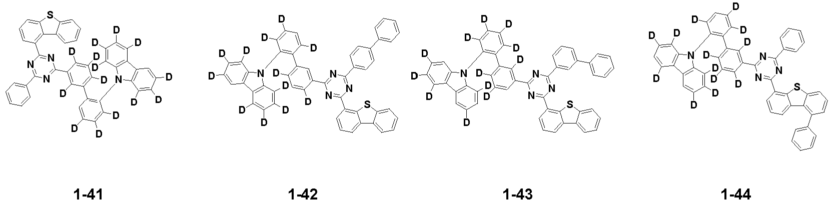

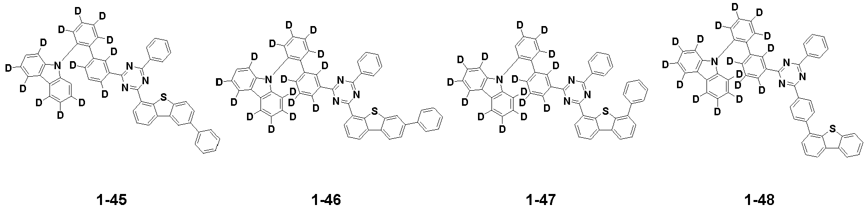

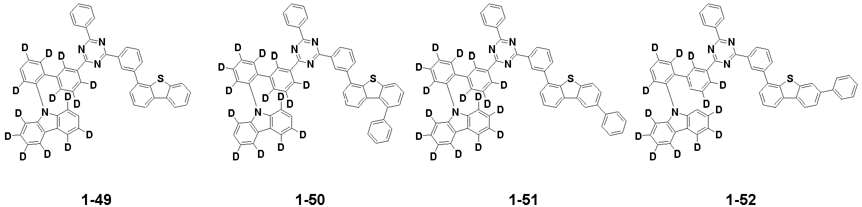

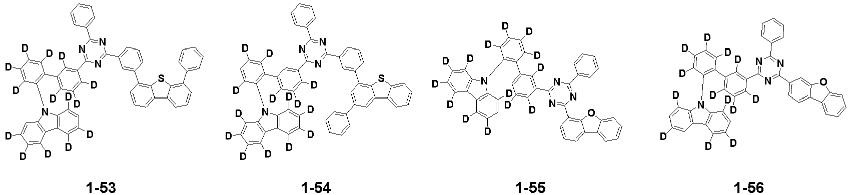

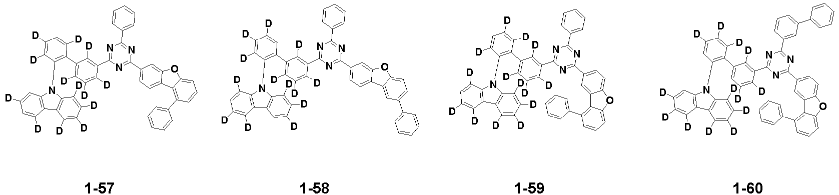

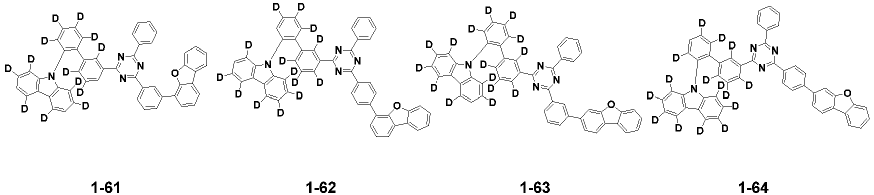

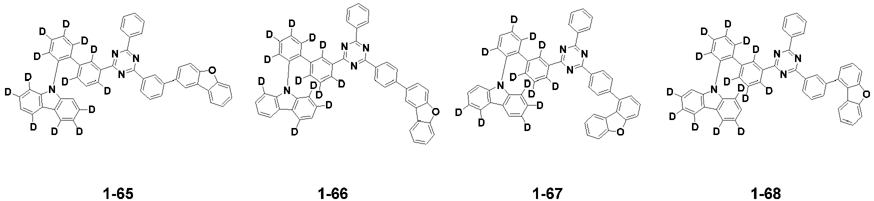

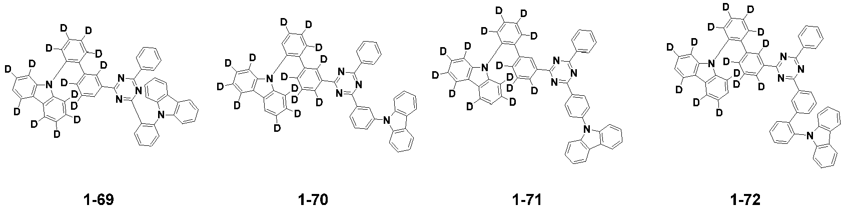

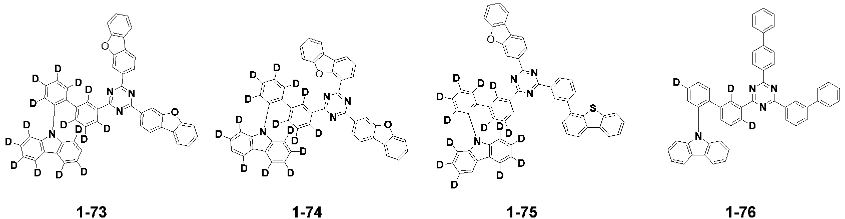

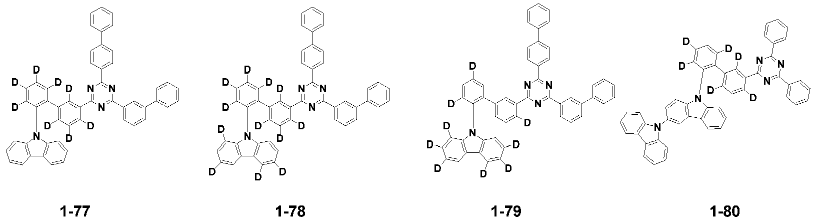

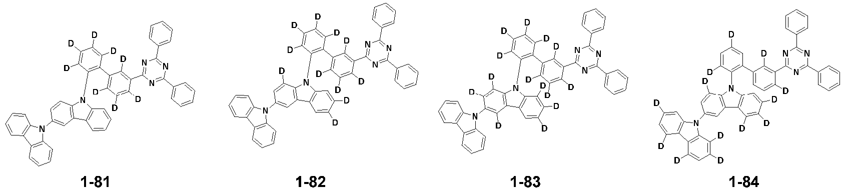

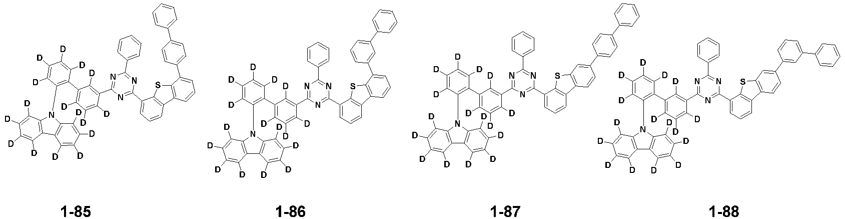

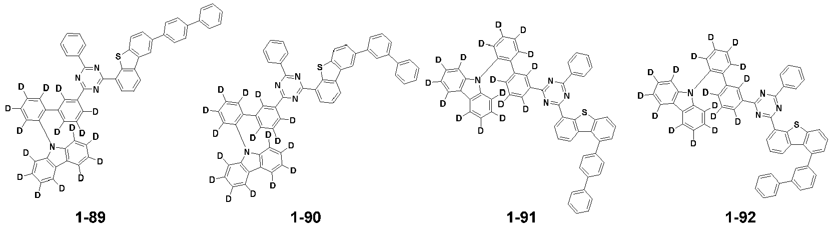

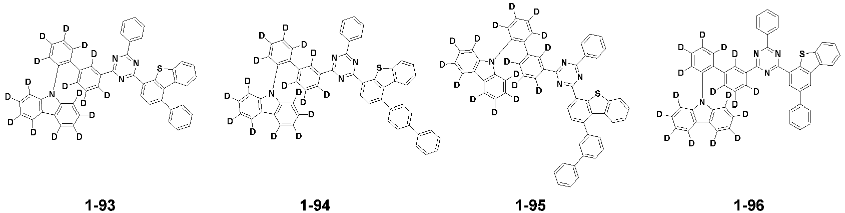

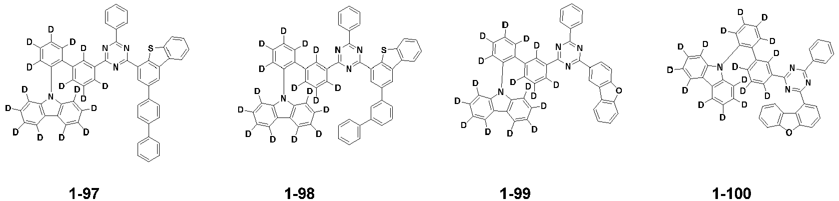

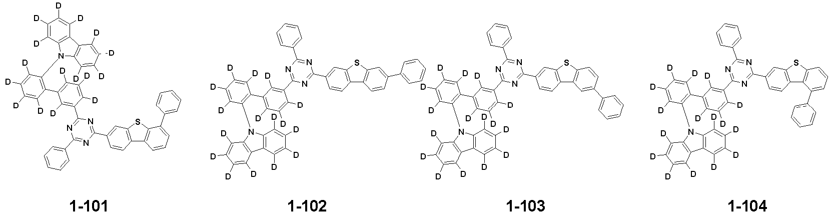

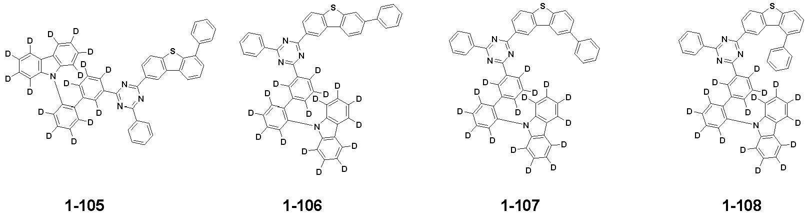

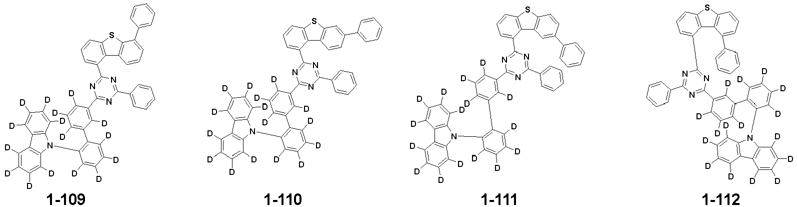

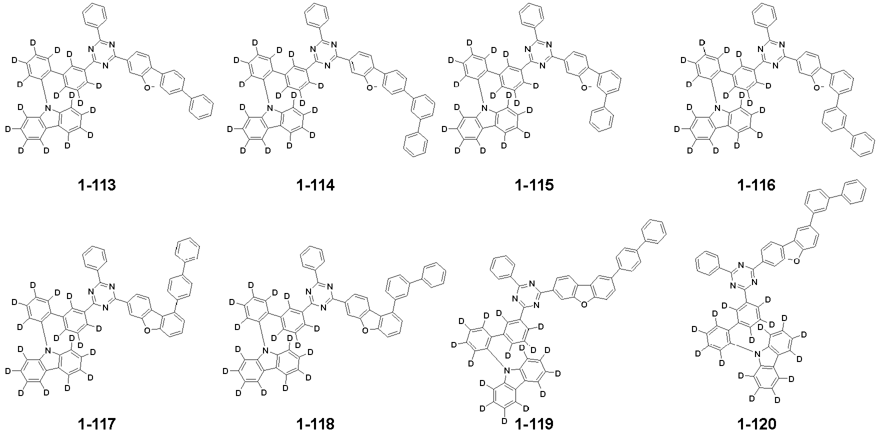

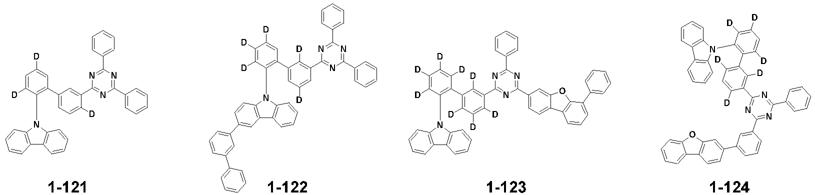

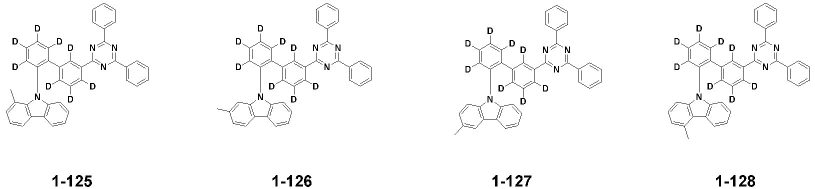

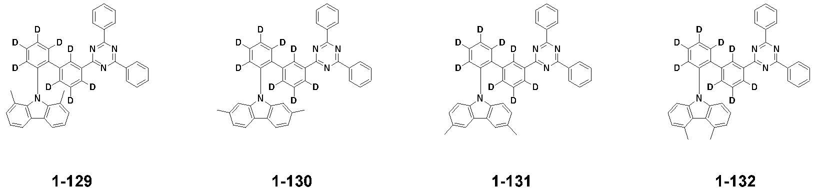

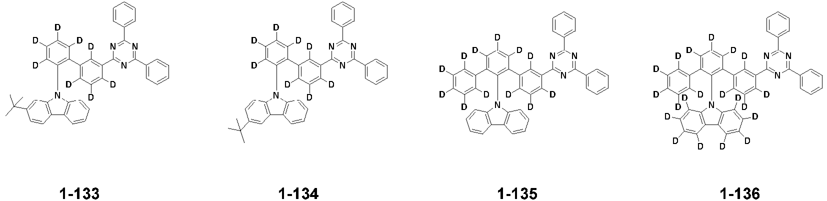

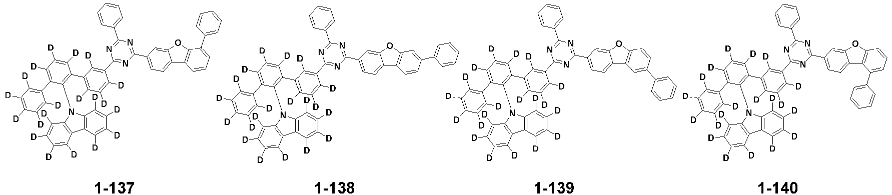

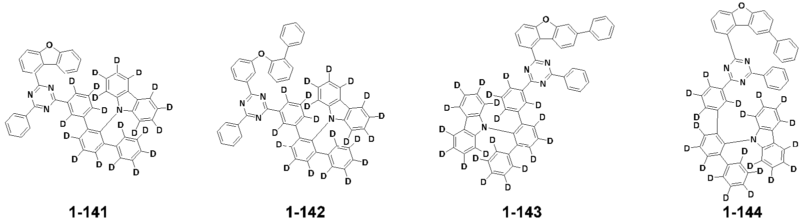

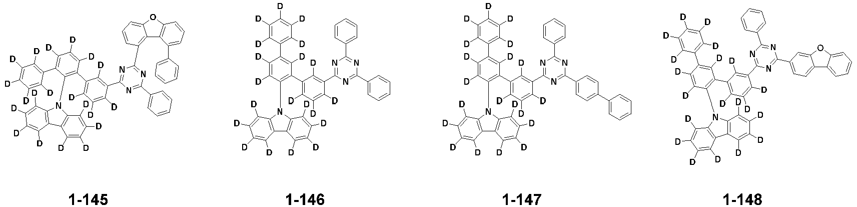

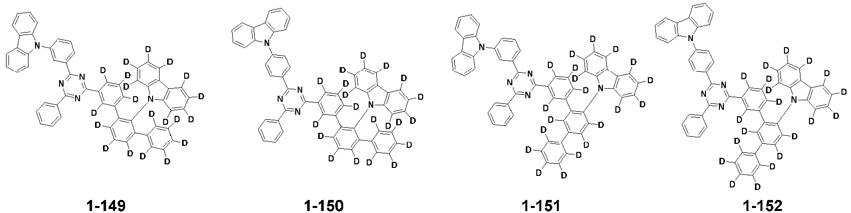

In addition, by introducing various substituents to the structure of Chemical Formula 1, compounds having unique properties of the introduced substituents may be synthesized. For example, by introducing substituents normally used for a hole injection layer material, a hole transport layer material, a light emitting layer material, an electron transport layer material and a charge generation layer material used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials may be enhanced, and material applications may become diverse.

Another embodiment of the present disclosure provides an organic light emitting device including the heterocyclic compound represented by Chemical Formula 1. The "organic light emitting device" may be expressed in terms such as an "organic light emitting diode", an "OLED", an "OLED device" and an "organic electroluminescent device".

One embodiment of the present application provides an organic light emitting device including: a first electrode; a second electrode provided opposite to the first electrode; and one or more organic material layers provided between the first electrode and the second electrode, wherein one or more layers of the organic material layers include the heterocyclic compound represented by Chemical Formula 1.

In one embodiment of the present application, the first electrode may be a positive electrode, and the second electrode may be a negative electrode.

In another embodiment, the first electrode may be a negative electrode, and the second electrode may be a positive electrode.

In one embodiment of the present application, the organic light emitting device may be a blue organic light emitting device, and the heterocyclic compound according to Chemical Formula 1 may be used as a material of the blue organic light emitting device.

In one embodiment of the present application, the organic light emitting device may be a green organic light emitting device, and the heterocyclic compound represented by Chemical Formula 1 may be used as a material of the green organic light emitting device.

In one embodiment of the present application, the organic light emitting device may be a red organic light emitting device, and the heterocyclic compound represented by Chemical Formula 1 may be used as a material of the red organic light emitting device.

In one embodiment of the present application, the organic light emitting device may be a blue organic light emitting device, and the heterocyclic compound according to Chemical Formula 1 may be used as a light emitting layer material of the blue organic light emitting device.

In one embodiment of the present application, the organic light emitting device may be a green organic light emitting device, and the heterocyclic compound represented by Chemical Formula 1 may be used as a light emitting layer material of the green organic light emitting device.

In one embodiment of the present application, the organic light emitting device may be a red organic light emitting device, and the heterocyclic compound represented by Chemical Formula 1 may be used as a light emitting layer material of the red organic light emitting device.

Specific descriptions on the heterocyclic compound represented by Chemical Formula 1 are the same as the descriptions provided above.

The organic light emitting device of the present disclosure may be manufactured using common organic light emitting device manufacturing methods and materials except that one or more organic material layers are formed using the heterocyclic compound described above.

The heterocyclic compound may be formed into the organic material layer using a solution coating method as well as a vacuum deposition method when the organic light emitting device is manufactured. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may also be formed in a multilayer structure in which two or more organic material layers are laminated. For example, the organic light emitting device of the present disclosure may have a structure including a hole injection layer, a hole transport layer, a light emitting layer, an electron transport layer, an electron injection layer and the like as the organic material layer. However, the structure of the organic light emitting device is not limited thereto, and may include a smaller number of organic material layers.

Particularly, the heterocyclic compound represented by Chemical Formula 1 has a structural feature that includes a hexagonal heteroring including Y1 to Y3 that may be in charge of LUMO (Lowest Unoccupied Molecular Orbital) in the molecule by functioning as a strong electron acceptor, and accordingly, achieves electron stabilization in the molecule by effectively attracting electrons, thereby improving a lifetime of a device.

In addition, the heterocyclic compound represented by Chemical Formula 1 has a structural feature that includes a carbazole-based heterocyclic functional group that may be in charge of HOMO (Highest Occupied Molecular Orbital) in the molecule by functioning as a strong electron donor, and accordingly, may have high hole mobility and smooth charge transfer in the molecule, thereby having low driving and high efficiency.

In addition thereto, the heterocyclic compound represented by Chemical Formula 1 has a structural feature in which the carbazole-based heteroring substitutes the biphenyl group at an ortho position, and accordingly, spatial steric hindrance is generated and spatial charge mobility increases, thereby improving lifetime properties while achieving high light emission efficiency.

In addition, the heterocyclic compound represented by Chemical Formula 1 has a structural feature in which the biphenyl group between the hexagonal heteroring including Y1 to Y3 and the carbazole-based heterocyclic functional group is substituted with at least one deuterium, and since the bond length of carbon-deuterium is shorter than the bond length of carbon-hydrogen, the Van der Waals force generated between the molecules is weakened, and interaction between the molecules is weakened. Accordingly, the volume of the thin film of a device increases, reducing crystallinity of the thin film, and the device may have an improved lifetime.

Accordingly, when the compound represented by Chemical Formula 1 is used in an organic material layer, it is possible to lower a driving voltage of an organic light emitting device, improve light emission efficiency thereof, and improve lifetime properties of the organic light emitting device by thermal stability of the compound.

In the organic light emitting device of the present disclosure, the organic material layer includes a light emitting layer, and the light emitting layer may include the heterocyclic compound represented by Chemical Formula 1.

In the organic light emitting device of the present disclosure, the organic material layer includes a light emitting layer, and the light emitting layer may include the heterocyclic compound represented by Chemical Formula 1 as a host of the light emitting layer.

In another embodiment of the present disclosure, the organic light emitting device may further include one, or two or more layers selected from the group consisting of a light emitting layer, a hole injection layer, a hole transport layer, a hole transport auxiliary layer, an electron injection layer, an electron transport layer, an electron blocking layer and a hole blocking layer.

In one embodiment of the present disclosure, the organic light emitting device may include one or more organic material layers, the organic material layers may include a hole transport layer, and the hole transport layer may include the heterocyclic compound represented by Chemical Formula 1.

In one embodiment of the present disclosure, the organic light emitting device may include one or more organic material layers, the organic material layers may include a hole transport auxiliary layer, and the hole transport auxiliary layer may include the heterocyclic compound represented by Chemical Formula 1.

In one embodiment of the present disclosure, the organic material layer includes the heterocyclic compound represented by Chemical Formula 1, and a phosphorescent dopant may be used therewith.

As the phosphorescent dopant material, those known in the art may be used. For example, phosphorescent dopant materials represented by LL'MX', LL'L"M, LMX'X", L₂MX' and L₃M may be used, however, the scope of the present disclosure is not limited by these examples.

M may be iridium, platinum, osmium or the like.

L is an anionic bidentate ligand coordinated to M by sp² carbon and heteroatom, and X may function to trap electrons or holes. Nonlimiting examples of L, L' and L" may include 2-(1-naphthyl)benzoxazole, 2-phenylbenzoxazole, 2-phenylbenzothiazole, 7,8-benzoquinoline, phenylpyridine, benzothiophenylpyridine, 3-methoxy-2-phenylpyridine, thiophenylpyridine, tolylpyridine and the like. Nonlimiting examples of X' and X" may include acetylacetonate (acac), hexafluoroacetylacetonate, salicylidene, picolinate, 8-hydroxyquinolinate and the like.

Specific examples of the phosphorescent dopant are shown below, however, the phosphorescent dopant is not limited to these examples:

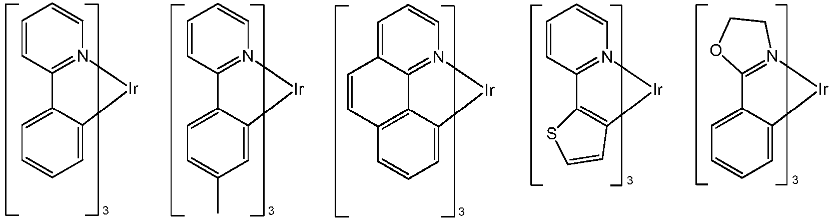

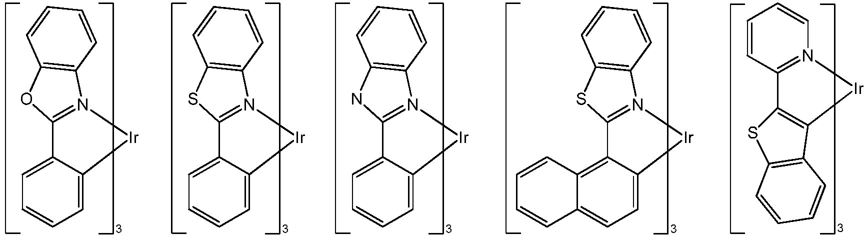

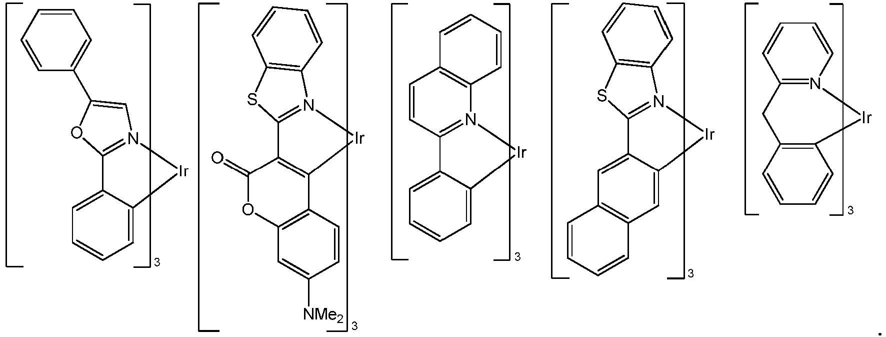

In one embodiment of the present disclosure, the organic material layer includes the heterocyclic compound represented by Chemical Formula 1, and an iridium-based dopant may be used therewith.

In one embodiment of the present disclosure, as the iridium-based dopant, (piq)₂(Ir) (acac), a red phosphorescent dopant, may be used.

In one embodiment of the present disclosure, as the iridium-based dopant, Ir(ppy)₃, a green phosphorescent dopant, may be used.

In one embodiment of the present disclosure, the content of the dopant may be from 1% to 15%, preferably from 2% to 10% and more preferably from 3% to 7% based on the total weight of the light emitting layer.

In the organic light emitting device according to one embodiment of the present disclosure, the organic material layer includes a hole transport layer or a hole transport auxiliary layer, and the hole transport layer or the hole transport auxiliary layer may include the heterocyclic compound represented by Chemical Formula 1.

In the organic light emitting device according to another embodiment of the present disclosure, the organic material layer includes an electron injection layer or an electron transport layer, and the electron injection layer or the electron transport layer may include the heterocyclic compound represented by Chemical Formula 1.

In the organic light emitting device according to another embodiment of the present disclosure, the organic material layer includes an electron blocking layer or a hole blocking layer, and the electron blocking layer or the hole blocking layer may include the heterocyclic compound represented by Chemical Formula 1.

In the organic light emitting device according to another embodiment of the present disclosure, the organic material layer includes an electron transport layer, a light emitting layer or a hole blocking layer, and the electron transport layer, the light emitting layer or the hole blocking layer may include the heterocyclic compound represented by Chemical Formula 1.

In the organic light emitting device according to another embodiment of the present disclosure, the organic material layer includes a light emitting layer, and the light emitting layer may include the heterocyclic compound represented by Chemical Formula 1.

In the organic light emitting device according to another embodiment of the present disclosure, the organic material layer includes a light emitting layer, the light emitting layer includes a host material, and the host material may include the heterocyclic compound represented by Chemical Formula 1.

In the organic light emitting device according to still another embodiment, the light emitting layer may include two or more host materials, and at least one of the host materials may include the heterocyclic compound represented by Chemical Formula 1.

In the organic light emitting device according to still another embodiment, two or more host materials may be pre-mixed and used in the light emitting layer, and at least one of the two or more host materials may include the heterocyclic compound represented by Chemical Formula 1.

The pre-mixing means, before depositing the two or more host materials on the organic material layer, putting and mixing the materials first in one source of supply.

In the organic light emitting device according to one embodiment of the present application, the organic material layer including the heterocyclic compound represented by Chemical Formula 1 further includes a heterocyclic compound represented by the following Chemical Formula 2 or Chemical Formula 3.

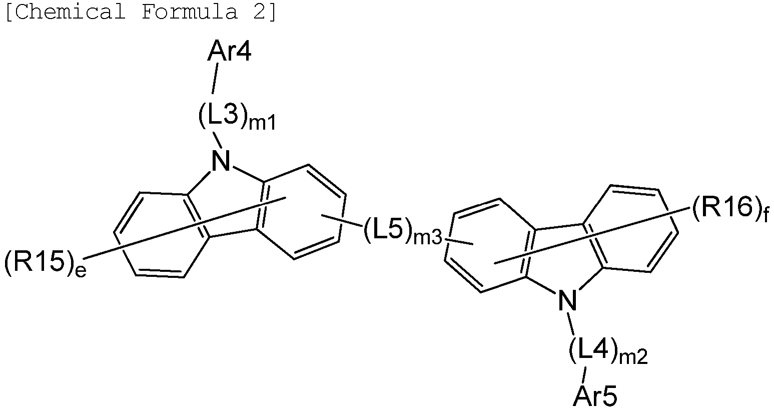

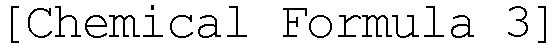

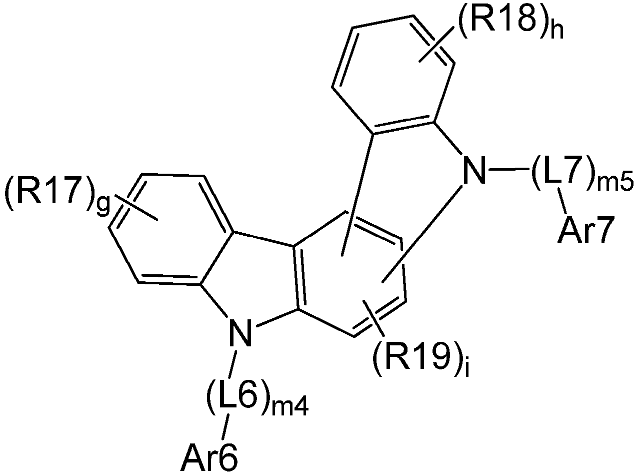

In Chemical Formula 2 and Chemical Formula 3,
L3 to L7 are the same as or different from each other, and each independently a single bond; a substituted or unsubstituted C6 to C60 arylene group; or a substituted or unsubstituted C2 to C60 heteroarylene group,
Ar4 to Ar7 are the same as or different from each other, and each independently a substituted or unsubstituted C6 to C60 aryl group; or a substituted or unsubstituted C2 to C60 heteroaryl group,
R15 to R19 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
m1 to m5 are the same as or different from each other, and each independently an integer of 0 to 3,
e and f are the same as or different from each other, and each independently an integer of 0 to 7,
g and h are the same as or different from each other, and each independently an integer of 0 to 4,
i is an integer of 0 to 2, and
when m1 to m5, e, f, g, h, and i are 2 or greater, L3s to L7s and R15s to R19s are the same as or different from each other.

When the heterocyclic compound represented by Chemical Formula 1 and the heterocyclic compound represented by Chemical Formula 2 or Chemical Formula 3 are included in an organic material layer of an organic light emitting device, improved efficiency and lifetime are obtained. From this result, it may be expected that an exciplex phenomenon occurs when the two compounds are included at the same time.

The exciplex phenomenon is a phenomenon of releasing energy having a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transport ability and an acceptor (n-host) having a favorable electron transport ability are used as a host of a light emitting layer, holes are injected to the p-host and electrons are injected to the n-host, and thus a driving voltage may be lowered, which resultantly helps with enhancement in the lifetime.

Particularly, the heterocyclic compound represented by Chemical Formula 1 has a structural feature that includes a hexagonal heteroring including Y1 to Y3 that may be in charge of LUMO (Lowest Unoccupied Molecular Orbital) in the molecule by functioning as a strong electron acceptor, and accordingly, achieves electron stabilization in the molecule by effectively attracting electrons, thereby improving a lifetime of a device.

In addition, the heterocyclic compound represented by Chemical Formula 1 has a structural feature that includes a carbazole-based heterocyclic functional group that may be in charge of HOMO (Highest Occupied Molecular Orbital) in the molecule by functioning as a strong electron donor, and accordingly, may have high hole mobility and smooth charge transfer in the molecule, thereby having low driving and high efficiency.

In addition thereto, the heterocyclic compound represented by Chemical Formula 1 has a structural feature in which the carbazole-based heteroring substitutes the biphenyl group at an ortho position, and accordingly, spatial steric hindrance is generated and spatial charge mobility increases, thereby improving lifetime properties while achieving high light emission efficiency.

In addition, the heterocyclic compound represented by Chemical Formula 1 has a structural feature in which the biphenyl group between the hexagonal heteroring including Y1 to Y3 and the carbazole-based heterocyclic functional group is substituted with at least one deuterium, and since the bond length of carbon-deuterium is shorter than the bond length of carbon-hydrogen, the Van der Waals force generated between the molecules is weakened, and interaction between the molecules is weakened. Accordingly, the volume of the thin film of a device increases, reducing crystallinity of the thin film, and the device may have an improved lifetime.

Accordingly, when the compound represented by Chemical Formula 1 is used in an organic material layer, it is possible to lower a driving voltage of an organic light emitting device, improve light emission efficiency thereof, and improve lifetime properties of the organic light emitting device by thermal stability of the compound.

In one embodiment of the present application, L3 to L7 are the same as or different from each other, and may be each independently a single bond; a substituted or unsubstituted C6 to C40 arylene group; or a substituted or unsubstituted C2 to C40 heteroarylene group.

In another embodiment, L3 to L7 are the same as or different from each other, and may be each independently a single bond; a substituted or unsubstituted C6 to C30 arylene group; or a substituted or unsubstituted C2 to C30 heteroarylene group.

In another embodiment, L3 to L7 are the same as or different from each other, and may be each independently a single bond; a substituted or unsubstituted C6 to C20 arylene group; or a substituted or unsubstituted C2 to C20 heteroarylene group.

In another embodiment, L3 to L7 are the same as or different from each other, and may be each independently a single bond; a substituted or unsubstituted C6 to C10 arylene group; or a substituted or unsubstituted C2 to C10 heteroarylene group.

In another embodiment, L3 to L7 are the same as or different from each other, and may be each independently a single bond; a substituted or unsubstituted phenylene group; a substituted or unsubstituted biphenylene group; a substituted or unsubstituted terphenylene group; a substituted or unsubstituted naphthylene group; a substituted or unsubstituted fluorene group; a substituted or unsubstituted dibenzofuranylene group; or a substituted or unsubstituted dibenzothiophenylene group.

In another embodiment, L3 to L7 are the same as or different from each other, and may be each independently a single bond; a substituted or unsubstituted phenylene group; a substituted or unsubstituted biphenylene group; a substituted or unsubstituted fluorene group; or a substituted or unsubstituted dibenzofuranylene group.

In one embodiment of the present application, Ar4 to Ar7 are the same as or different from each other, and may be each independently a substituted or unsubstituted C6 to C40 aryl group; or a substituted or unsubstituted C2 to C40 heteroaryl group.

In another embodiment, Ar4 to Ar7 are the same as or different from each other, and may be each independently a substituted or unsubstituted C6 to C30 aryl group; or a substituted or unsubstituted C2 to C30 heteroaryl group.

In another embodiment, Ar4 to Ar7 are the same as or different from each other, and may be each independently a substituted or unsubstituted C6 to C20 aryl group; or a substituted or unsubstituted C2 to C20 heteroaryl group.

In another embodiment, Ar4 to Ar7 are the same as or different from each other, and may be each independently a substituted or unsubstituted C6 to C10 aryl group; or a substituted or unsubstituted C2 to C10 heteroaryl group.

In another embodiment, Ar4 to Ar7 are the same as or different from each other, and may be each independently a substituted or unsubstituted phenyl group; a substituted or unsubstituted biphenyl group; a substituted or unsubstituted naphthyl group; a substituted or unsubstituted terphenyl group; a substituted or unsubstituted fluorenyl group; a substituted or unsubstituted triphenyl group; a substituted or unsubstituted dibenzofuranyl group; a substituted or unsubstituted dibenzothiophenyl group; or a substituted or unsubstituted tetraphenylsilanyl group.

In one embodiment of the present application, R15 to R19 are the same as or different from each other, and each independently selected from the group consisting of hydrogen; deuterium; halogen; a cyano group; a substituted or unsubstituted C1 to C40 alkyl group; a substituted or unsubstituted C2 to C40 alkenyl group; a substituted or unsubstituted C2 to C40 alkynyl group; a substituted or unsubstituted C1 to C40 alkoxy group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; -P(=O)R101R102; and -SiR101R102R103, or two or more groups adjacent to each other bond to each other to form a substituted or unsubstituted C6 to C40 aromatic hydrocarbon ring; or a substituted or unsubstituted C2 to C40 heteroring, and R101, R102 and R103 are the same as or different from each other and may be each independently a substituted or unsubstituted C1 to C40 alkyl group; a substituted or unsubstituted C6 to C40 aryl group; or a substituted or unsubstituted C2 to C40 heteroaryl group.

In another embodiment, R15 to R19 are the same as or different from each other, and each independently selected from the group consisting of hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R101R102; and -SiR101R102R103, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment, R15 to R19 are the same as or different from each other, and each independently selected from the group consisting of hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; and a substituted or unsubstituted C2 to C20 heteroaryl group, or two or more groups adjacent to each other may bond to each other to form a substituted or unsubstituted C6 to C20 aromatic hydrocarbon ring; or a substituted or unsubstituted C2 to C20 heteroring.

In another embodiment, R15 to R19 are the same as or different from each other, and each independently selected from the group consisting of hydrogen; deuterium; halogen; a cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C1 to C10 alkoxy group; a substituted or unsubstituted C3 to C10 cycloalkyl group; a substituted or unsubstituted C2 to C10 heterocycloalkyl group; a substituted or unsubstituted C6 to C10 aryl group; and a substituted or unsubstituted C2 to C10 heteroaryl group, or two or more groups adjacent to each other may bond to each other to form a substituted or unsubstituted C6 to C10 aromatic hydrocarbon ring; or a substituted or unsubstituted C2 to C10 heteroring.

In another embodiment, R15 to R19 are the same as or different from each other, and may be each independently hydrogen; deuterium; a substituted or unsubstituted phenyl group; a substituted or unsubstituted biphenyl group; a substituted or unsubstituted naphthyl group; or a substituted or unsubstituted terphenyl group.

In another embodiment, R15 to R19 are the same as or different from each other, and may be each independently hydrogen; or deuterium.

In one embodiment of the present application, Ar4 to Ar7 may be represented by any one of the following Chemical Formula 2-1 and Chemical Formula 2-2.

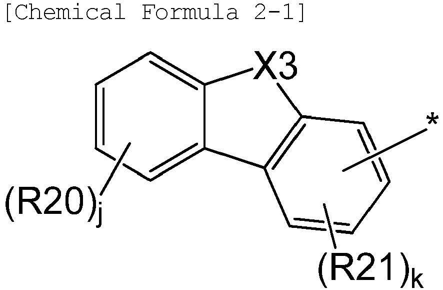

[Chemical Formula 2-2] Ar8-*

In Chemical Formula 2-1 and Chemical Formula 2-2,
X3 is O; S; or CRaRb,
Ar8 is a substituted or unsubstituted C6 to C60 aryl group,
R20, R21, Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
* is a site linked to Chemical Formula 2 or Chemical Formula 3,
j is an integer of 0 to 4,
k is an integer of 0 to 3, and
when j and k are 2 or greater, R20s and R21s are the same as or different from each other.

In one embodiment of the present application, X3 may be O.

In another embodiment, X3 may be S.

In another embodiment, X3 may be CRaRb.

In one embodiment of the present application, Ar8 may be a substituted or unsubstituted C6 to C40 aryl group.

In another embodiment, Ar8 may be a substituted or unsubstituted C6 to C30 aryl group.

In another embodiment, Ar8 may be a substituted or unsubstituted C6 to C20 aryl group.

In another embodiment, Ar8 may be a substituted or unsubstituted C6 to C10 aryl group.

In another embodiment, Ar8 may be selected from a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; or a substituted or unsubstituted triphenyl group.

In one embodiment of the present application, R20, R21, Ra and Rb are the same as or different from each other, and each independently selected from the group consisting of hydrogen; deuterium; halogen; a cyano group; a substituted or unsubstituted C1 to C40 alkyl group; a substituted or unsubstituted C2 to C40 alkenyl group; a substituted or unsubstituted C2 to C40 alkynyl group; a substituted or unsubstituted C1 to C40 alkoxy group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; -P(=O)R101R102; and -SiR101R102R103, or two or more groups adjacent to each other bond to each other to form a substituted or unsubstituted C6 to C40 aromatic hydrocarbon ring; or a substituted or unsubstituted C2 to C40 heteroring, and R101, R102 and R103 are the same as or different from each other and may be each independently a substituted or unsubstituted C1 to C40 alkyl group; a substituted or unsubstituted C6 to C40 aryl group; or a substituted or unsubstituted C2 to C40 heteroaryl group.

In another embodiment, R20, R21, Ra and Rb are the same as or different from each other, and each independently selected from the group consisting of hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R101R102; and -SiR101R102R103, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment, R20, R21, Ra and Rb are the same as or different from each other, and each independently selected from the group consisting of hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; and a substituted or unsubstituted C2 to C20 heteroaryl group, or two or more groups adjacent to each other may bond to each other to form a substituted or unsubstituted C6 to C20 aromatic hydrocarbon ring; or a substituted or unsubstituted C2 to C20 heteroring.

In another embodiment, R20, R21, Ra and Rb are the same as or different from each other, and each independently selected from the group consisting of hydrogen; deuterium; halogen; a cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C1 to C10 alkoxy group; a substituted or unsubstituted C3 to C10 cycloalkyl group; a substituted or unsubstituted C2 to C10 heterocycloalkyl group; a substituted or unsubstituted C6 to C10 aryl group; and a substituted or unsubstituted C2 to C10 heteroaryl group, or two or more groups adjacent to each other may bond to each other to form a substituted or unsubstituted C6 to C10 aromatic hydrocarbon ring; or a substituted or unsubstituted C2 to C10 heteroring.

In another embodiment, R20, R21, Ra and Rb are the same as or different from each other, and each independently hydrogen; deuterium; a substituted or unsubstituted methyl group; a substituted or unsubstituted phenyl group; a substituted or unsubstituted biphenyl group; a substituted or unsubstituted naphthyl group; a substituted or unsubstituted terphenyl group; a substituted or unsubstituted fluorenyl group; a substituted or unsubstituted 9,9-dimethylfluorenyl group; a substituted or unsubstituted anthracenyl group; a substituted or unsubstituted pyrenyl group; a substituted or unsubstituted chrysenyl group; a substituted or unsubstituted fluoranthenyl group; a substituted or unsubstituted 9,9-diphenylfluorenyl group; a substituted or unsubstituted phenanthrenyl group; a substituted or unsubstituted dibenzofuranyl group; a substituted or unsubstituted dibenzothiophenyl group; a substituted or unsubstituted spirobifluorenyl group; a substituted or unsubstituted triphenylenyl group; or a substituted or unsubstituted tetraphenylsilanyl group, or two or more groups adjacent to each other may bond to each other to form a substituted or unsubstituted spirobifluorenyl group.

In another embodiment, R20, R21, Ra and Rb are the same as or different from each other, and may be each independently hydrogen; deuterium; a substituted or unsubstituted methyl group; a substituted or unsubstituted phenyl group; or a substituted or unsubstituted fluorenyl group.

In one embodiment of the present application, the heterocyclic compound represented by Chemical Formula 3 may be represented by any one of the following Chemical Formula 3-1 to Chemical Formula 3-5.

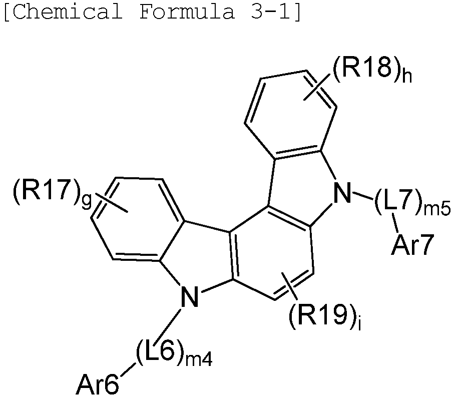

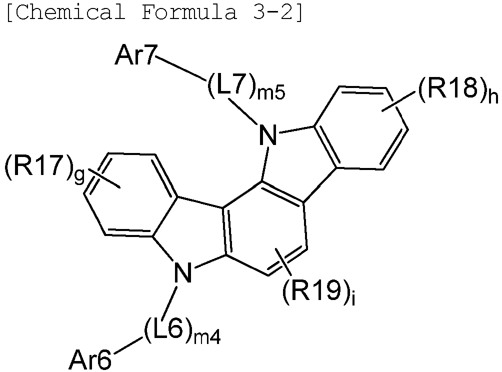

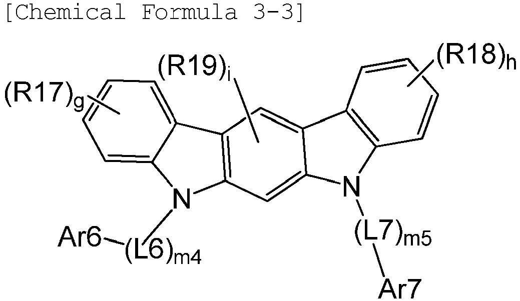

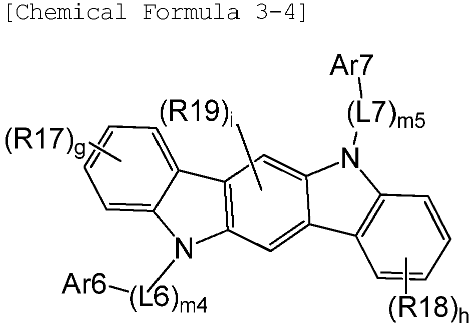

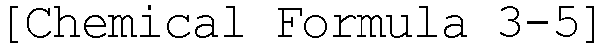

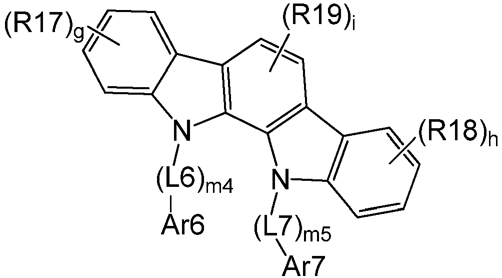

In Chemical Formula 3-1 to Chemical Formula 3-5,
R17 to R19, L6, L7, Ar6, Ar7, g to i, m4, and m5 have the same definitions as in Chemical Formula 3.

In one embodiment of the present application, the heterocyclic compound represented by Chemical Formula 2 or Chemical Formula 3 may not include deuterium as a substituent, or may have a deuterium content of, for example, 1% or greater, 5% or greater, 10% or greater, 15% or greater, 20% or greater, 25% or greater, 30% or greater, 35% or greater, 40% or greater, 45% or greater, or 50% or greater, and 100% or less, 95% or less, 90% or less, 85% or less, 80% or less, 75% or less, 70% or less, 65% or less or 60% or less based on the total number of hydrogen atoms and deuterium atoms.

In one embodiment of the present application, the heterocyclic compound represented by Chemical Formula 2 may not include deuterium, or may have a deuterium content of 1% to 100% based on the total number of hydrogen atoms and deuterium atoms.

In one embodiment of the present application, the heterocyclic compound represented by Chemical Formula 2 may not include deuterium, or may have a deuterium content of 10% to 100% based on the total number of hydrogen atoms and deuterium atoms.

In one embodiment of the present application, the heterocyclic compound represented by Chemical Formula 2 may not include deuterium, or may have a deuterium content of 20% to 90% based on the total number of hydrogen atoms and deuterium atoms.

In one embodiment of the present application, the heterocyclic compound represented by Chemical Formula 2 may not include deuterium, or may have a deuterium content of 30% to 80% based on the total number of hydrogen atoms and deuterium atoms.

In one embodiment of the present application, the heterocyclic compound represented by Chemical Formula 2 may not include deuterium, or may have a deuterium content of 40% to 70% based on the total number of hydrogen atoms and deuterium atoms.

In one embodiment of the present application, the heterocyclic compound represented by Chemical Formula 2 may not include deuterium, or may have a deuterium content of 50% to 60% based on the total number of hydrogen atoms and deuterium atoms.

In one embodiment of the present application, the heterocyclic compound represented by Chemical Formula 3 may not include deuterium, or may have a deuterium content of 1% to 100% based on the total number of hydrogen atoms and deuterium atoms.

In one embodiment of the present application, the heterocyclic compound represented by Chemical Formula 3 may not include deuterium, or may have a deuterium content of 10% to 100% based on the total number of hydrogen atoms and deuterium atoms.

In one embodiment of the present application, the heterocyclic compound represented by Chemical Formula 3 may not include deuterium, or may have a deuterium content of 20% to 90% based on the total number of hydrogen atoms and deuterium atoms.

In one embodiment of the present application, the heterocyclic compound represented by Chemical Formula 3 may not include deuterium, or may have a deuterium content of 30% to 80% based on the total number of hydrogen atoms and deuterium atoms.

In one embodiment of the present application, the heterocyclic compound represented by Chemical Formula 3 may not include deuterium, or may have a deuterium content of 40% to 70% based on the total number of hydrogen atoms and deuterium atoms.

In one embodiment of the present application, the heterocyclic compound represented by Chemical Formula 3 may not include deuterium, or may have a deuterium content of 50% to 60% based on the total number of hydrogen atoms and deuterium atoms.

One embodiment of the present application provides a heterocyclic compound, in which Chemical Formula 2 is represented by any one of the following compounds. In addition, in one embodiment of the present application, the following compounds are just one example, and the present application is not limited thereto and may include other compounds included in Chemical Formula 2 that includes additional substituents.

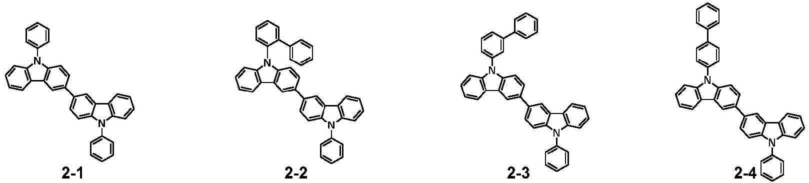

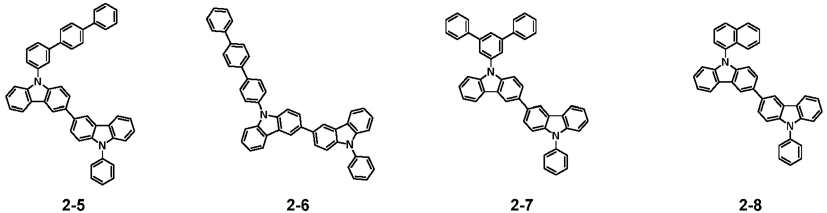

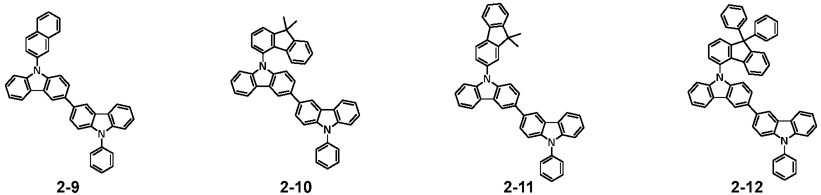

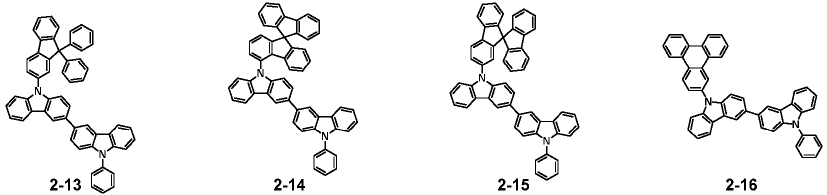

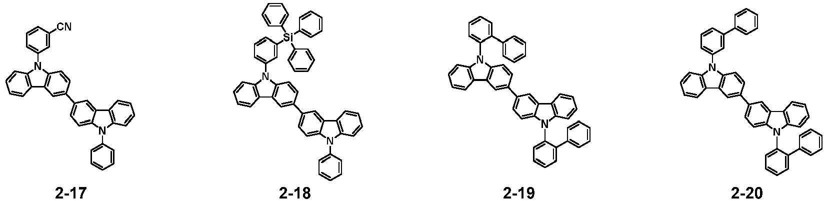

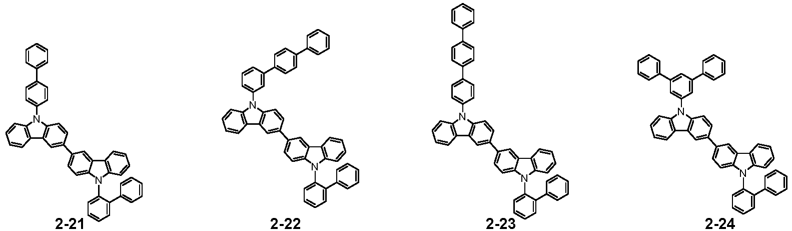

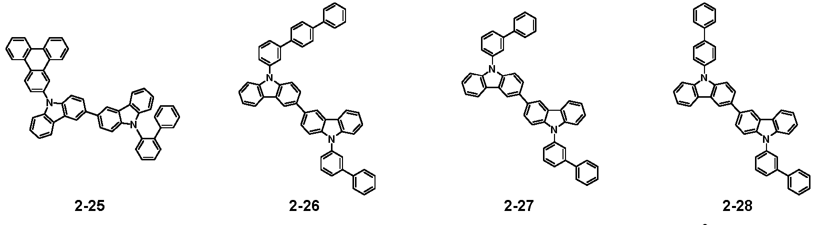

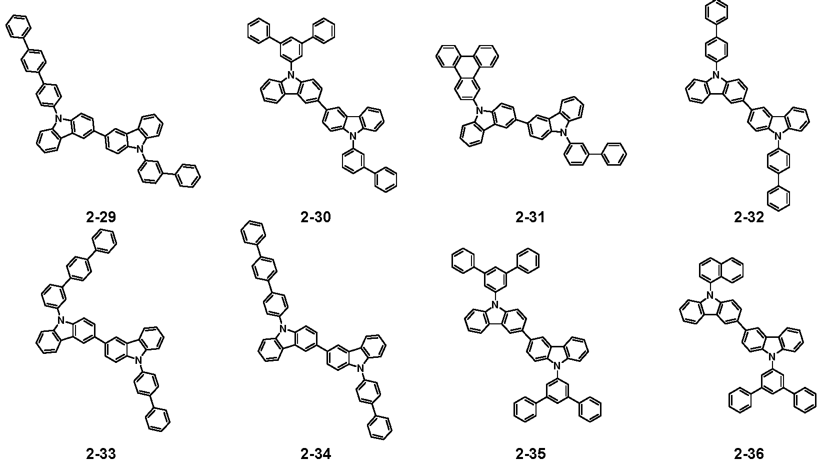

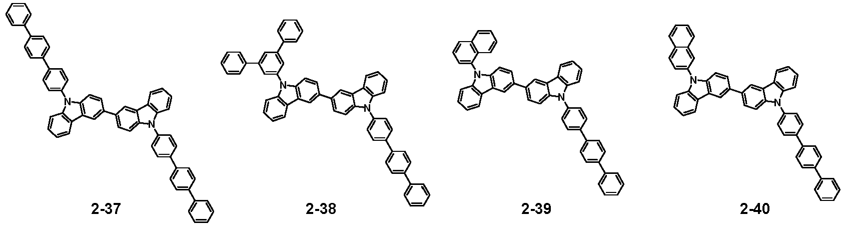

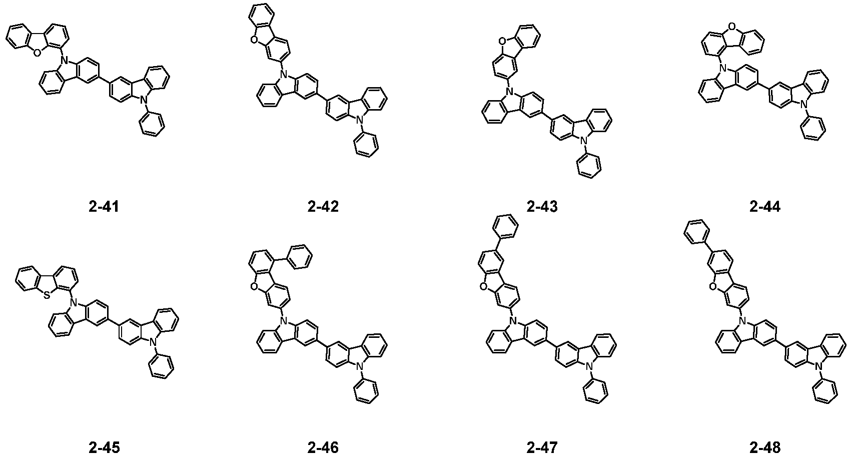

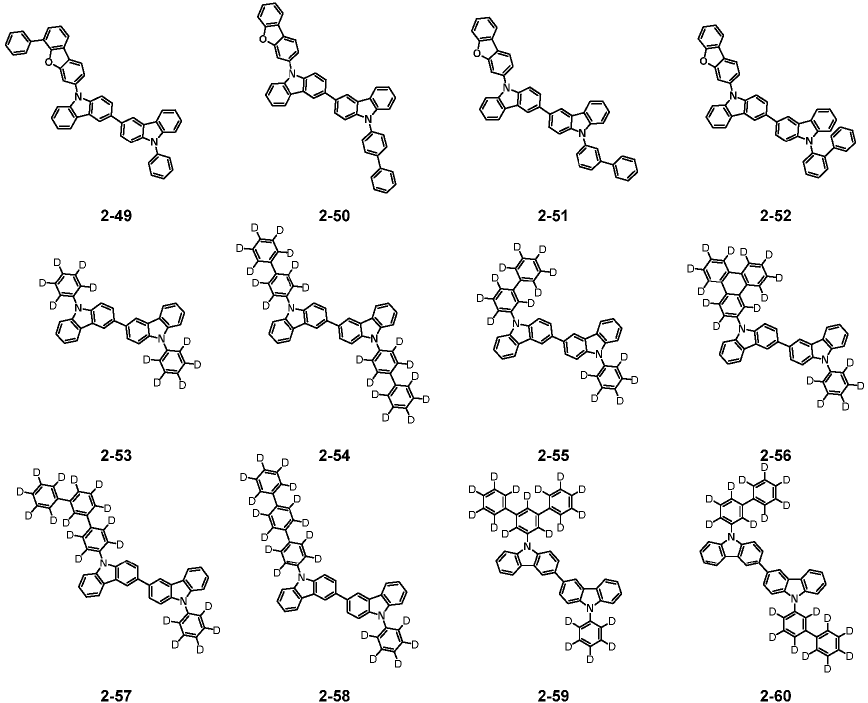

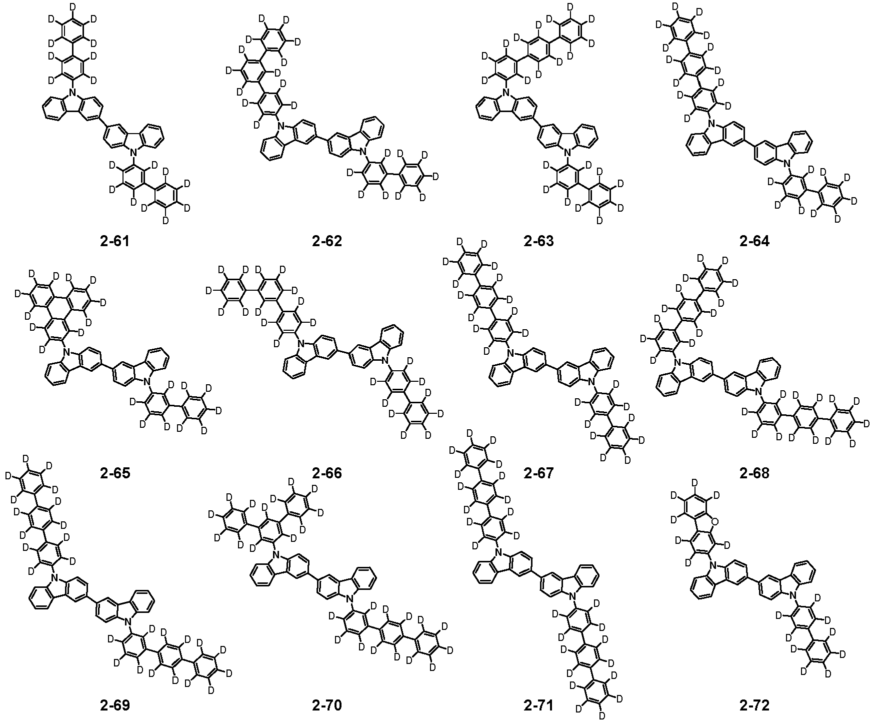

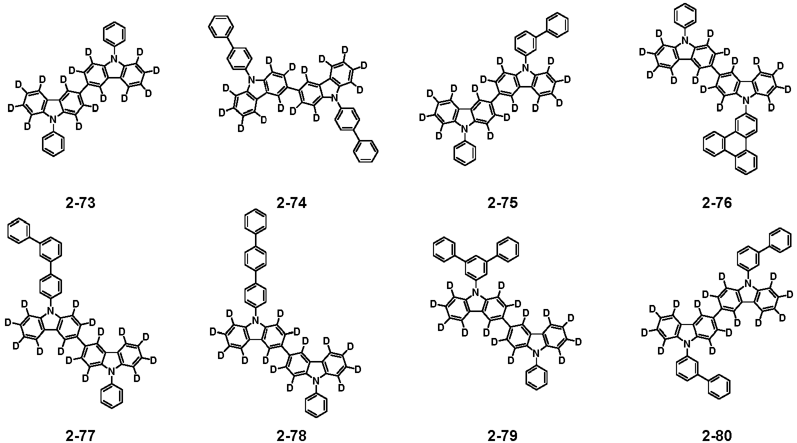

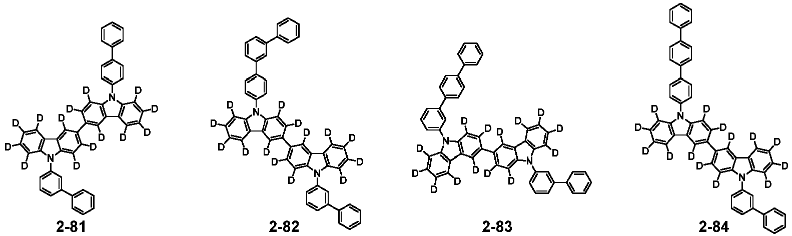

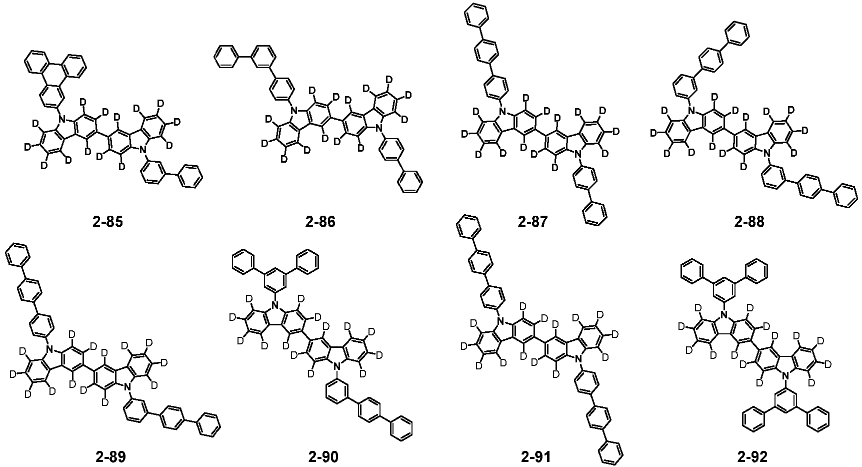

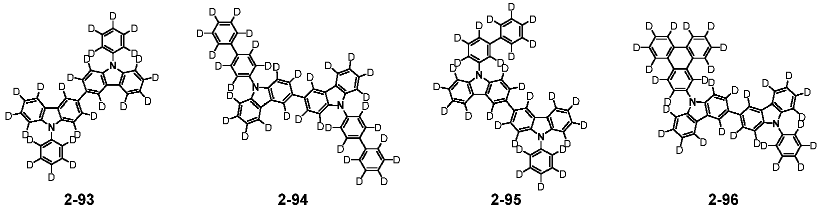

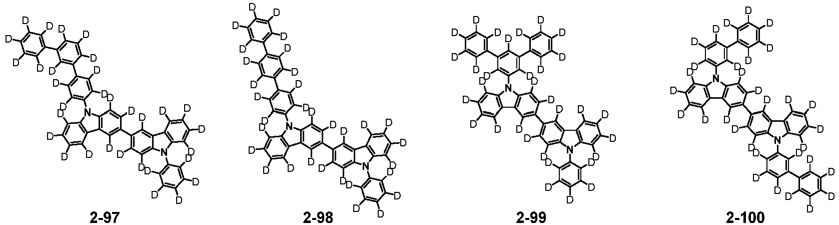

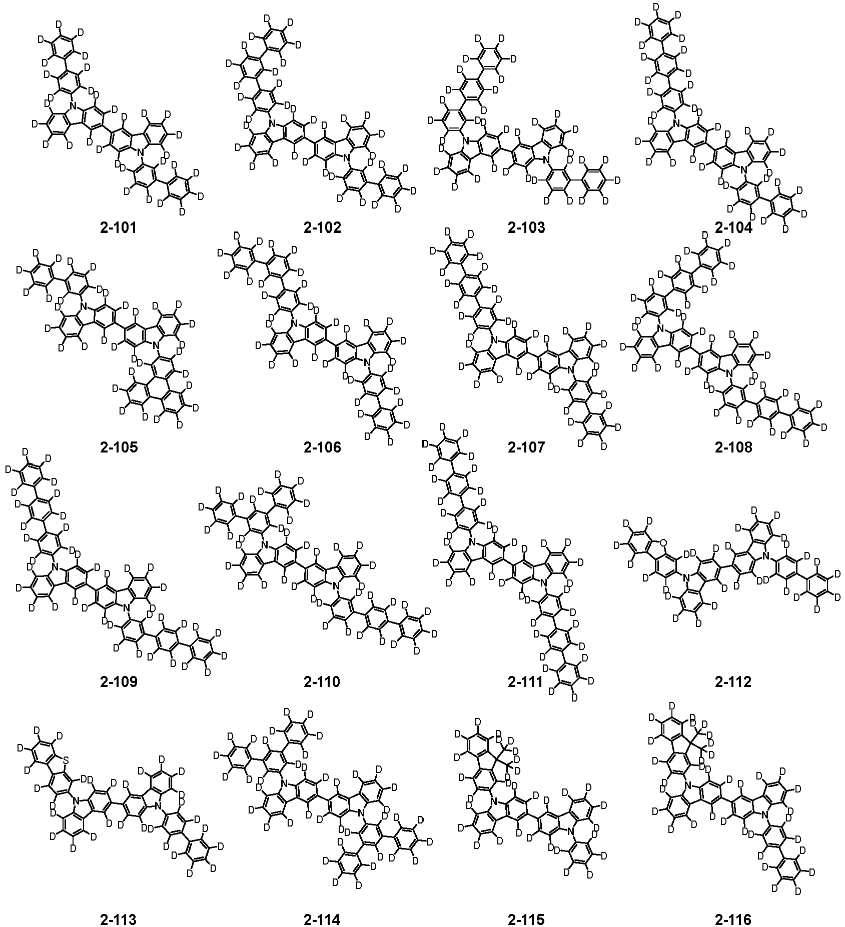

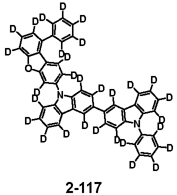

One embodiment of the present application provides a heterocyclic compound, in which Chemical Formula 3 is represented by any one of the following compounds. In addition, in one embodiment of the present application, the following compounds are just one example, and the present application is not limited thereto and may include other compounds included in Chemical Formula 3 that includes additional substituents.

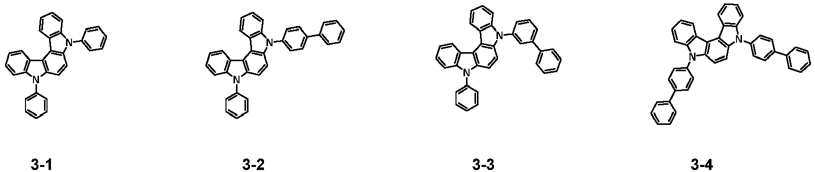

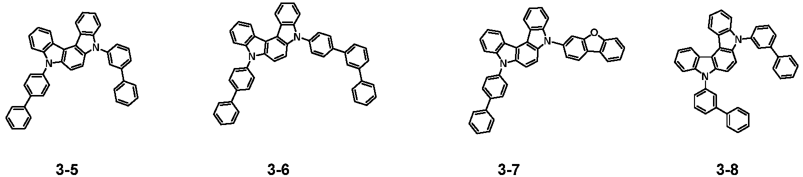

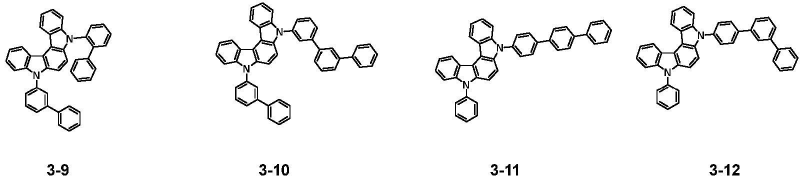

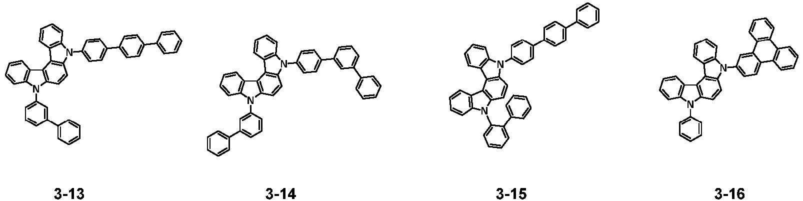

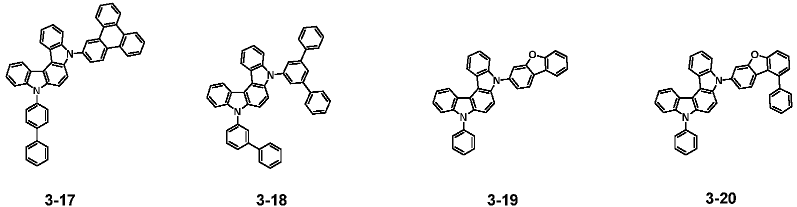

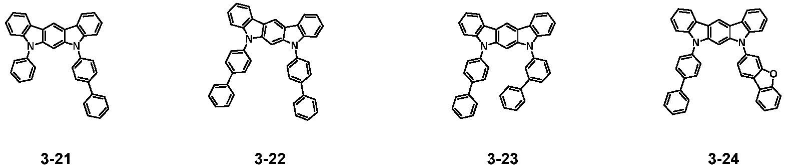

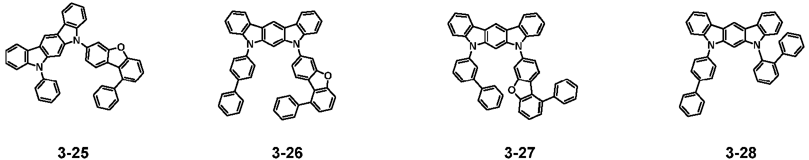

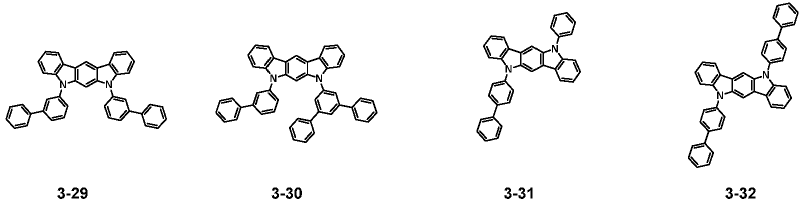

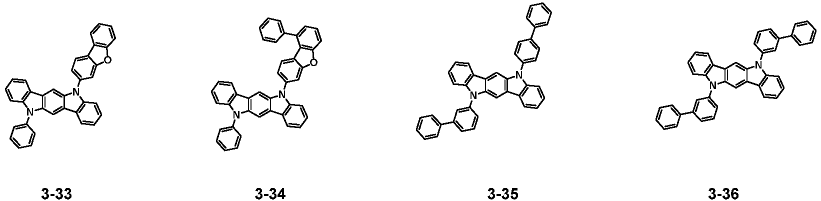

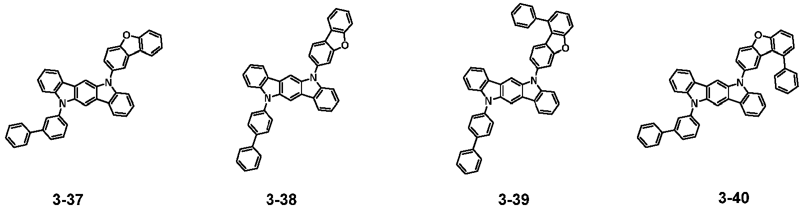

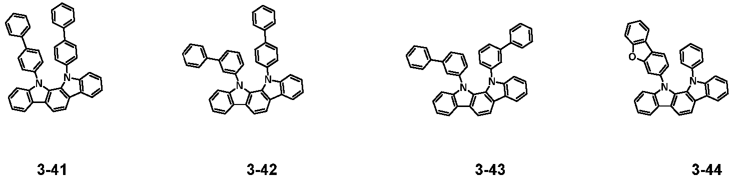

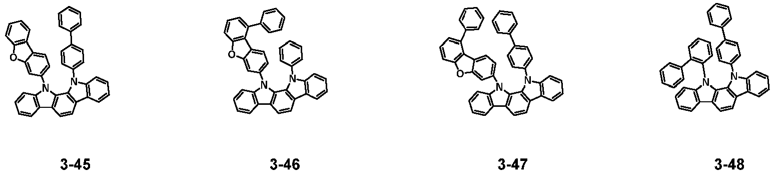

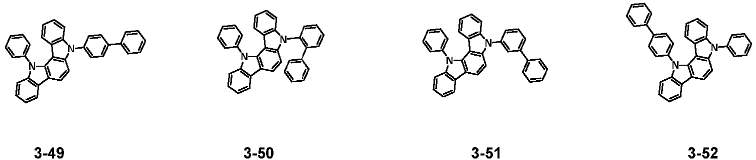

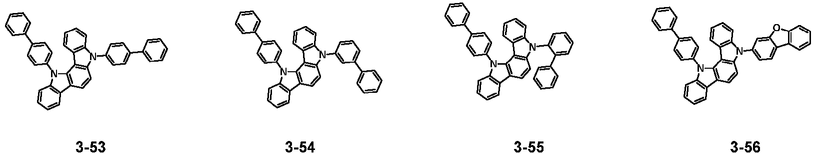

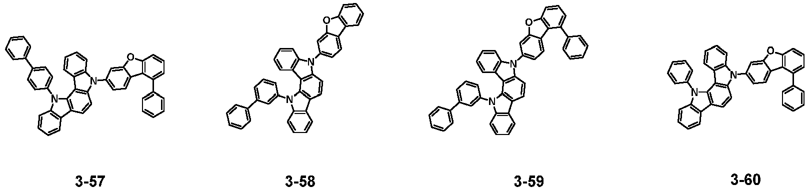

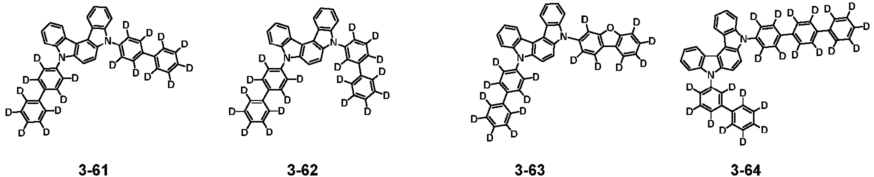

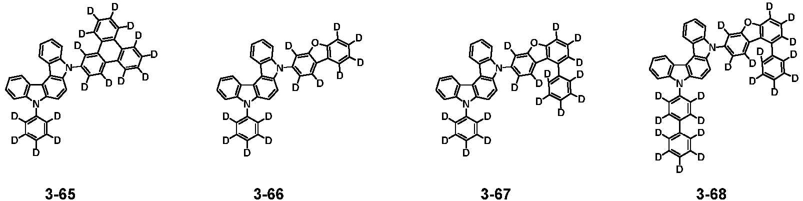

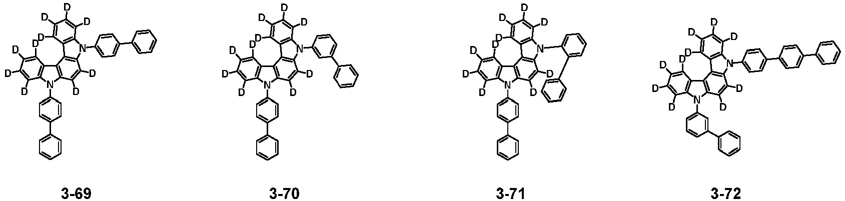

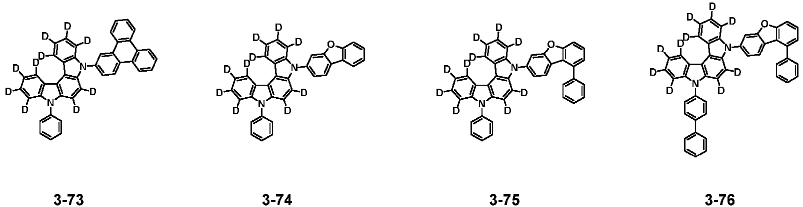

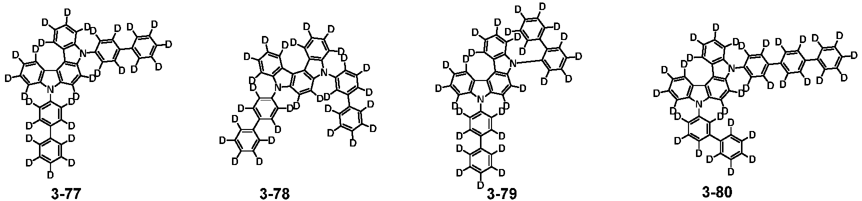

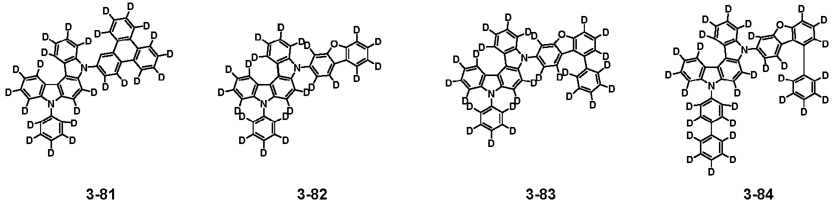

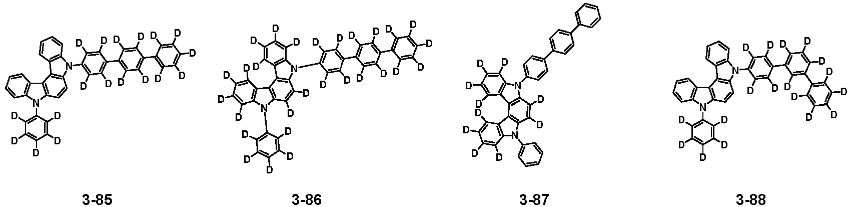

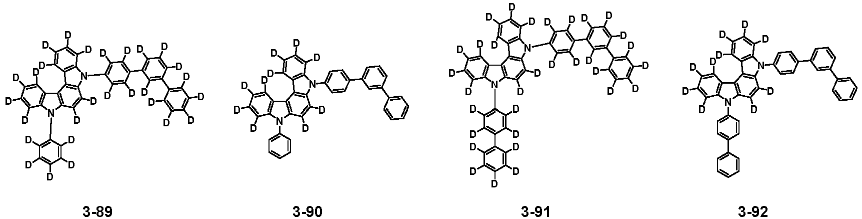

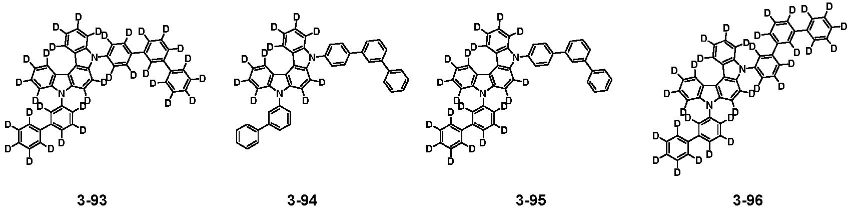

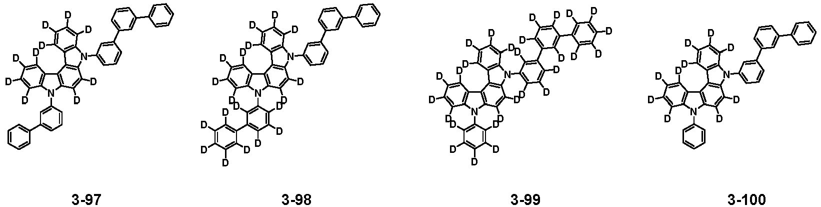

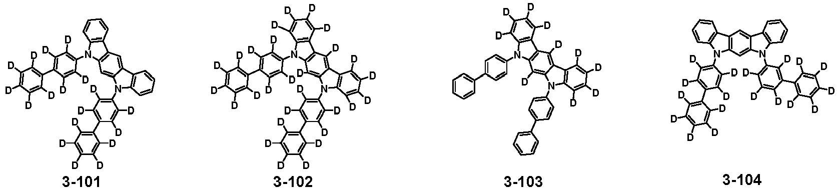

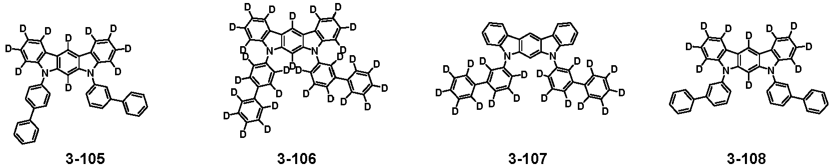

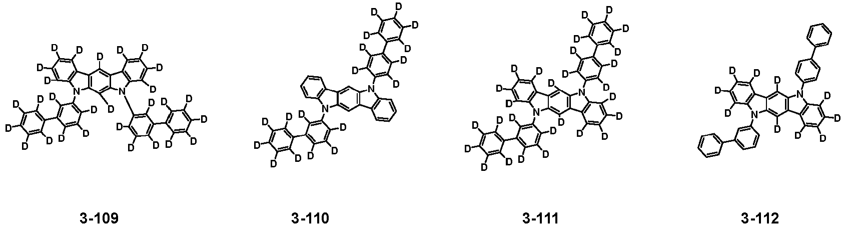

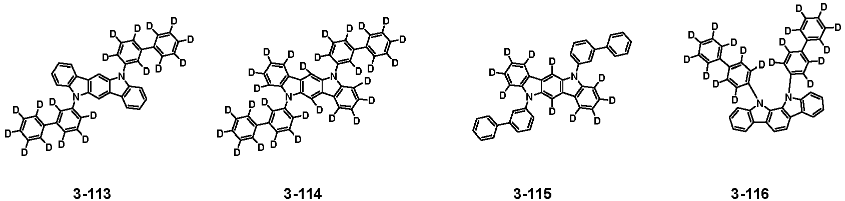

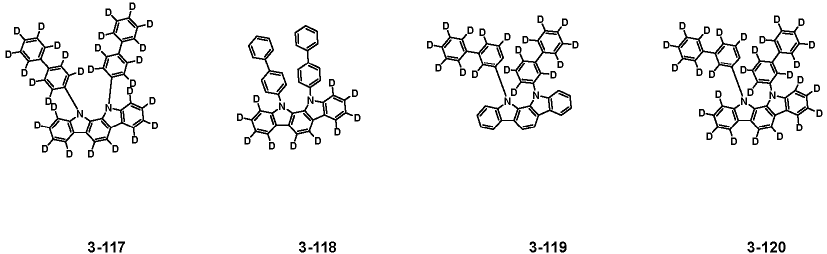

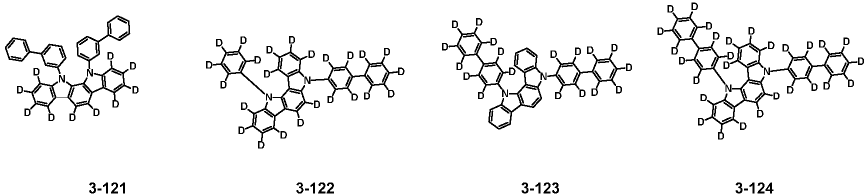

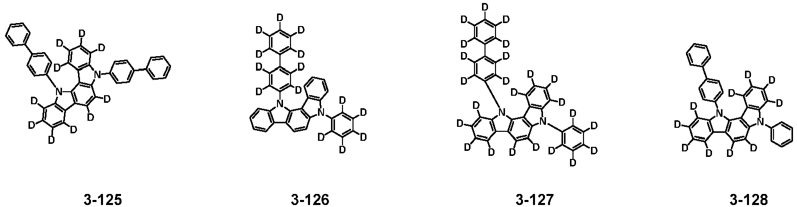

In addition, another embodiment of the present application provides a composition for an organic material layer, the composition including: the heterocyclic compound represented by Chemical Formula 1; and the heterocyclic compound represented by Chemical Formula 2 or Chemical Formula 3.

Specific descriptions on the heterocyclic compound represented by Chemical Formula 1, and the heterocyclic compound represented by Chemical Formula 2 or Chemical Formula 3 are the same as the descriptions provided above.

In the composition, the heterocyclic compound represented by Chemical Formula 1 and the heterocyclic compound represented by Chemical Formula 2 or Chemical Formula 3 may have a weight ratio of 1:10 to 10:1, 1:8 to 8:1, 1:5 to 5:1 or 1:2 to 2:1, however, the ratio is not limited thereto.

The composition may be used when forming an organic material of an organic light emitting device, and particularly, may be more preferably used when forming a host of a light emitting layer.

The composition has a form in which two or more compounds are simply mixed, and materials in a powder state may be mixed before forming an organic material layer of an organic light emitting device, or compounds in a liquid state at a proper temperature or higher may be mixed. The composition is in a solid state at a melting point of each material or lower, and may be kept as a liquid when adjusting a temperature.

The composition may further include materials known in the art such as solvents and additives.

The organic light emitting device according to one embodiment of the present application may be manufactured using common organic light emitting device manufacturing methods and materials except that one or more organic material layers are formed using the heterocyclic compound represented by Chemical Formula 1 and the heterocyclic compound represented by Chemical Formula 2 or Chemical Formula 3 described above.

The compound represented by Chemical Formula 1 and the heterocyclic compound represented by Chemical Formula 2 or Chemical Formula 3 may be formed into the organic material layer using a solution coating method as well as a vacuum deposition method when the organic light emitting device is manufactured. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may also be formed in a multilayer structure in which two or more organic material layers are laminated. For example, the organic light emitting device of the present disclosure may have a structure including a hole injection layer, a hole transport layer, a light emitting layer, an electron transport layer, an electron injection layer and the like as the organic material layer. However, the structure of the organic light emitting device is not limited thereto, and may include a smaller number of organic material layers.

In one embodiment of the present application, the organic light emitting device may be a blue organic light emitting device, and the heterocyclic compound according to Chemical Formula 1 and the heterocyclic compound represented by Chemical Formula 2 or Chemical Formula 3 may be used as a material of the blue organic light emitting device.

In one embodiment of the present application, the organic light emitting device may be a green organic light emitting device, and the compound represented by Chemical Formula 1 and the heterocyclic compound represented by Chemical Formula 2 or Chemical Formula 3 may be used as a material of the green organic light emitting device.

In one embodiment of the present application, the organic light emitting device may be a red organic light emitting device, and the compound represented by Chemical Formula 1 and the heterocyclic compound represented by Chemical Formula 2 or Chemical Formula 3 may be used as a material of the red organic light emitting device.

The organic light emitting device of the present disclosure may further include one, or two or more layers selected from the group consisting of a light emitting layer, a hole injection layer, a hole transport layer, a hole transport auxiliary layer, an electron injection layer, an electron transport layer, an electron blocking layer and a hole blocking layer.

One embodiment of the present application provides an organic light emitting device, wherein the organic material layer includes at least one of a hole blocking layer, an electron injection layer and an electron transport layer, and at least one of the hole blocking layer, the electron injection layer and the electron transport layer includes the heterocyclic compound represented by Chemical Formula 1 and the heterocyclic compound represented by Chemical Formula 2 or Chemical Formula 3.

One embodiment of the present application provides an organic light emitting device, wherein the organic material layer includes a light emitting layer, and the light emitting layer includes the heterocyclic compound represented by Chemical Formula 1 and the heterocyclic compound represented by Chemical Formula 2 or Chemical Formula 3.

One embodiment of the present application provides an organic light emitting device, wherein the organic material layer includes a light emitting layer, the light emitting layer includes a host material, and the host material includes the heterocyclic compound represented by Chemical Formula 1 and the heterocyclic compound represented by Chemical Formula 2 or Chemical Formula 3.

FIGS. 1 to 3 illustrate a lamination order of electrodes and organic material layers of the organic light emitting device according to one embodiment of the present disclosure. However, it is not intended that the scope of the present application be limited by these drawings, and structures of organic light emitting devices known in the art may also be applied to the present application.

FIG. 1 illustrates an organic light emitting device in which a positive electrode 200, an organic material layer 300 and a negative electrode 400 are sequentially laminated on a substrate 100. However, the structure is not limited only to such a structure, and as illustrated in FIG. 2, an organic light emitting device in which a negative electrode, an organic material layer and a positive electrode are sequentially laminated on a substrate may also be obtained.

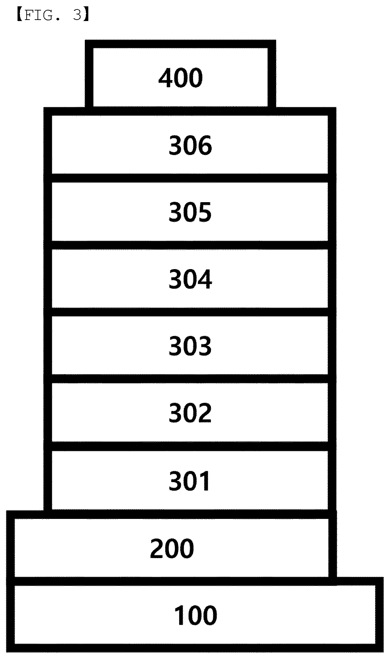

FIG. 3 illustrates a case of the organic material layer being a multilayer. An organic light emitting device according to FIG. 3 includes a hole injection layer 301, a hole transport layer 302, a light emitting layer 303, a hole blocking layer 304, an electron transport layer 305 and an electron injection layer 306. However, the scope of the present application is not limited by such a lamination structure, and as necessary, the layers other than the light emitting layer may not be included, and other necessary functional layers may be further added.

One embodiment of the present disclosure provides a method for manufacturing an organic light emitting device, the method including: preparing a substrate; forming a first electrode on the substrate; forming one or more organic material layers on the first electrode; and forming a second electrode on the organic material layers, wherein the forming of organic material layers includes forming the one or more organic material layers using the composition for an organic material layer according to one embodiment of the present disclosure.

In one embodiment of the present disclosure, the forming of organic material layers may be forming the organic material layers using a thermal vacuum deposition method after pre-mixing the heterocyclic compound represented by Chemical Formula 1.

The pre-mixing means, before depositing the heterocyclic compound represented by Chemical Formula 1 on the organic material layer, putting and mixing the materials first in one source of supply.

The pre-mixed material may be referred to as the composition for an organic material layer according to one embodiment of the present application.

The organic material layer including the heterocyclic compound represented by Chemical Formula 1 may further include other materials as necessary.

In the organic light emitting device according to one embodiment of the present disclosure, materials other than the heterocyclic compound represented by Chemical Formula 1 are illustrated below, however, these are for illustrative purposes only and not for limiting the scope of the present application, and these materials may be replaced by materials known in the art.

As the positive electrode material, materials each having a relatively large work function may be used, and transparent conductive oxides, metals, conductive polymers or the like may be used. Specific examples of the positive electrode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the negative electrode material, materials each having a relatively small work function may be used, and metals, metal oxides, conductive polymers or the like may be used. Specific examples of the negative electrode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection layer material, known hole injection layer materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], conductive polymers having solubility such as polyaniline/dodecylbenzenesulfonic acid or poly(3,4-ethylenedioxythiophene)/poly(4-styrenesulfonate), polyaniline/camphor sulfonic acid or polyaniline/poly(4-styrenesulfonate), and the like, may be used.

As the hole transport layer material, pyrazoline derivatives, arylamine-based derivatives, stilbene derivatives, triphenyldiamine derivatives and the like may be used, and low molecular or high molecular materials may also be used.

As the electron transport layer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials as well as low molecular materials may also be used.

As examples of the electron injection layer material, LiF is typically used in the art, however, the present application is not limited thereto.

As the light emitting layer material, red, green or blue light emitting materials may be used, and as necessary, two or more light emitting materials may be mixed and used. Herein, the two or more light emitting materials may be deposited as individual sources of supply or pre-mixed and deposited as one source of supply when used. In addition, fluorescent materials may also be used as the light emitting layer material, however, phosphorescent materials may also be used. As the light emitting layer material, materials emitting light alone by binding holes and electrons injected from a positive electrode and a negative electrode, respectively, may be used, however, materials having a host material and a dopant material involved in light emission together may also be used.

When hosts of the light emitting layer material are mixed and used, same series hosts may be mixed and used, or different series hosts may be mixed and used. For example, any two or more types of materials among n-type host materials and p-type host materials may be selected and used as a host material of a light emitting layer.

The organic light emitting device according to one embodiment of the present disclosure may be a top-emission type, a bottom-emission type or a dual-emission type depending on the materials used.

The heterocyclic compound according to one embodiment of the present disclosure may also be used in an organic electronic device including an organic solar cell, an organic photo conductor, an organic transistor and the like under a principle similar to that in the organic light emitting device.

Hereinafter, preferred examples are provided to help to understand the present disclosure, however, the following examples are only provided to more readily understand the present disclosure, and the present disclosure is not limited thereto.

### <Preparation Example>

### <Preparation Example 1> Preparation of Compound 1-1

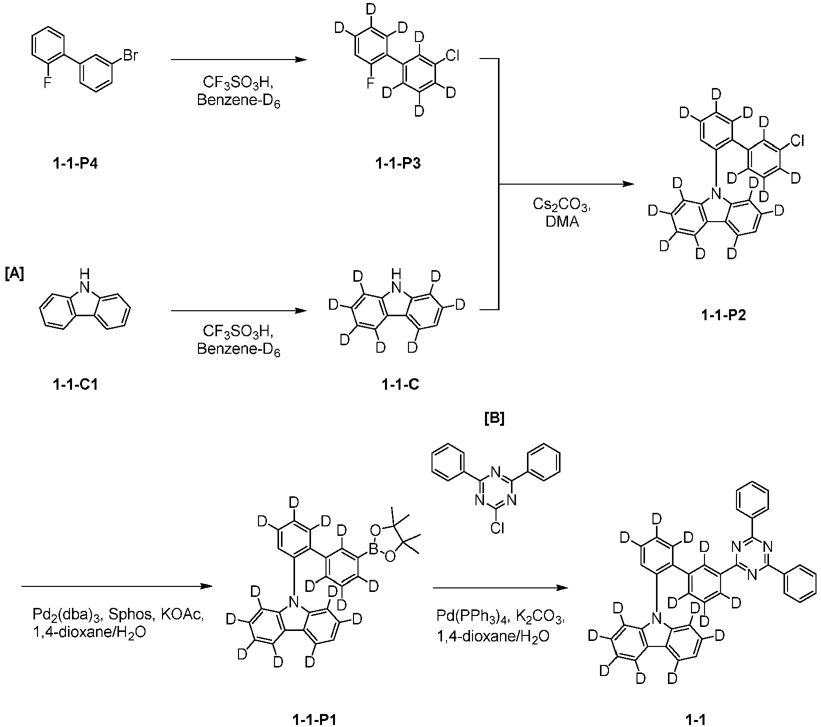

### 1) Preparation of Compound 1-1-P3

1-1-P4 (10 g, 48.4 mmol) was dissolved in benzene-d6 (500 mL) and then in CF₃SO₃H (123 g, 822.7 mmol), and the solution was refluxed for 1 hour at 60°C. After the reaction was completed, the reaction solution was neutralized with D₂O and Na₂CO₃. After the neutralization, distilled water and ethyl acetate were introduced to the mixture solution to perform extraction. The organic layer was dried with MgSO₄, and then the solvent was removed using a rotary evaporator. The reaction material was purified by column chromatography (DCM (dichloromethane) :Hex (hexane)=1:4), and recrystallized with methanol to obtain target Compound 1-1-P3 (9.56 g, 92%).

### 1-1) Preparation of Compound 1-1-C

9H-carbazole [A] (10 g, 59.8 mmol) was dissolved in benzene-d6 (500 mL) and then in CF₃SO₃H (153 g, 1017 mmol), and the solution was refluxed for 1 hour at 60°C. After the reaction was completed, the reaction solution was neutralized with D₂O and Na₂CO₃. After the neutralization, distilled water and ethyl acetate were introduced to the mixture solution to perform extraction. The organic layer was dried with MgSO₄, and then the solvent was removed using a rotary evaporator. The reaction material was purified by column chromatography (DCM:Hex=1:4), and recrystallized with methanol to obtain target Compound 1-1-C (9.85 g, 94%).

### 2) Preparation of Compound 1-1-P2

After dissolving Compound 1-1-P3 (9.56 g, 44.5 mmol) and 1-1-C (7.80 g, 44.5 mmol) in DMA (dimethylacetamide) (95 mL), Cs₂CO₃ (14.5 g, 44.5 mmol) was introduced thereto, and then the mixture was refluxed for 3 hours at 180°C. After the reaction was completed, a solid was obtained using H₂O. The solid compound was purified by column chromatography (DCM:Hex=1:2), and recrystallized with methanol to obtain target Compound 1-1-P2 (12.6 g, 76.4%).

### 3) Preparation of Compound 1-1-P1

Compound 1-1-P2 (12.6 g, 34.1 mmol), 4,4,4',4',5,5,5',5'-octamethyl-2,2'-bi(1,3,2-dioxaborolane) (13.0 g, 51.15 mmol), Pd₂(dba)₃ (tris(dibenzylideneacetone)dipalladium(0)) (1.97 g, 1.71 mmol), Sphos (2-dicyclohexylphosphino-2',6'-dimethoxybiphenyl) (1.40 g, 3.41 mmol) and KOAc (potassium acetate) (6.62 g, 68.2 mmol) were dissolved in 1,4-dioxane (120 mL), and then the mixture was stirred under reflux for 6 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:2) to obtain target Compound 1-1-P1 (15.1 g, 96.2%).

### 4) Preparation of Compound 1-1

Compound 1-1-P1 (15.1 g, 32.7 mmol), 2-chloro-4,6-diphenyl-1,3,5-triazine (8.75 g, 32.7 mmol), Pd(PPh₃)₄ (1.89 g, 1.64 mmol) and K₂CO₃ (9.04 g, 65.4 mmol) were dissolved in 1,4-dioxane/H₂O (150 mL/45 mL), and then the mixture was refluxed for 24 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:3), and recrystallized with methanol to obtain target Compound 1-1 (16.3 g, 88%).

### <Preparation Example 2> Preparation of Compounds 1-4, 1-9, 1-13, 1-15, 1-25, 1-29, 1-32, 1-34, 1-43, 1-45, 1-49, 1-56, 1-57, 1-59, 1-61, 1-62, 1-63, 1-69, 1-77, 1-78, 1-86 and 1-115

Target compounds were synthesized in the same manner as in Preparation Example 1, except that Intermediate A of the following Table 1 was used instead of 9H-carbazole, and Intermediate B of the following Table 1 was used instead of 2-chloro-4,6-diphenyl-1,3,5-triazine.

**[Table 1]**

| Compoun d No. | Intermediate A | Intermediate B | Target Compound |
|---|---|---|---|
| 1-4 | 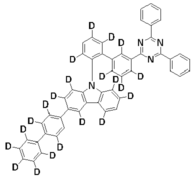 | 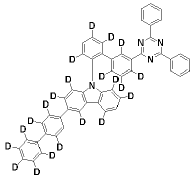 | 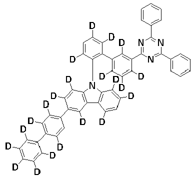 |
| 1-9 | 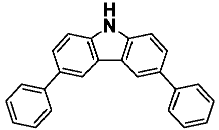 | 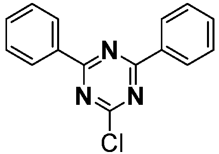 | 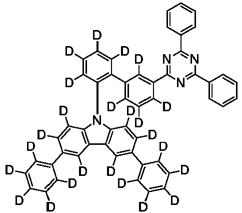 |
| 1-13 | 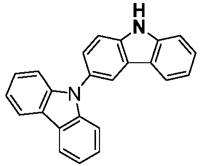 | 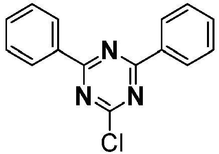 | 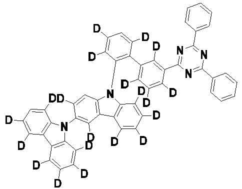 |
| 1-15 | 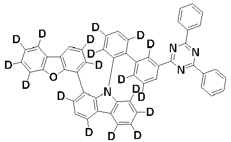 | 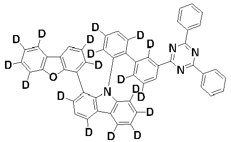 | 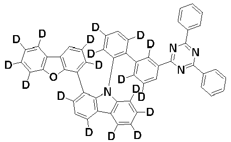 |
| 1-25 | 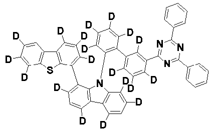 | 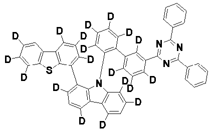 | 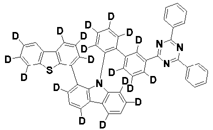 |
| 1-29 | 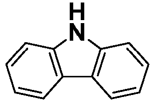 | 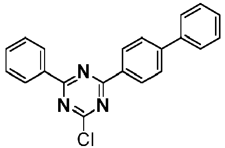 | 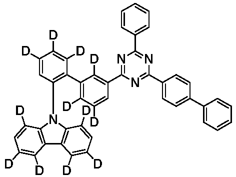 |
| 1-32 | 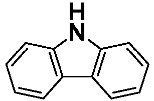 | 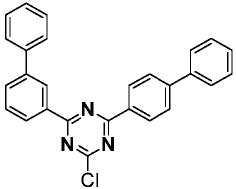 | 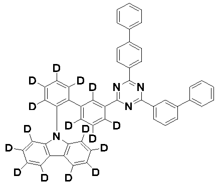 |
| 1-34 | 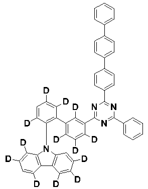 | 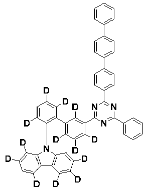 | 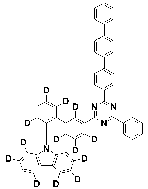 |
| 1-43 | 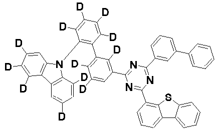 | 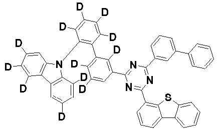 | 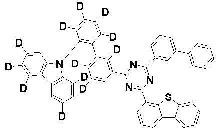 |
| 1-45 | 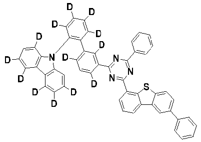 | 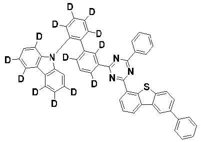 | 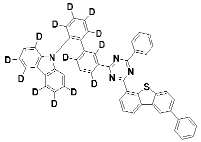 |
| 1-49 | 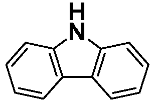 | 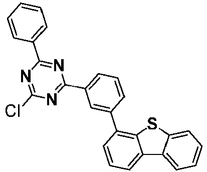 | 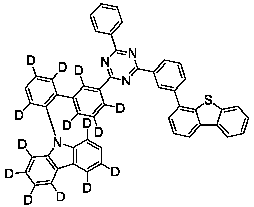 |
| 1-56 | 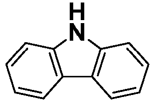 | 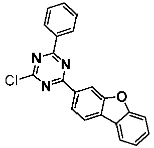 | 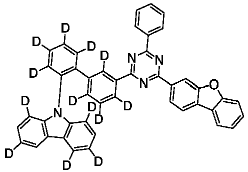 |
| 1-57 | 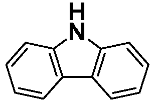 | 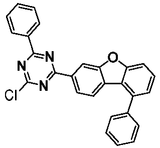 | 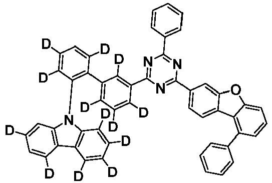 |
| 1-59 | 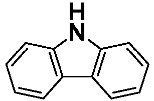 | 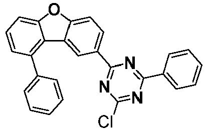 | 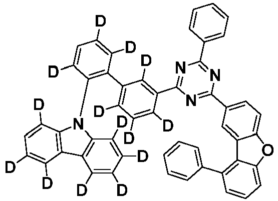 |
| 1-61 | 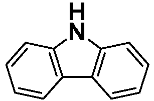 | 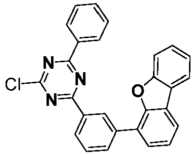 | 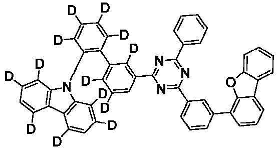 |
| 1-62 | 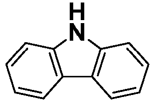 | 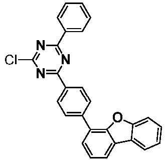 | 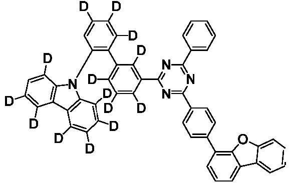 |
| 1-63 | 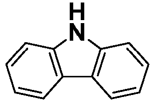 | 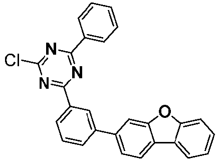 | 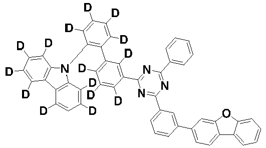 |
| 1-69 | 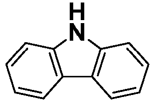 | 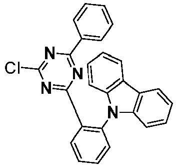 | 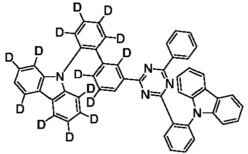 |
| 1-77 | 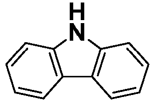 | 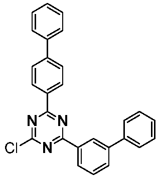 | 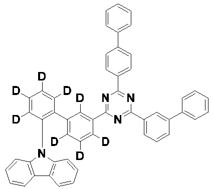 |
| 1-78 | 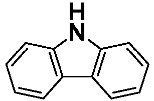 | 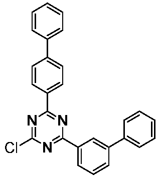 | 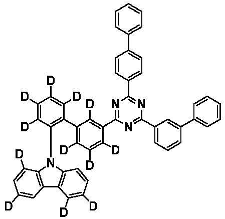 |
| 1-86 | 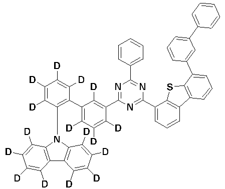 | 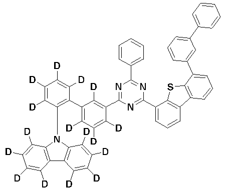 | 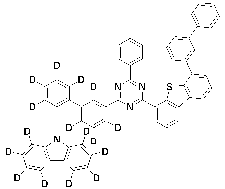 |
| 1-115 | 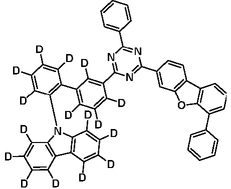 | 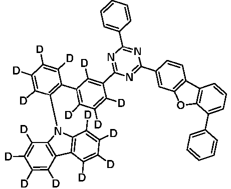 | 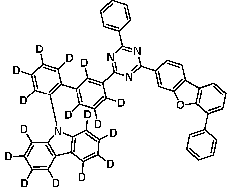 |

### <Preparation Example 3> Preparation of Compound 1-76

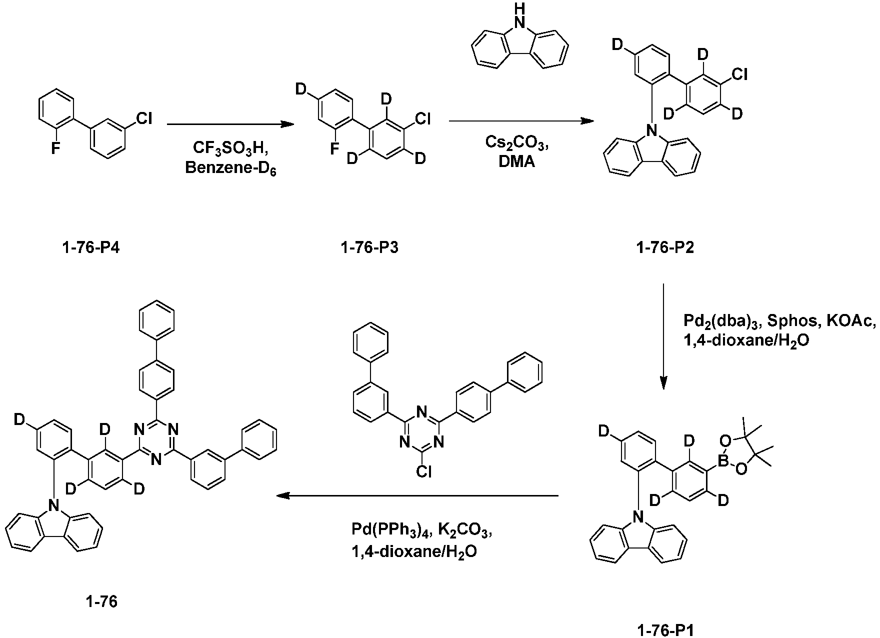

### 1) Preparation of Compound 1-76-P3

Compound 1-76-P4 (10 g, 48.4 mmol) was dissolved in benzene-d6 (100 mL) and then in CF₃SO₃H (25 g, 165 mmol), and the solution was refluxed for 1 hour at 60°C. After the reaction was completed, the reaction solution was neutralized with D₂O and Na₂CO₃. After the neutralization, distilled water and ethyl acetate were introduced to the mixture solution to perform extraction. The organic layer was dried with MgSO₄, and then the solvent was removed using a rotary evaporator. The reaction material was purified by column chromatography (DCM:Hex=1:4), and recrystallized with methanol to obtain target Compound 1-76-P3 (9.30 g, 91.2%).

### 2) Preparation of Compound 1-76-P2

After dissolving Compound 1-76-P3 (9.3 g, 44.1 mmol) and 9H-carbazole (7.58 g, 44.1 mmol) in DMA (95 mL), Cs₂CO₃ (14.1 g, 44.5 mmol) was introduced thereto, and then the mixture was refluxed for 3 hours at 180°C. After the reaction was completed, a solid was obtained using H₂O. The solid compound was purified by column chromatography (DCM:Hex=1:2), and recrystallized with methanol to obtain target Compound 1-76-P2 (11.9 g, 75.5%).

### 3) Preparation of Compound 1-76-P1

Compound 1-76-P2 (11.9 g, 33.3 mmol), 4,4,4',4',5,5,5',5'-octamethyl-2,2'-bi(1,3,2-dioxaborolane) (12.70 g, 50.0 mmol), Pd₂(dba)₃ (1.93 g, 1.67 mmol), Sphos (1.37 g, 3.33 mmol) and KOAc (6.47 g, 66.6 mmol) were dissolved in 1,4-dioxane (120 mL), and then the mixture was stirred under reflux for 6 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:2) to obtain target Compound 1-76-P1 (14.5 g, 94.8%).

### 4) Preparation of Compound 1-76

Compound 1-76-P1 (14.5 g, 31.4 mmol), 2-([1,1'-biphenyl]-3-yl)-4-([1,1'-biphenyl]-4-yl)-6-chloro-1,3,5-triazine (13.7 g, 32.7 mmol), Pd(PPh₃)₄ (1.89 g, 1.64 mmol) and K₂CO₃ (9.04 g, 65.4 mmol) were dissolved in 1,4-dioxane/H₂O (150 mL/45 mL), and then the mixture was refluxed for 24 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:3), and recrystallized with methanol to obtain target Compound 1-76 (20.4 g, 89.4%).

### <Preparation Example 4> Preparation of Compounds 1-121, 1-124, 1-131 and 1-134

Target compounds were synthesized in the same manner as in Preparation Example 3, except that Intermediate A of the following Table 2 was used instead of 9H-carbazole, and Intermediate B of the following Table 2 was used instead of 2-([1,1'-biphenyl]-3-yl)-4-([1,1'-biphenyl]-4-yl)-6-chloro-1,3,5-triazine.

**[Table 2]**

| Compound No. | Intermediate A | Intermediate B | Target Compound |
|---|---|---|---|
| 1-121 | 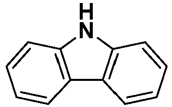 | 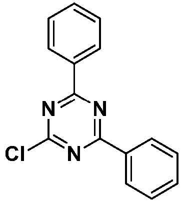 | 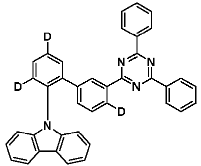 |
| 1-124 | 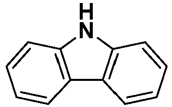 | 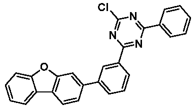 | 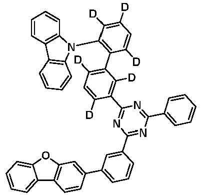 |
| 1-131 | 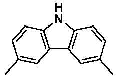 | 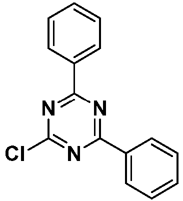 | 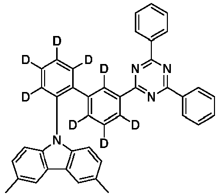 |
| 1-134 | 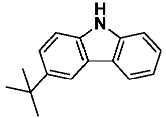 | 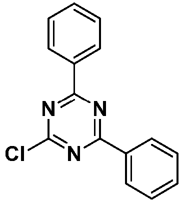 | 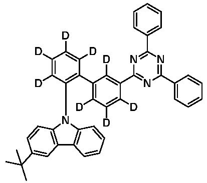 |

### <Preparation Example 5> Preparation of Compound 1-135

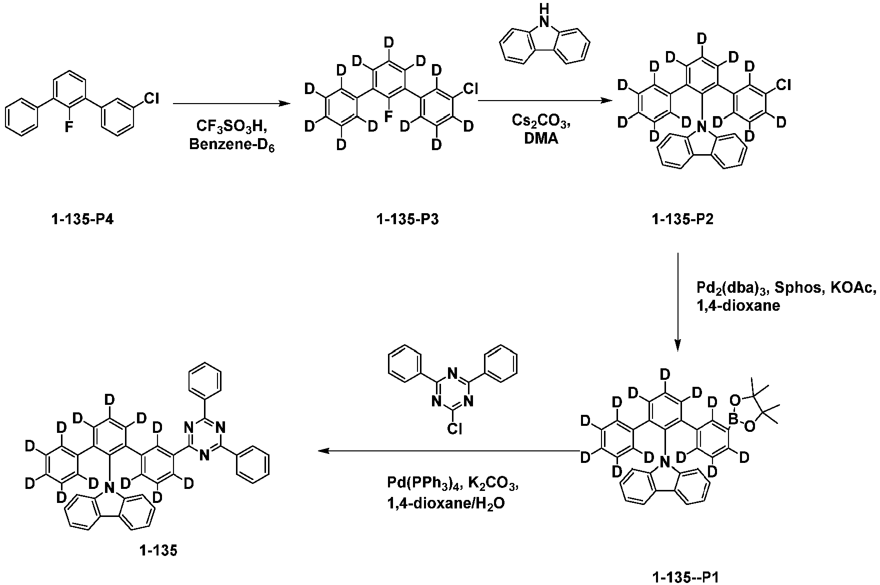

### 1) Preparation of Compound 1-135-P3

Compound 1-135-P4 (10 g, 35.4 mmol) was dissolved in benzene-d6 (500 mL) and then in CF₃SO₃H (90.3 g, 601.8 mmol), and the solution was refluxed for 1 hour at 60°C. After the reaction was completed, the reaction solution was neutralized with D₂O and Na₂CO₃. After the neutralization, distilled water and ethyl acetate were introduced to the mixture solution to perform extraction. The organic layer was dried with MgSO₄, and then the solvent was removed using a rotary evaporator. The reaction material was purified by column chromatography (DCM:Hex=1:4), and recrystallized with methanol to obtain target Compound 1-135-P3 (9.69 g, 93%).

### 2) Preparation of Compound 1-135-P2

After dissolving Compound 1-135-P3 (9.69 g, 32.9 mmol) and 9H-carbazole (5.50 g, 32.9 mmol) in DMA (97 mL), Cs₂CO₃ (10.7 g, 32.9 mmol) was introduced thereto, and then the mixture was refluxed for 3 hours at 180°C. After the reaction was completed, a solid was obtained using H₂O. The solid compound was purified by column chromatography (DCM:Hex=1:2), and recrystallized with methanol to obtain target Compound 1-135-P2 (12.1 g, 88.3%).

### 3) Preparation of Compound 1-135-P1

Compound 1-135-P2 (12.1 g, 27.4 mmol), 4,4,4' ,4' ,5,5,5' ,5'-octamethyl-2,2'-bi(1,3,2-dioxaborolane) (10.4 g, 41.1 mmol), Pd₂(dba)₃ (1.25 g, 1.37 mmol), Sphos (1.12 g, 2.74 mmol) and KOAc (5.32 g, 54.8 mmol) were dissolved in 1,4-dioxane (120 mL), and then the mixture was stirred under reflux for 6 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:2) to obtain target Compound 1-135-P1 (14.0 g, 95.9%).

### 4) Preparation of Compound 1-135

Compound 1-135-P1 (14.0 g, 26.2 mmol), 2-chloro-4,6-diphenyl-1,3,5-triazine (7.01 g, 26.2 mmol), Pd(PPh₃)₄ (1.5 g, 1.31 mmol) and K₂CO₃ (7.24 g, 52.4 mmol) were dissolved in 1,4-dioxane/H₂O (150 mL/45 mL), and then the mixture was refluxed for 24 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:3), and recrystallized with methanol to obtain target Compound 1-135 (14.7 g, 87.7%).

### <Preparation Example 6> Preparation of Compounds 1-136, 1-140 and 1-149

Target compounds were synthesized in the same manner as in Preparation Example 5, except that Intermediate A of the following Table 3 was used instead of 9H-carbazole, and Intermediate B of the following Table 3 was used instead of 2-chloro-4,6-diphenyl-1,3,5-triazine.

**[Table 3]**

| Compoun d No. | Intermediate A | Intermediate B | Target Compound |
|---|---|---|---|
| 1-136 | 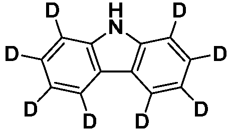 | 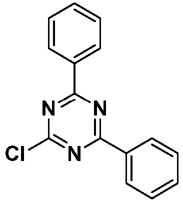 | 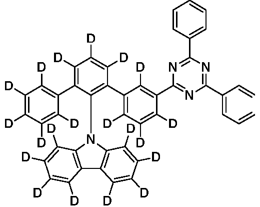 |
| 1-140 | 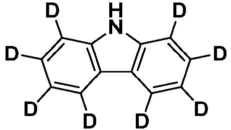 | 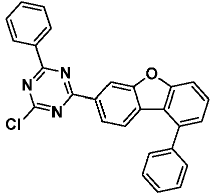 | 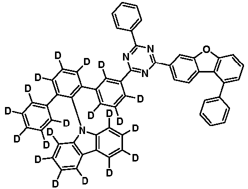 |
| 1-149 | 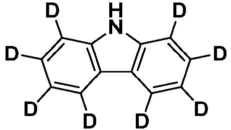 | 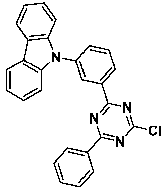 | 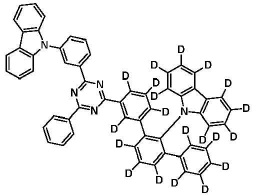 |

### <Preparation Example 7> Preparation of Compound 1-146

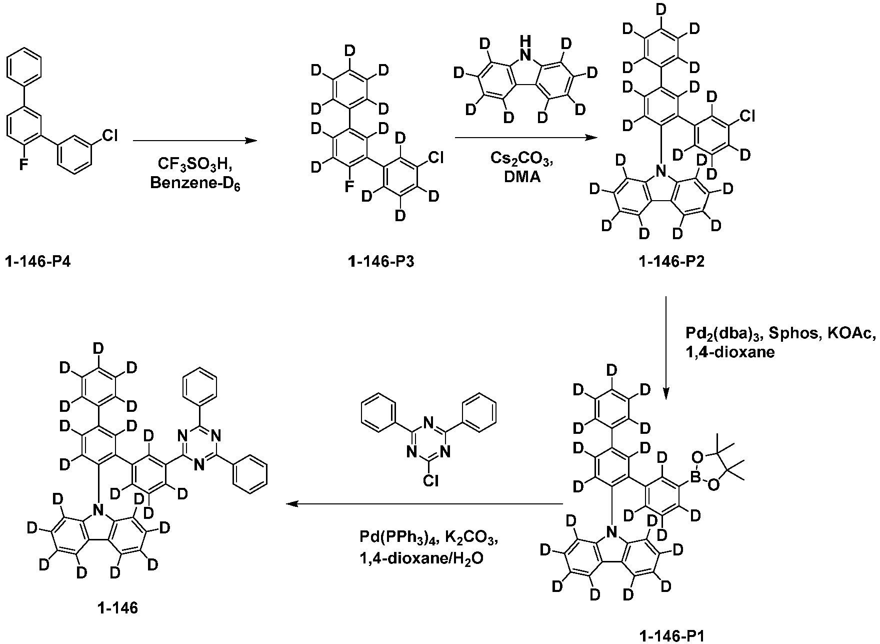

### 1) Preparation of Compound 1-146-P3

Compound 1-146-P4 (10 g, 35.4 mmol) was dissolved in benzene-d6 (500 mL) and then in CF₃SO₃H (90.3 g, 601.8 mmol), and the solution was refluxed for 1 hour at 60°C. After the reaction was completed, the reaction solution was neutralized with D₂O and Na₂CO₃. After the neutralization, distilled water and ethyl acetate were introduced to the mixture solution to perform extraction. The organic layer was dried with MgSO₄, and then the solvent was removed using a rotary evaporator. The reaction material was purified by column chromatography (DCM:Hex=1:4), and recrystallized with methanol to obtain target Compound 1-146-P3 (10.1 g, 97%).

### 2) Preparation of Compound 1-146-P2

After dissolving 1-146-P3 (10.1 g, 34.3 mmol) and 9H-carbazole-1,2,3,4,5,6,7,8-d8 (6.01 g, 34.3 mmol) in DMA (97 mL), Cs₂CO₃ (11.2 g, 34.3 mmol) was introduced thereto, and then the mixture was refluxed for 3 hours at 180°C. After the reaction was completed, a solid was obtained using H₂O. The solid compound was purified by column chromatography (DCM:Hex=1:2), and recrystallized with methanol to obtain target Compound 1-146-P2 (13.1 g, 85.1%).

### 3) Preparation of Compound 1-146-P1

Compound 1-146-P2 (13.1 g, 29.1 mmol), 4,4,4' ,4' ,5,5,5' ,5'-octamethyl-2,2'-bi(1,3,2-dioxaborolane) (11.1 g, 43.7 mmol), Pd₂(dba)₃ (1.33 g, 1.45 mmol), Sphos (1.19 g, 2.90 mmol) and KOAc (5.65 g, 58.2 mmol) were dissolved in 1,4-dioxane (130 mL), and then the mixture was stirred under reflux for 6 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:2) to obtain target Compound 1-146-P1 (13.8 g, 87.5%).

### 4) Preparation of Compound 1-146

Compound 1-146-P1 (13.8 g, 25.5 mmol), 2-chloro-4,6-diphenyl-1,3,5-triazine (6.83 g, 25.5 mmol), Pd(PPh₃)₄ (1.48 g, 1.28 mmol) and K₂CO₃ (7.05 g, 51.0 mmol) were dissolved in 1,4-dioxane/H₂O (150 mL/45 mL), and then the mixture was refluxed for 24 hours. After the reaction was completed, distilled water and DCM were introduced to the reaction mixture at room temperature to perform extraction. The organic layer was dried with MgSO₄, and then the solvent was removed using a rotary evaporator. The reaction material was purified by column chromatography (DCM:Hex=1:3), and recrystallized with methanol to obtain target Compound 1-146 (15.1 g, 91.6%).

### <Preparation Example 8> Preparation of Compounds 1-147, 1-148 and 1-151

Target compounds were synthesized in the same manner as in Preparation Example 7, except that Intermediate A of the following Table 4 was used instead of 9*H*-carbazole-1,2,3,4,5,6,7,8-d8, and Intermediate B of the following Table 4 was used instead of 2-chloro-4,6-diphenyl-1,3,5-triazine.

**[Table 4]**

| Compoun d No. | Intermediate A | Intermediate B | Target Compound |
|---|---|---|---|
| 1-147 | 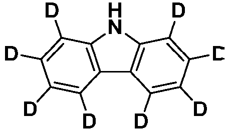 | 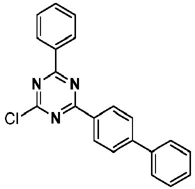 | 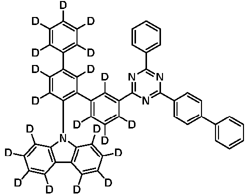 |
| 1-148 | 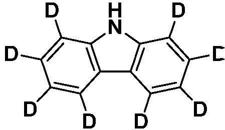 | 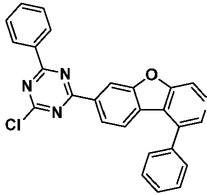 | 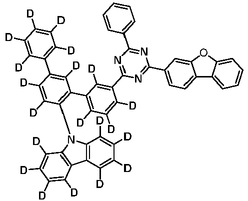 |
| 1-151 | 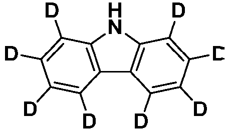 | 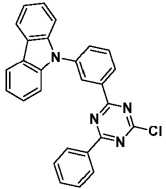 | 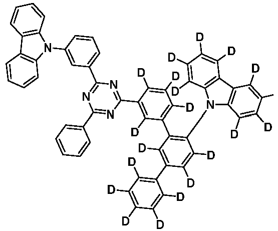 |

### <Preparation Example 9> Preparation of Compound 2-1

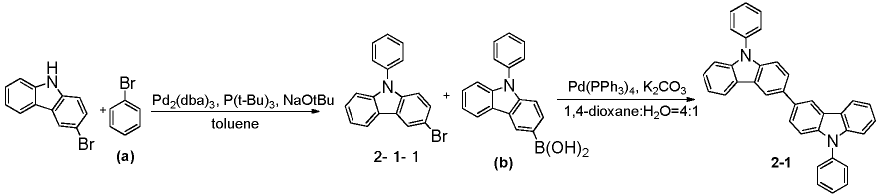

### 1) Preparation of Compound 2-1-1

After introducing 3-bromo-9H-carbazole (10 g, 49.59 mmol), 2-bromobenzene-1-ylium (a) (24.2 g, 148.77 mmol), tris(dibenzylideneacetone) dipalladium (Pd₂(dba)₃) (2.27 g, 2.48 mmol), tri-tert-butylphosphine (P(t-Bu)₃) (2.42 mL, 9.92 mmol) and sodium tert-butoxide (NatOBu) (9.53 g, 99.18 mmol) to a reaction flask, toluene (100 mL) was introduced thereto, and the mixture was heated for 15 hours at 135°C. When the reaction was finished, the reaction mixture was extracted with methylene chloride (MC) and water, and then purified by column chromatography to obtain Compound 2-1-1 (14 g, yield 98%).

### 2) Preparation of Compound 2-1

After introducing Compound 2-1-1 (14 g, 43.4 mmol), (9-phenyl-9H-carbazol-3-yl)boronic acid (b) (14.9 g, 52 mmol), tetrakis(triphenylphosphine)palladium(0) (Pd(PPh₃)₄) (2.5 g, 2.17 mmol) and potassium carbonate (K₂CO₃) (17.9 g, 130 mmol) to a reaction flask, 1,4-dioxane (140 mL) and distilled water (35 mL) were added thereto, and the mixture was stirred for 4 hours at 120°C.

After that, the temperature was lowered to room temperature, and a produced solid was washed with distilled water and methanol to obtain Compound 2-1 (17 g, yield 80%).

### <Preparation Example 10> Preparation of Compounds 2-2, 2-3, 2-4, 2-5, 2-6, 2-7, 2-11, 2-16, 2-19, 2-20, 2-21, 2-22, 2-23, 2-26, 2-27, 2-28, 2-29, 2-30, 2-32, 2-33, 2-34, 2-38, 2-40, 2-53, 2-54, 2-55, 2-57, 2-58, 2-60, 2-61, 2-62, 2-63, 2-64, 2-67, 2-69 and 2-72

Compounds 2-2, 2-3, 2-4, 2-5, 2-6, 2-7, 2-11, 2-16, 2-19, 2-20, 2-21, 2-22, 2-23, 2-26, 2-27, 2-28, 2-29, 2-30, 2-32, 2-33, 2-34, 2-38, 2-40, 2-53, 2-54, 2-55, 2-57, 2-58, 2-60, 2-61, 2-62, 2-63, 2-64, 2-67, 2-69 and 2-72 were synthesized in the same manner as in Preparation Example 9, except that Compound a of the following Table 5 was used instead of 2-bromobenzene-1-ylium (a), and Compound b of the following Table 5 was used instead of (9-phenyl-9H-carbazol-3-yl)boronic acid (b).

**[Table 5]**

| Compoun d No. | Compound a | Compound b | Target Compound (Yield%) |
|---|---|---|---|
| 2-2 | 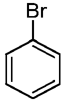 | 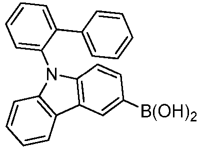 | 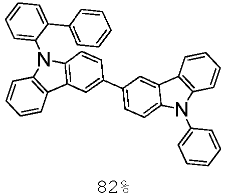 |
| 2-3 | 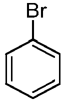 | 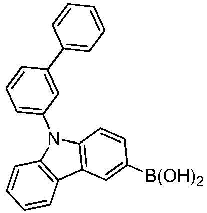 | 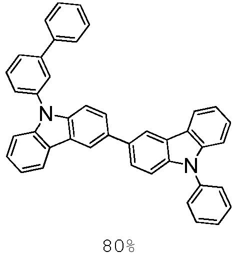 |
| 2-4 | 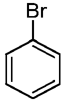 | 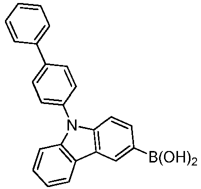 | 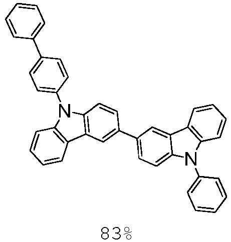 |
| 2-5 | 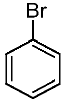 | 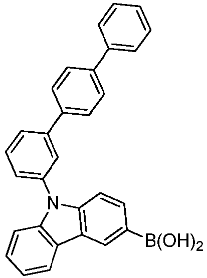 | 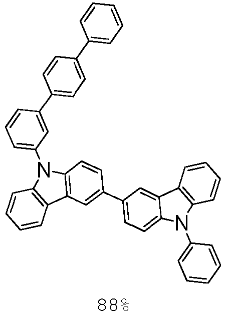 |
| 2-6 | 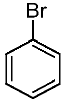 | 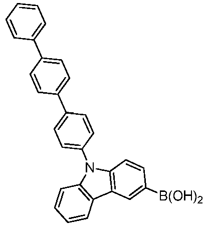 | 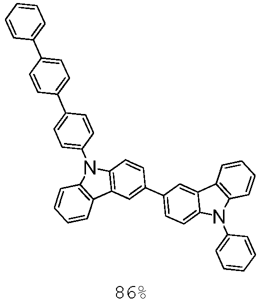 |
| 2-7 | 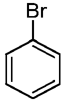 | 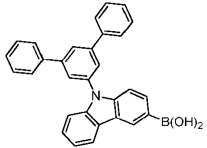 | 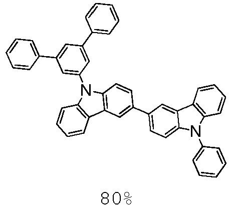 |
| 2-11 | 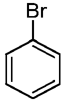 | 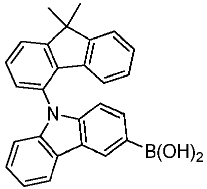 | 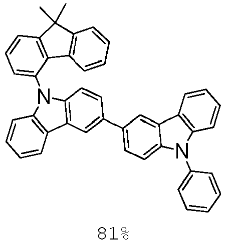 |
| 2-16 | 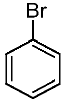 | 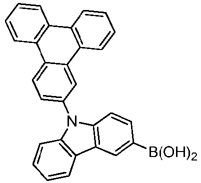 | 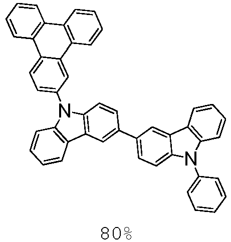 |
| 2-19 | 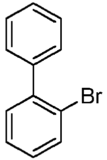 | 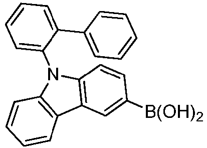 | 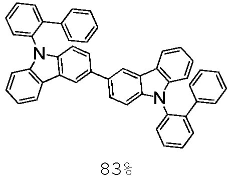 |
| 2-20 | 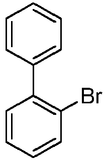 | 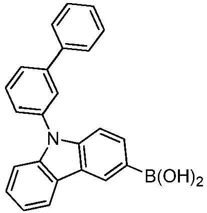 | 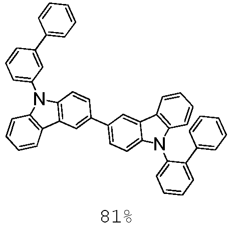 |
| 2-21 | 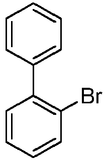 | 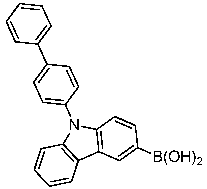 | 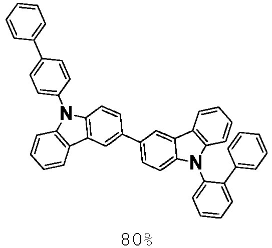 |
| 2-22 | 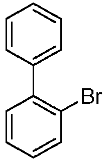 | 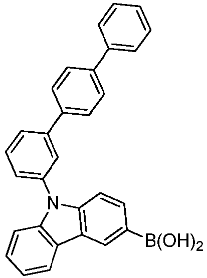 | 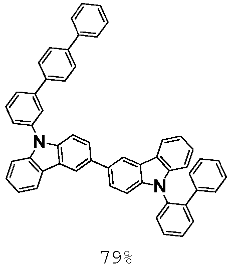 |
| 2-23 | 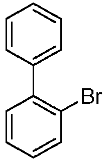 | 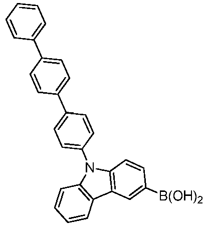 | 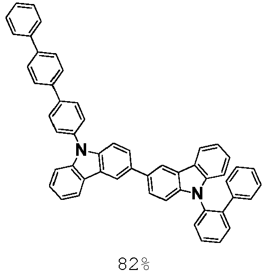 |
| 2-26 | 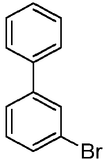 | 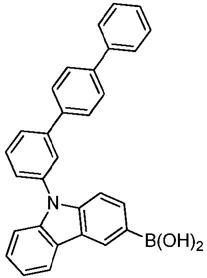 | 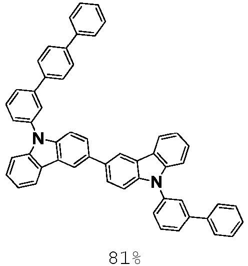 |
| 2-27 | 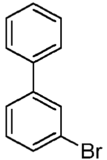 | 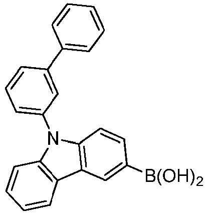 | 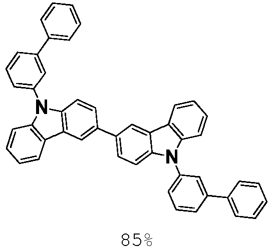 |
| 2-28 | 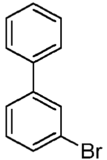 | 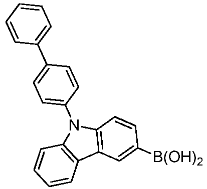 | 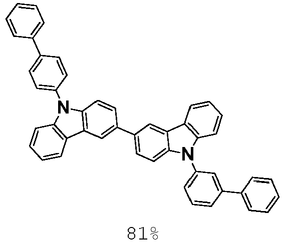 |
| 2-29 | 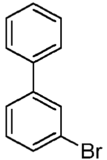 | 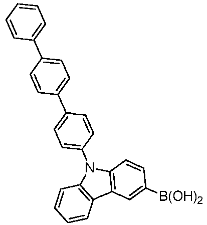 | 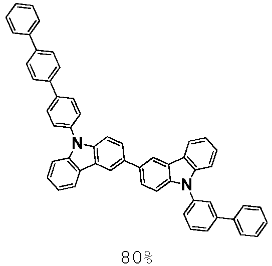 |
| 2-30 | 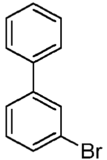 | 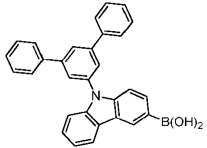 | 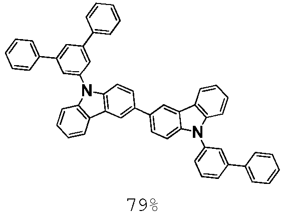 |
| 2-32 | 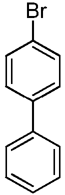 | 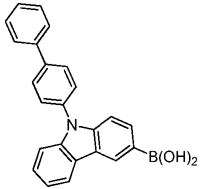 | 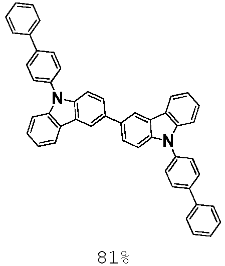 |
| 2-33 | 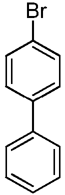 | 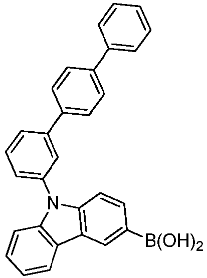 | 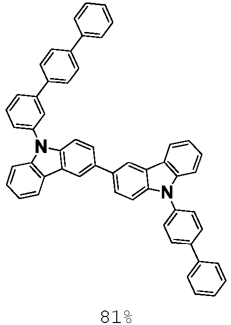 |
| 2-34 | 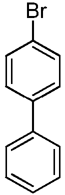 | 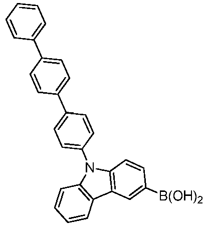 | 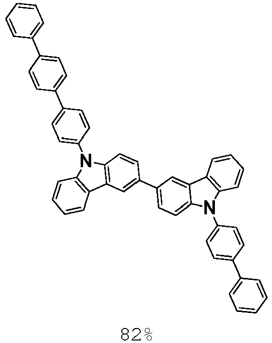 |
| 2-38 | 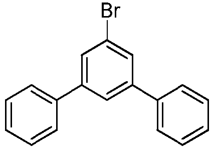 | 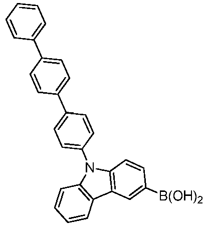 | 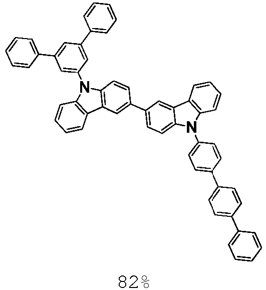 |
| 2-40 | 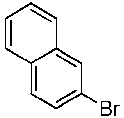 | 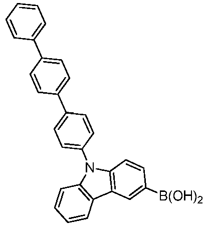 | 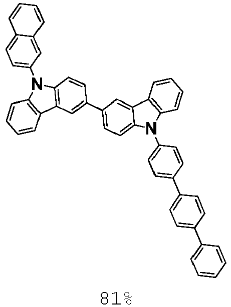 |
| 2-53 | 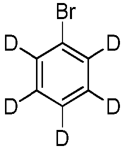 | 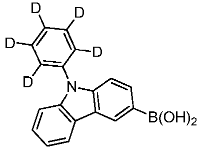 | 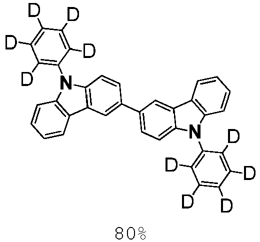 |
| 2-54 | 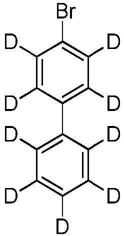 | 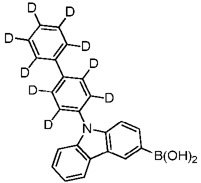 | 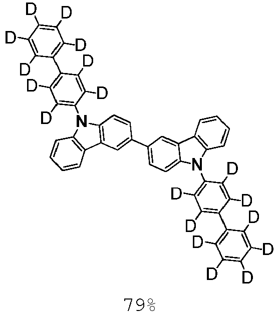 |
| 2-55 | 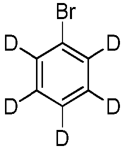 | 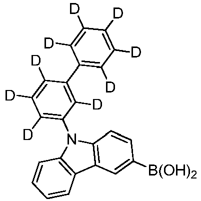 | 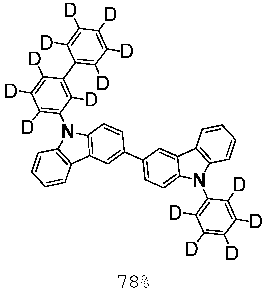 |
| 2-57 | 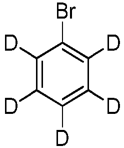 | 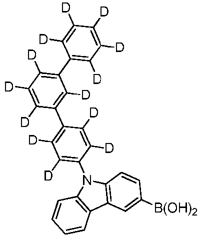 | 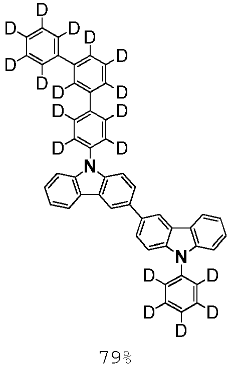 |
| 2-58 | 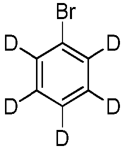 | 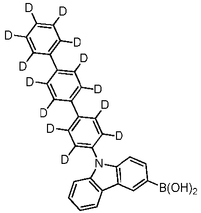 | 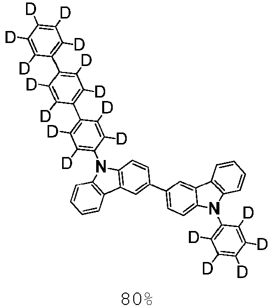 |
| 2-60 | 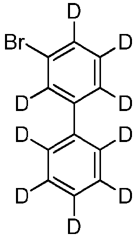 | 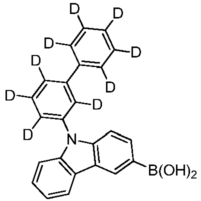 | 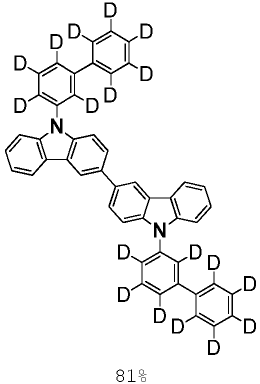 |
| 2-61 | 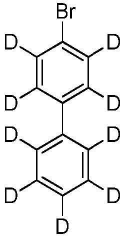 | 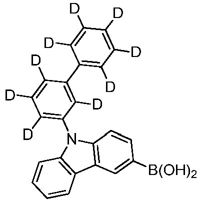 | 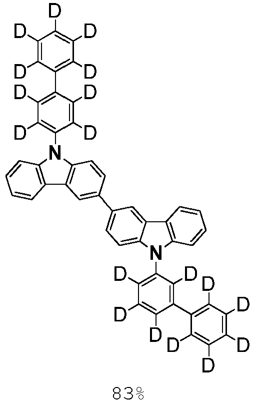 |
| 2-62 | 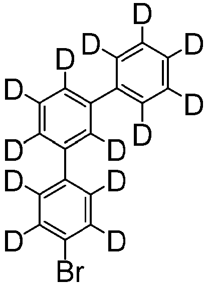 | 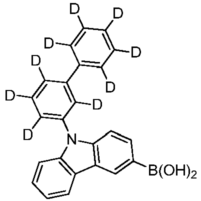 | 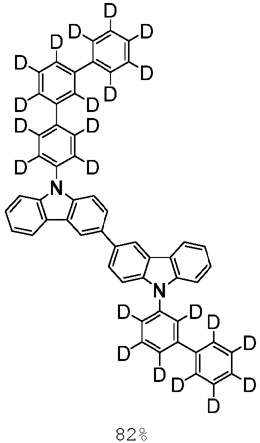 |
| 2-63 | 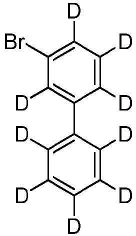 | 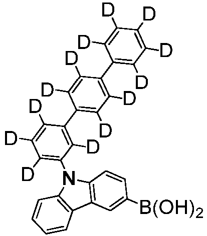 | 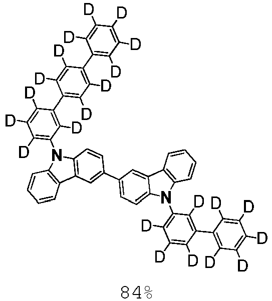 |
| 2-64 | 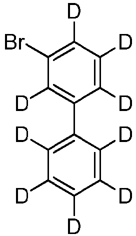 | 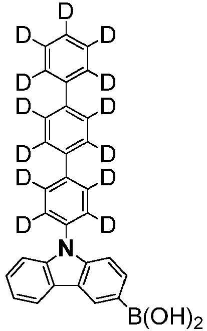 | 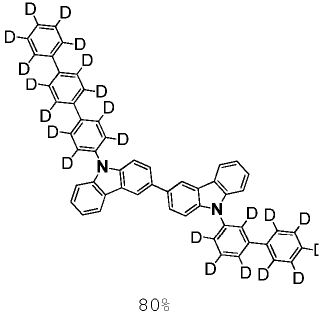 |
| 2-67 | 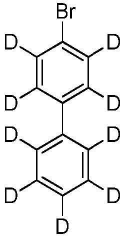 | 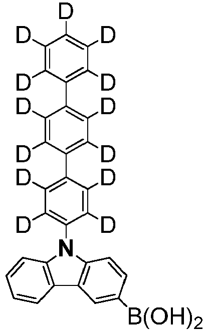 | 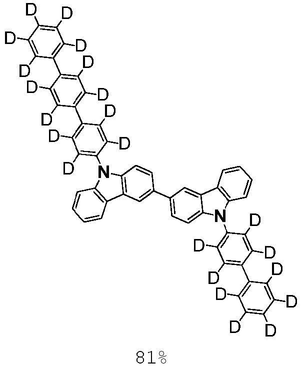 |
| 2-69 | 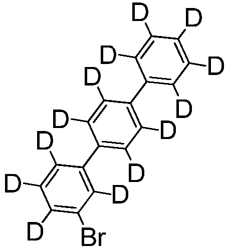 | 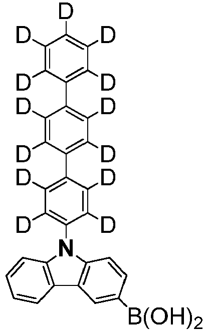 | 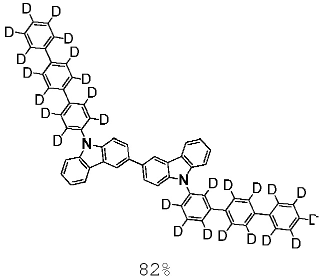 |
| 2-72 | 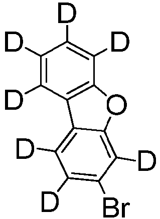 | 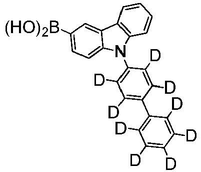 | 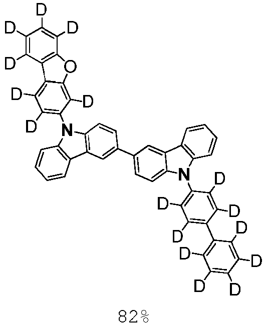 |

### <Preparation Example 11> Preparation of Compound 2-73

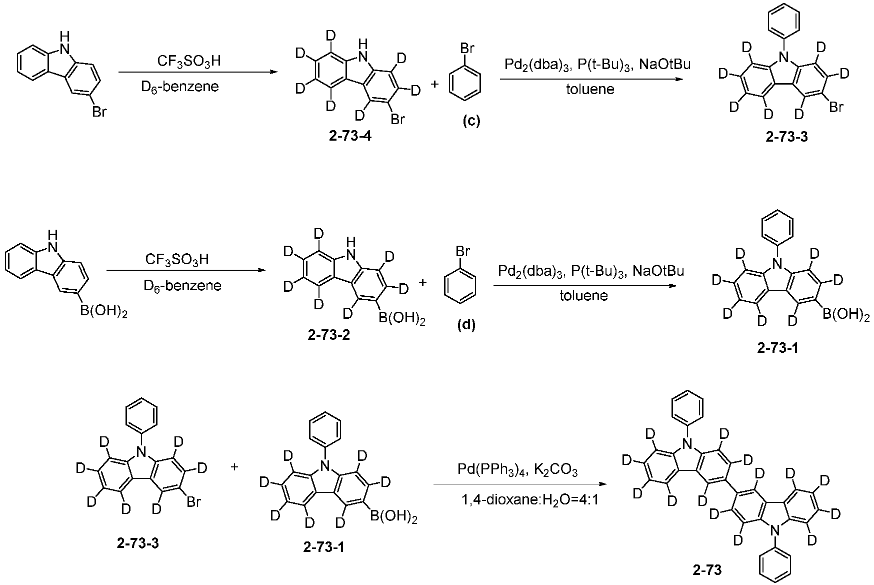

### 1) Preparation of Compound 2-73-4

3-bromo-9H-carbazole (10 g, 40.23 mmol), D₆-benzene (1,000 mL) and triflic acid (CF₃SO₃H) (170 g, 1,075 mmol) were introduced, and stirred at 50°C.

When the reaction was completed, the reaction solution was neutralized with D₂O, and then extracted with an aqueous sodium carbonate (Na₂CO₃) solution and dichloromethane (DCM) at room temperature. The organic layer was dried with anhydrous magnesium sulfate (MgSO₄), and then the solvent was removed using a rotary evaporator. The reaction material was purified by column chromatography (dichloromethane:hexane=1:2), and recrystallized with methanol to obtain target Compound 2-73-4 (10 g, yield 98%).

### 2) Preparation of Compound 2-73-3

After introducing Compound 2-73-4 (10 g, 39.5 mmol), bromobenzene (c) (12.4 g, 79 mmol), tris(dibenzylideneacetone)dipalladium (Pd₂(dba)₃) (1.81 g, 1.98 mmol), tri-tert-butylphosphine (P(t-Bu)₃) (1.93 mL, 7.9 mmol) and sodium tert-butoxide (NatOBu) (11.4 g, 118.51 mmol), toluene (100 mL) was introduced thereto, and the mixture was heated for 15 hours at 135°C. When the reaction was finished, the reaction mixture was extracted with methylene chloride (MC) and water, and then purified by column chromatography to obtain Compound 2-73-3 (11 g, yield 84%).

### 3) Preparation of Compound 2-73-2

9H-carbazol-3-ylboronic acid (10 g, 47.3 mmol), D₆-benzene (1,000 mL) and triflic acid (CF₃SO₃H) (170 g, 1,075 mmol) were introduced, and stirred at 50°C.

When the reaction was completed, the reaction solution was neutralized with D₂O, and then extracted with an aqueous sodium carbonate (Na₂CO₃) solution and dichloromethane (DCM) at room temperature. The organic layer was dried with anhydrous magnesium sulfate (MgSO₄), and then the solvent was removed using a rotary evaporator. The reaction material was purified by column chromatography (dichloromethane:hexane=1:2), and recrystallized with methanol to obtain target Compound 2-73-2 (9 g, yield 87%).

### 4) Preparation of Compound 2-73-1

After introducing Compound 2-73-2 (9 g, 41.3 mmol), bromobenzene (d) (12.9 g, 82.5 mmol), tris(dibenzylideneacetone)dipalladium (Pd₂(dba)₃) (1.89 g, 2.06 mmol), tri-tert-butylphosphine (P(t-Bu)₃) (2 mL, 8.25 mmol) and sodium tert-butoxide (NatOBu) (7.93 g, 82.574 mmol), toluene (100 mL) was introduced thereto, and the mixture was heated for 10 hours at 135°C. When the reaction was finished, the reaction mixture was extracted with methylene chloride (MC) and water, and then purified by column chromatography to obtain Compound 2-73-1 (10 g, yield 82%).

### 5) Preparation of Compound 2-73

After introducing Compound 2-73-3 (10 g, 30.37 mmol), Compound 2-61-1 (17.87 g, 60.75 mmol), tetrakis(triphenylphosphine)palladium(0) (Pd(PPh₃)₄) (1.39 g, 1.52 mmol) and potassium carbonate (K₂CO₃) (12.59 g, 91.13 mmol), 1,4-dioxane (140 mL) and distilled water (35 mL) were added thereto, and the mixture was stirred for 4 hours at 120°C.

After that, the temperature was lowered to room temperature, and a produced solid was washed with distilled water and methanol to obtain Compound 2-73 (13 g, yield 85%).

### <Preparation Example 12> Preparation of Compounds 2-74, 2-75, 2-76, 2-77, 2-78, 2-80, 2-81, 2-82, 2-86, 2-87, 2-94, 2-95, 2-97, 2-98, 2-99, 2-100, 2-101, 2-102, 2-104, 2-112 and 2-114

Compounds 2-74, 2-75, 2-76, 2-77, 2-78, 2-80, 2-81, 2-82, 2-86, 2-87, 2-94, 2-95, 2-97, 2-98, 2-99, 2-100, 2-101, 2-102, 2-104, 2-112 and 2-114 were synthesized in the same manner as in Preparation Example 11, except that Compound c of the following Table 6 was used instead of bromobenzene (c), and Compound d of the following Table 6 was used instead of bromobenzene (d).

**[Table 6]**

| Compoun d No. | Compound c | Compound d | Target Compound (Yield%) |
|---|---|---|---|
| 2-74 | 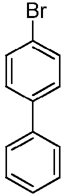 | 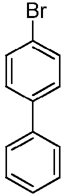 | 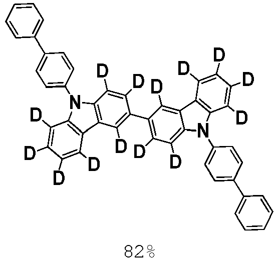 |
| 2-75 | 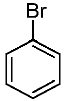 | 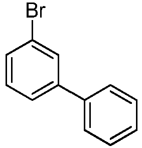 | 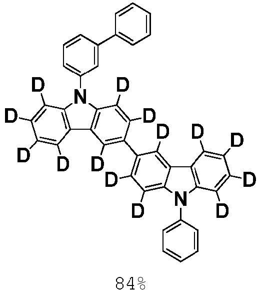 |
| 2-76 | 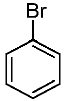 | 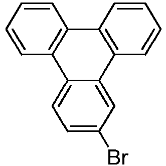 | 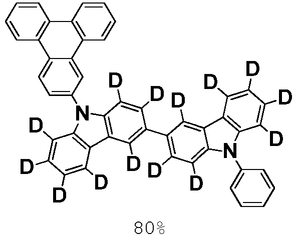 |
| 2-77 | 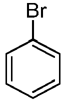 | 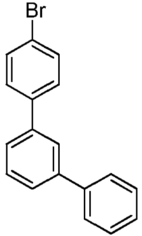 | 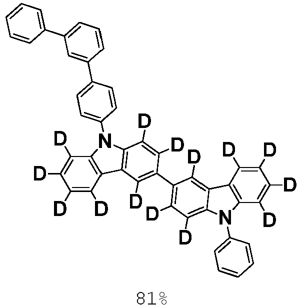 |
| 2-78 | 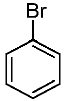 | 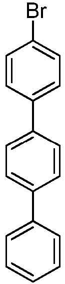 | 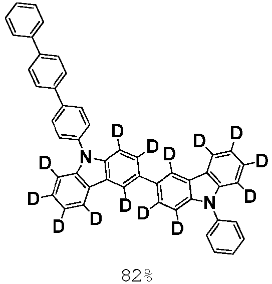 |
| 2-80 | 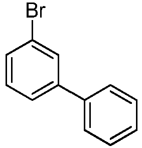 | 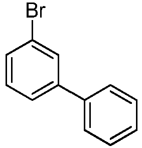 | 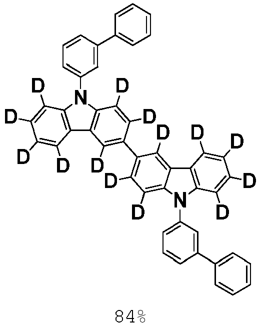 |
| 2-81 | 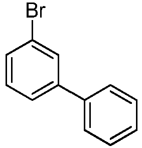 | 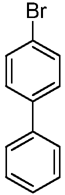 | 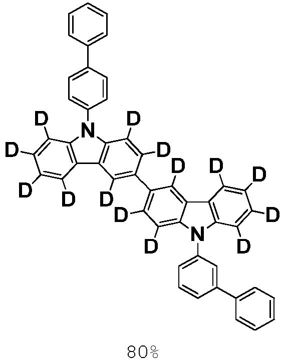 |
| 2-82 | 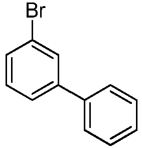 | 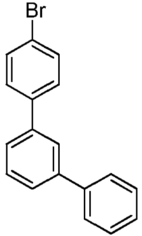 | 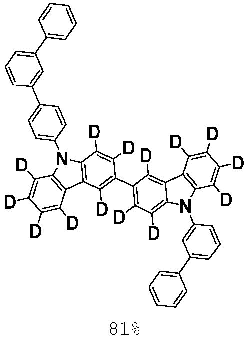 |
| 2-86 | 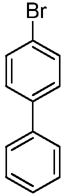 | 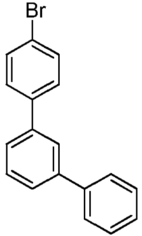 | 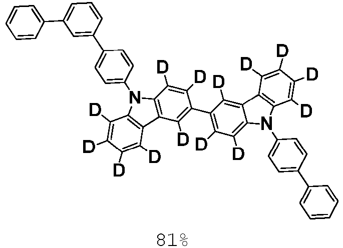 |
| 2-87 | 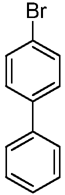 | 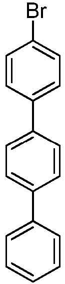 | 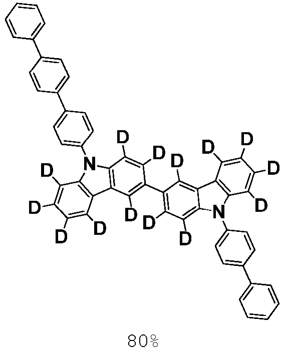 |
| 2-94 | 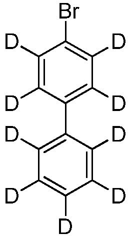 | 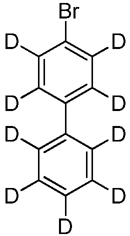 | 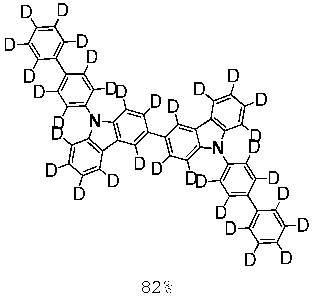 |
| 2-95 | 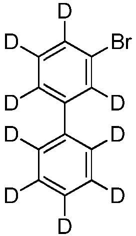 | 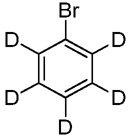 | 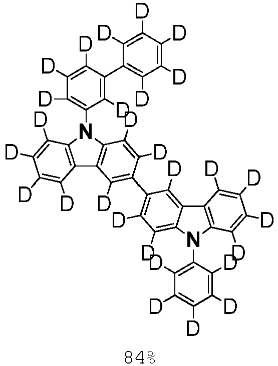 |
| 2-97 | 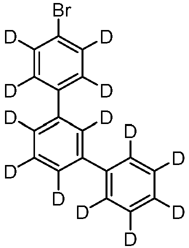 | 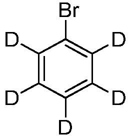 | 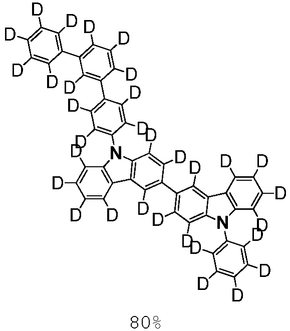 |
| 2-98 | 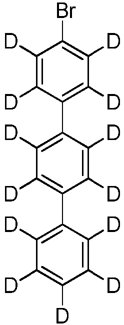 | 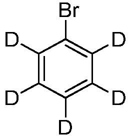 | 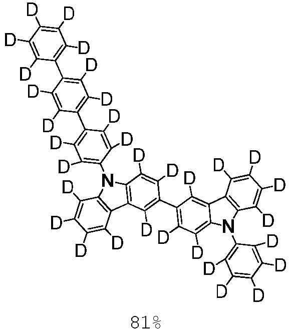 |
| 2-99 | 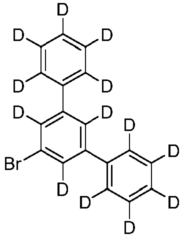 | 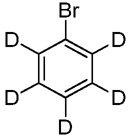 | 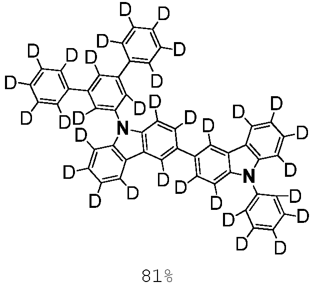 |
| 2-100 | 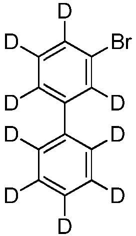 | 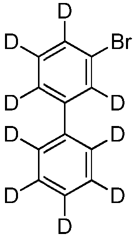 | 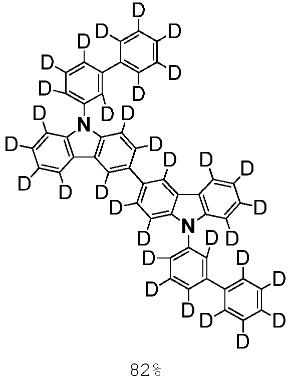 |
| 2-101 | 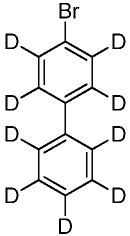 | 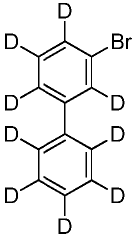 | 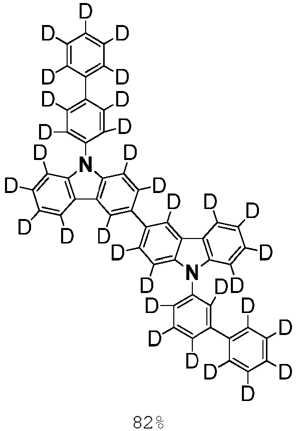 |
| 2-102 | 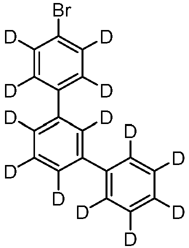 | 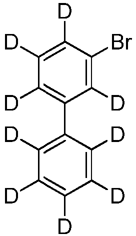 | 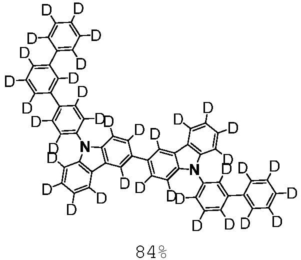 |
| 2-104 | 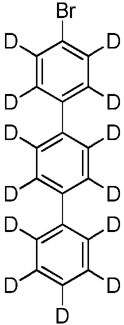 | 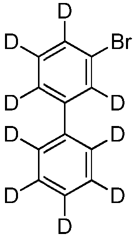 | 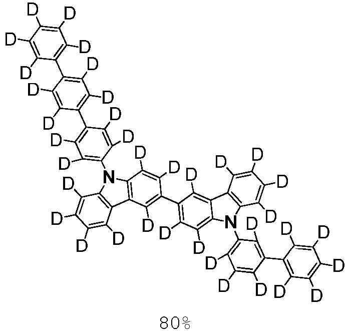 |
| 2-112 | 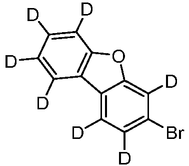 | 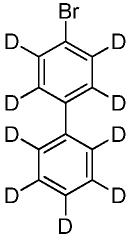 | 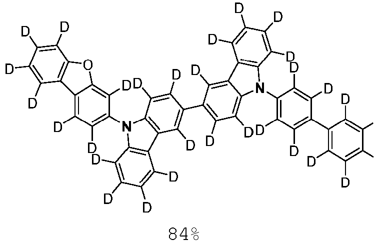 |
| 2-114 | 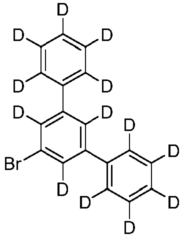 | 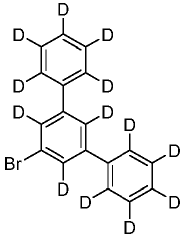 | 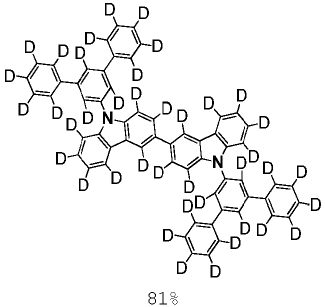 |

### <Preparation Example 13> Preparation of Compound 2-93

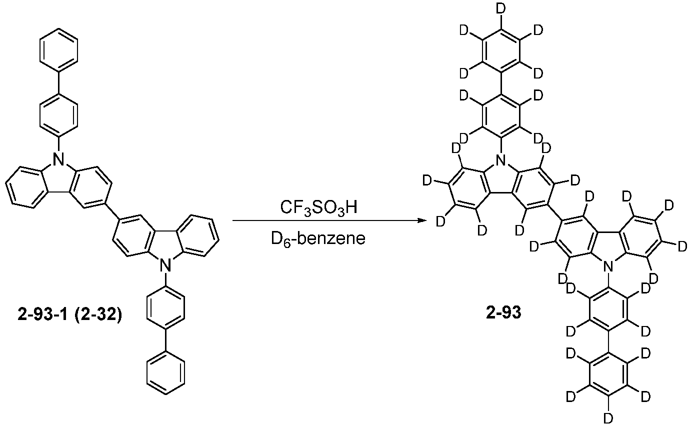

Compound 2-93-1 (Compound 2-32) (10 g, 15.7 mmol), D₆-benzene (1,000 mL) and triflic acid (CF₃SO₃H) (170 g, 1,075 mmol) were introduced, and stirred at 50°C.

When the reaction was completed, the reaction solution was neutralized with D₂O, and then extracted with an aqueous sodium carbonate (Na₂CO₃) solution and dichloromethane (DCM) at room temperature. The organic layer was dried with anhydrous magnesium sulfate (MgSO₄), and then the solvent was removed using a rotary evaporator. The reaction material was purified by column chromatography (dichloromethane:hexane=1:2), and recrystallized with methanol to obtain target Compound 2-93 (10.0 g, yield 95%).

### <Preparation Example 14> Preparation of Compound 3-1

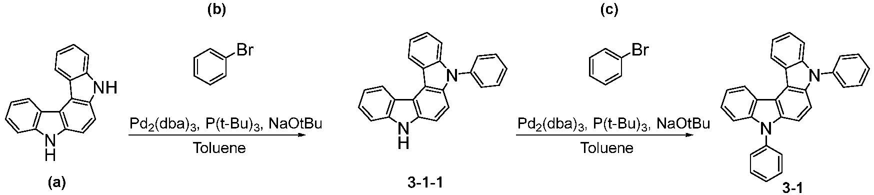

### 1) Preparation of Compound 3-1-1

After introducing 5,8-dihydroindolo[2,3-c]carbazole (a) (10 g, 39.0 mmol), 1-bromobenzene (b) (6.12 g, 39.0 mmol), tris(dibenzylideneacetone)dipalladium (Pd₂(dba)₃) (1.79 g, 1.95 mmol), tri-tert-butylphosphine (P(t-Bu)₃) (0.92 mL, 3.9 mmol) and sodium tert-butoxide (NatOBu) (7.50 g, 78.0 mmol) to a reaction flask, toluene (100 mL) was introduced thereto, and the mixture was heated for 15 hours at 135°C. When the reaction was finished, the reaction mixture was extracted with methylene chloride (MC) and water, and then purified by column chromatography to obtain Compound 3-1-1 (7.3 g, yield 56%).

### 2) Preparation of Compound 3-1

After introducing Compound 3-1-1 (7.3 g, 22.0 mmol), 1-bromobenzene (b) (3.8 g, 24.2 mmol), tris(dibenzylideneacetone)dipalladium (Pd₂(dba)₃) (1.01 g, 1.1 mmol), tri-tert-butylphosphine (P(t-Bu)₃) (0.52 mL, 3.9 mmol) and sodium tert-butoxide (NatOBu) (4.23 g, 44.0 mmol), toluene (70 mL) was introduced thereto, and the mixture was heated for 15 hours at 135°C. When the reaction was finished, the reaction mixture was extracted with methylene chloride (MC) and water, and then purified by column chromatography to obtain Compound 3-1 (8.3 g, yield 93%).

### <Preparation Example 15> Preparation of Compounds 3-4, 3-5, 3-22, 3-23, 3-32, 3-35, 3-41, 3-61, 3-69, 3-77, 3-96 and 3-99

Target compounds were synthesized in the same manner as in Preparation Example 14, except that Intermediate A of the following Table 7 was used instead of (a), Intermediate B of the following Table 7 was used instead of (b), and Intermediate C of the following Table 7 was used instead of (c).

**[Table 7]**

| | Intermediate A | Intermediate B | Intermediate C | Target Compound |
|---|---|---|---|---|
| 3-4 | 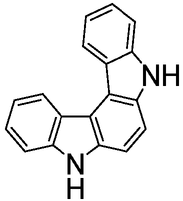 | 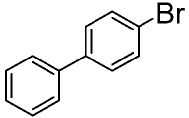 | 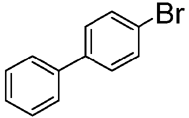 | 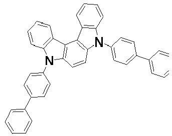 |
| 3-5 | 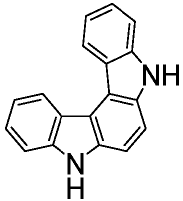 | 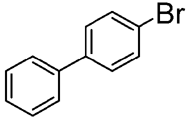 | 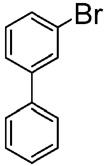 | 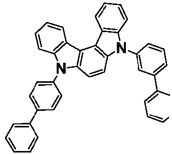 |
| 3-22 | 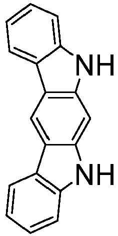 | 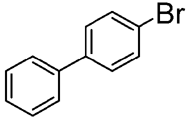 | 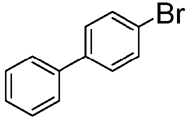 | 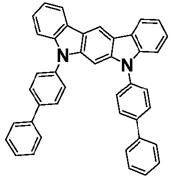 |
| 3-23 | 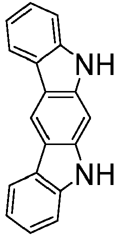 | 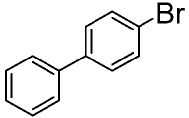 | 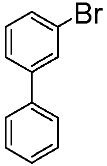 | 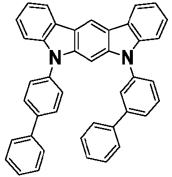 |
| 3-32 | 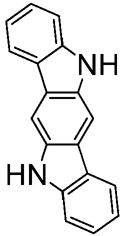 | 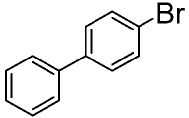 | 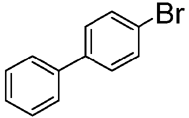 | 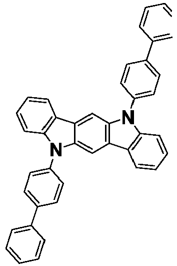 |
| 3-35 | 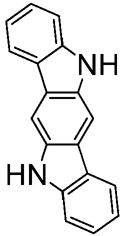 | 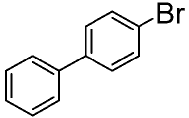 | 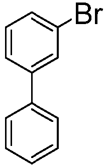 | 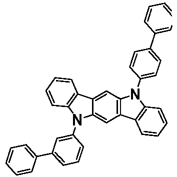 |
| 3-41 | 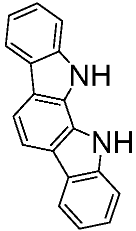 | 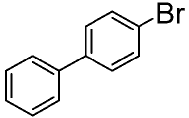 | 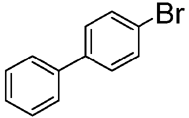 | 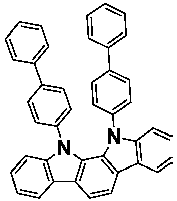 |
| 3-61 | 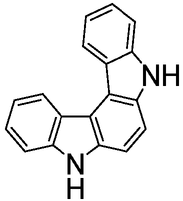 | 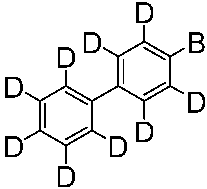 | 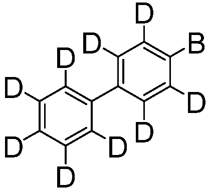 | 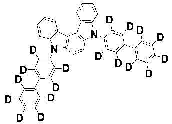 |
| 3-69 | 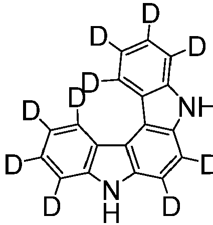 | 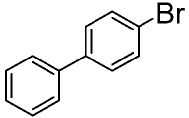 | 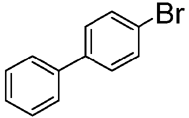 | 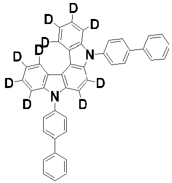 |
| 3-77 | 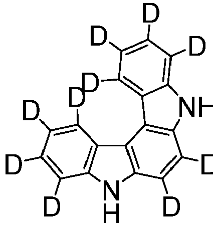 | 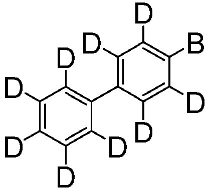 | 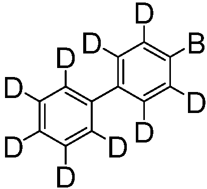 | 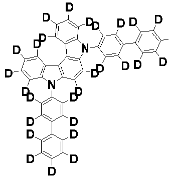 |
| 3-96 | 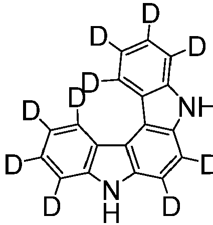 | 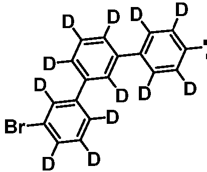 | 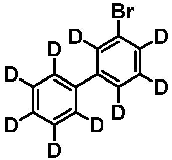 | 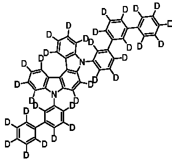 |
| 3-99 | 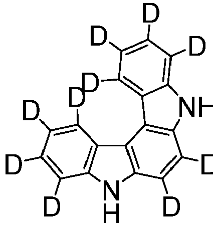 | 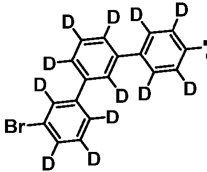 | 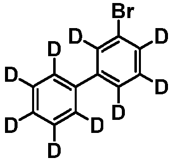 | 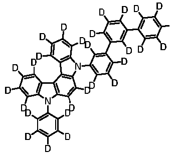 |

The rest of compounds other than the compounds described in Preparation Examples 1 to 15 and Tables 1 to 7 were also prepared in the same manner as the methods described in the Preparation Examples described above, and the synthesis results are shown in the following Tables 8 and 9. The following Table 8 shows measurement values of ¹H NMR (CDCl₃, 400 MHz), and the following Table 9 shows measurement values of field desorption mass spectrometry (FD-MS).

**[Table 8]**

| Compound No. | ¹H NMR (CDCl₃, 400 MHz) |
|---|---|
| 1-1 | δ=8.36(4H, s), 7.64(1H, d), 7.5(6H, d), 7.41(1H, d), |
| 1-4 | δ=8.36(4H, s), 7.86(1H, d), 7.57(1H, d), 7.5(6H, d), 7.41(1H, d), |
| 1-9 | δ=8.36(4H, s), 7.5(6H, d), |
| 1-13 | δ=8.36(4H, s), 7.95(1H, d), 7.5(6H, d), 7.49(1H, d), 7.48(1H, d), |
| 1-15 | δ=8.36(4H, s), 7.82(1H, d), 7.77(1H, d), 7.6(1H, d), 7.5(6H, d), 7.48 (1H, d), |
| 1-25 | δ=8.36(4H, s), 7.91(1H, d), 7.6(1H, d), 7.5(6H, d), 7.42(1H, d), |
| 1-29 | δ=8.36(2H, s), 7.96(2H, d), 7.77(1H, d), 7.75(2H, d), 7.64(1H, d), 7.5(4H, d), 7.49(3H, d), 7.41(1H, d), 7.25(2H, d), |
| 1-32 | δ=8.38(1H, s), 7.96(2H, d), 7.94(1H, d), 7.75(4H, d), 7.73(1H, d), 7.61(1H, d), 7.49(4H, d), 7.41(2H, d), 7.25(2H, d) |
| 1-34 | δ=8.36(2H, s), 7.96(2H, d), 7.81(1H, d), 7.75(2H, d), 7.57(1H, d), 7.5(3H, d), 7.49(2H, d), 7.42(1H, d), 7.41(1H, d), 7.25(6H, dd), |
| 1-43 | δ=8.55(1H, s), 8.45(1H, d), 8.38(1H, d), 7.97(1H, d), 7.94(1H, d), 7.93(1H, d), 7.92(1H, d), 7.81(1H, d), 7.77(1H, d), 7.75(2H, dd), 7.73(1H, d), 7.7(1H, d), 7.61(1H, dd), 7.56(1H, dd), 7.5(1H, s), 7.49 (3H, t), 7.41 (1H, t), |
| 1-45 | δ=8.55(1H, s), 8.36(2H, d), 8.12(2H, d), 7.99(1H, d), 7.92(1H, d), 7.81(1H, d), 7.75(2H, d), 7.71(1H, d), 7.7(1H, d), 7.5(4H, dd), 7.49(2H, d), 7.41(1H, d), |
| 1-49 | δ=8.55(1H, s), 8.45(1H, d), 8.38(1H, d), 8.36(2H, d), 8.32(1H, d), 7.94(1H, d), 7.93(1H, d), 7.73(1H, d), 7.7(1H, d), 7.61(1H, dd), 7.57(1H, d), 7.56(1H, d), 7.5(3H, dd), 7.49(2H, dd), |
| 1-56 | δ=8.36(2H, s), 8.03(1H, d), 7.98(1H, d), 7.96(1H, d), 7.82(1H, d), 7.76(1H, d), 7.54(1H, d), 7.5(4H, d), 7.49(1H, d), 7.39(1H, dd), 7.31(1H, d), |
| 1-57 | δ=8.36(2H, s), 8.03(1H, d), 7.82(2H, d), 7.81(1H, d), 7.79(2H, d), 7.76(1H, d), 7.69(1H, d), 7.57(1H, d), 7.5(3H, d), 7.48(1H, dd), 7.46(2H, d), 7.41(2H, d), |
| 1-59 | δ=8.36(2H, s), 7.88(1H, d), 7.83(1H, d), 7.82(1H, d), 7.79(3H, d), 7.69(1H, d), 7.57(2H, d), 7.5(3H, d), 7.49(1H, d), 7.46(2H, dd), 7.41(1H, d), |
| 1-61 | δ=8.38(1H, s), 8.36(2H, d), 8.08(1H, d), 8.02(1H, d), 7.98(1H, d), 7.94(1H, d), 7.77(1H, d), 7.73(1H, d), 7.61(1H, d), 7.54(1H, dd), 7.51(1H, d), 7.5(3H, d), 7.42(1H, dd), 7.41(1H, dd), 7.39(1H, s), 7.31 (1H, t), |
| 1-62 | δ=8.36(2H, s), 8.08(1H, d), 8.02(1H, d), 7.98(1H, d), 7.96(2H, d), 7.64(1H, d), 7.54(1H, d), 7.51(1H, d), 7.5(3H, d), 7.49(1H, dd), 7.39(1H, d), 7.31(1H, d), 7.25(2H, dd), |
| 1-63 | δ=8.38(1H, s), 8.36(2H, d), 8.03(1H, d), 7.98(1H, d), 7.94(1H, d), 7.82(1H, d), 7.76(1H, d), 7.73(1H, d), 7.61(1H, d), 7.54(1H, dd), 7.5(3H, d), 7.41(1H, d), 7.39(1H, dd), 7.31(1H, dd), |
| 1-69 | δ=8.55(1H, s), 8.36(2H, d), 8.19(1H, d), 7.94(1H, d), 7.92(1H, d), 7.91(1H, d), 7.8(1H, d), 7.77(1H, d), 7.58(1H, d), 7.5(4H, dd), 7.46(1H, d), 7.41(1H, d), 7.35(1H, dd), 7.2(1H, dd), 7.16(1H, s), |
| 1-76 | δ=8.55(1H, s), 8.38(1H, d), 8.19(1H, d), 7.96(2H, d), 7.94(2H, d), 7.91(1H, d), 7.87(1H, d), 7.75(4H, d), 7.73(1H, d), 7.71(1H, dd), 7.64(1H, d), 7.61(1H, d), 7.58(1H, dd), 7.5(1H, dd), 7.49(4H, s), 7.48 (1H, t), 7.41 (2H, t), 7.35 (1H, dd), 7.25 (2H, dd), 7.2 (1H, t), 7.16 (1H, d), |
| 1-77 | δ=8.55(1H, s), 8.38(1H, d), 8.19(1H, d), 7.96(2H, d), 7.94(2H, d), 7.75(4H, d), 7.73(1H, d), 7.61(1H, d), 7.58(1H, d), 7.5(1H, dd), 7.49(4H, d), 7.41(2H, d), 7.35(1H, dd), 7.25(2H, dd), 7.2(1H, s), 7.16 (1H, t), |
| 1-78 | δ=8.38(1H, s), 7.97(1H, d), 7.96(2H, d), 7.94(2H, d), 7.75(4H, d), 7.73(1H, d), 7.61(1H, d), 7.5(1H, d), 7.49(5H, d), 7.41(2H, dd), 7.25(2H, d), |
| 1-86 | δ=8.55(2H, s), 8.36(2H, d), 8.32(1H, d), 7.94(1H, d), 7.92(1H, d), 7.75(2H, d), 7.73(1H, d), 7.7(2H, d), 7.61(2H, d), 7.5(3H, dd), 7.49(2H, d), 7.41(1H, d), |
| 1-115 | δ=8.36(2H, s), 8.08(1H, d), 8.03(1H, d), 8.02(1H, d), 7.82(1H, d), 7.76(1H, d), 7.51(3H, d), 7.5(3H, d), 7.46(2H, d), 7.41(1H, dd), |
| 1-121 | δ=8.55(1H, s), 8.36(4H, d), 8.19(1H, d), 8.01(1H, d), 7.94(1H, d), 7.87(1H, d), 7.81(1H, d), 7.71(1H, d), 7.58(1H, d), 7.57(1H, dd), 7.5(7H, d), 7.35(1H, d), 7.2(1H, dd), 7.16(1H, dd), |
| 1-124 | δ=8.55(1H, s), 8.38(1H, d), 8.36(2H, d), 8.19(1H, d), 8.03(1H, d), 7.98(1H, d), 7.94(2H, d), 7.82(1H, d), 7.76(1H, d), 7.73(1H, dd), 7.71(1H, d), 7.61(1H, d), 7.58(1H, dd), 7.54(1H, dd), 7.5(4H, s), 7.48 (1H, t), 7.39 (1H, t), 7.35 (1H, dd), 7.31 (1H, dd), 7.2 (1H, t), 7.16 (1H, d), |
| 1-131 | δ=8.8(1H, s), 8.36(4H, d), 8.03(1H, d), 7.89(1H, d), 7.53(1H, d), 7.5(6H, d), 7.38(1H, d), 6.96(1H, d), 2.46(2H, d), |
| 1-134 | δ=8.55(1H, s), 8.36(5H, d), 7.94(1H, d), 7.62(1H, d), 7.5(7H, d), 7.35(1H, d), 7.16(1H, d), 1.43(3H, d), |
| 1-135 | δ=8.55(1H, s), 8.36(4H, d), 8.19(1H, d), 7.94(1H, d), 7.58(1H, d), 7.5(7H, d), 7.35(1H, d), 7.2(1H, d), 7.16(1H, d), |
| 1-136 | δ=8.36(4H, s), 7.5(6H, d), |
| 1-140 | δ=8.36(2H, s), 8.03(1H, d), 7.82(2H, d), 7.79(2H, d), 7.76(1H, d), 7.69(1H, d), 7.57(1H, d), 7.5(3H, d), 7.46(2H, d), 7.41(1H, dd), |
| 1-146 | δ=8.36(4H, s), 7.5(6H, d), |
| 1-147 | δ=8.36(2H, s), 7.96(2H, d), 7.75(2H, d), 7.5(3H, d), 7.49(2H, d), 7.41(1H, d), 7.25(2H, d), |
| 1-148 | δ=8.36(2H, s), 8.03(1H, d), 7.98(1H, d), 7.82(1H, d), 7.76(1H, d), 7.54(1H, d), 7.5(3H, d), 7.39(1H, d), 7.31(1H, d), |
| 1-149 | δ=8.55(1H, s), 8.36(2H, d), 8.24(1H, d), 8.21(1H, d), 8.19(1H, d), 7.94(1H, d), 7.68(1H, d), 7.6(1H, d), 7.58(1H, d), 7.5(4H, dd), 7.35(1H, d), 7.2(1H, d), 7.16(1H, dd), |
| 1-151 | δ=8.55(1H, s), 8.36(2H, d), 8.24(1H, d), 8.21(1H, d), 8.19(1H, d), 7.94(1H, d), 7.68(1H, d), 7.6(1H, d), 7.58(1H, d), 7.5(4H, dd), 7.35(1H, d), 7.2(1H, d), 7.16(1H, dd), |
| 2-1 | δ=8.55(d, 1H), 8.30(d, 1H), 8.19-8.13(m, 2H), 7.99-7.89(m, 4H), 7.77(d, 1H), 7.62-7.50(m, 12H), 7.35(t, 1H), 7.20-7.16(m, 2H) |
| 2-2 | δ=8.55(d, 1H), 8.30(d, 1H), 8.19-8.13(m, 2H), 7.99-7.89(m, 6H), 7.80-7.77(m, 2H), 7.62-7.35(m, 10H), 7.20-7.16(m, 6H) |
| 2-3 | δ=8.55(d, 1H), 8.18-8.09(m, 3H), 8.00-7.87(m, 3H), 7.77(s, 2H), 7.58-7.25(m, 18H) |
| 2-4 | δ=8.55(d, 1H), 8.18-8.12(m, 2H), 8.00-7.84(m, 3H), 7.79-7.77(m, 4H), 7.68-7.25(m, 22H) |
| 2-5 | δ=8.55(d, 1H), 8.30(d, 1H), 8.21-8.13(m, 3H), 7.99-7.89(m, 4H), 7.77-7.35(m, 17H), 7.25-7.16(m, 6H) |
| 2-6 | δ=8.55(d, 1H), 8.30(d, 1H), 8.19-8.13(m, 2H), 7.94-7.89(m, 8H), 7.77-7.75(m, 3H), 7.62-7.35(m, 11H), 7.25-7.16(m, 6H) |
| 2-7 | δ=8.55(d, 1H), 8.18-8.09(m, 4H), 8.00-7.94(m, 2H), 7.87(m, 1H), 7.77(m, 2H), 7.69-7.63(m, 2H), 7.52-7.25(m, 20H) |
| 2-11 | δ=8.55(d, 1H), 8.30(d, 1H), 8.19-8.13(m, 2H), 7.99-7.89(m, 5H), 7.77(d, 1H), 7.58-7.28(m, 16H), 1.69(s, 6H) |
| 2-16 | δ=9.05(s, 1H), 8.55(d, 1H), 8.33-8.13(m, 7H), 7.99-7.89(m, 5H), 7.77-7.50(m, 13H), 7.35(t, 1H), 7.20-7.16(m, 2H) |
| 2-19 | δ=8.55(d, 1H), 8.30(d, 1H), 8.19-8.13(m, 2H), 7.99-7.89(m, 8H), 7.80-7.77(m, 3H), 7.58(d, 1H), 7.50-7.35(m, 6H), 7.20-7.16(m, 10H) |
| 2-20 | δ=8.55(d, 1H), 8.30(d, 1H), 8.21-8.13(m, 3H), 7.99-7.89(m, 6H), 7.80-7.35(m, 15H), 7.20-7.16(6H) |
| 2-21 | δ=8.55(d, 1H), 8.30(d, 1H), 8.19-8.13(m, 2h), 7.99-7.89(m, 10H), 7.80-7.75(m, 4H), 7.50-7.35(m, 8H), 7.20-7.16(m, 6H) |
| 2-22 | δ=8.55(d, 1H), 8.30(d, 1H), 8.21-8.13(m, 3H), 7.99-7.89(m, 6H), 7.80-7.35(m, 15H), 7.25-7.16(10H) |
| 2-23 | δ=8.55(d, 1H), 8.30(d, 1H), 8.19-8.13(m, 2h), 7.99-7.89(m, 10H), 7.80-7.75(m, 4H), 7.50-7.35(m, 8H), 7.25-7.16(m, 10H) |
| 2-26 | δ=8.55(m, 1H), 8.30(d, 1H), 8.21-8.13(m, 4h), 7.99-7.89(m, 4H), 7.77-7.35(m, 20H), 7.25-7.16(6H) |
| 2-27 | δ=8.55(m, 1H), 8.30(d, 1H), 8.21-8.13(m, 4h), 7.99-7.89(m, 4H), 7.77-7.35(m, 20H), 7.20-7.16(2H) |
| 2-28 | δ=8.55(m, 1H), 8.18-8.09(m, 3H), 8.00-8.79(m, 2H), 7.87(m, 1H), 7.79-7.77(m, 4H), 7.69-7.63(m, 4H), 7.52-7.25(m, 12H) |
| 2-29 | δ=8.55(m, 1H), 8.18-8.09(m, 3H), 8.00-7.94(m, 2H0, 7.87(m, 1H), 7.87(m, 1H), 7.79-7.77(m, 4H), 7.69-7.63(m, 4H), 7.52-7.25(m, 21H) |
| 2-30 | δ=8.55(m, 1H), 8.31-8.30(m, 3H), 8.21-8.13(m, 3h), 7.99-7.89(m, 3H), 7.75-7.35(m, 22H), 7.20-7.16(m, 2H) |
| 2-32 | δ=8.55(m, 1H), 8.18-8.12(m, 2H), 8.00-7.87(m, 3H), 7.79-7.77(m, 6H), 7.69-7.63(m, 6H), 7.52-7.25(m, 14H) |
| 2-33 | δ=8.55(m, 1H), 8.30(d, 1H), 8.21-8.13(m, 3H), 7.99-7.89(m, 8H), 7.77-7.35(m, 17H), 7.25-7.16(6H) |
| 2-34 | δ=8.55(m, 1H), 8.18-8.12(m, 2H), 8.00-7.87(m, 3H), 7.79-7.77(m, 6H), 7.67-7.63(m, 6H), 7.52-7.25(m, 18H) |
| 2-38 | δ=8.55(m, 1H), 8.18-8.12(m, 2H), 8.05-7.87(m, 6H), 7.79-7.77(m, 4H), 7.69-7.63(m, 4H), 7.52-7.25(m, 23H) |
| 2-40 | δ=8.55(m, 1H), 8.30(d, 1H), 8.19-8.13(m, 2H), 8.03-7.75(m, 15H), 7.58-7.35(m, 9H), 7.25-7.16(m, 6H) |
| 2-53 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-54 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-55 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-57 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-58 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-60 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-61 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-62 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-63 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-64 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-67 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-69 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-72 | δ=8.55(m, 1H), 8.30(d, 1H), 8.19-8.13(m, 2H), 7.99-7.89(m, 4H), 7.77(d, 1H), 7.58-7.50(m, 2H), 7.35(t, 1H), 7.20-7.16(m, 2H) |
| 2-73 | δ=7.62-7.50(m, 10H) |
| 2-74 | δ=7.79(m, 4H), 7.68(m. 4H), 7.52-7.41(m, 10H) |
| 2-75 | δ=8.21(s, 1H) 7.75-7.41(m, 13H) |
| 2-76 | δ=9.05(s, 1H), 8.33-8.25(m, 4H), 7.94(d, 1H), 7.70-7.50(m, 10H) |
| 2-77 | δ=7.79(m, 2H), 7.70-7.68(m, 3H), 7.58-7.41(m, 13H) |
| 2-78 | δ=7.92-7.91(m, 4H), 7.75(d, 2H), 7.62-7.41(m, 8H), 7.25(s, 4H) |
| 2-80 | δ=8.21(s, 2H), 7.75-7.60(m, 8H), 7.49-7.41(8H) |
| 2-81 | δ=8.21(s, 1H), 7.92-7.91(m, 4H), 7.75-7.60(m, 6H), 7.49-7.41(m, 7H) |
| 2-82 | δ=8.21(s, 1H), 7.94-7.91(m, 5H), 7.75-7.61(m, 9H), 7.49-7.41(m, 7H) |
| 2-86 | δ=7.94-7.91(m, 9H), 7.75-7.73(m, 5H), 7.61(d, 2H), 7.49-7.41(m, 6H) |
| 2-87 | δ=7.92-7.91(m, 8H), 7.75(d, 4H), 7.49-7.41(m, 6H), 7.25(s, 4H) |
| 3-1 | δ=8.55(2H, d), 7.94(2H, d), 7.62-7.35(14H, m), 7.16(2H, d) |
| 3-4 | δ=8.55(2H, d), 7.94-7.91(10H, m), 7.75(4H, d), 7.49-7.35(10H, m), 7.16 (2H, t) |
| 3-5 | δ=8.55(2H, d), 8.21(1H, s), 7.94-7.91(6H, m), 7.75-7.35(16H, m), 7.26(1H, d), 7.16(2H, t) |
| 3-22 | δ=8.55(1H, d), 8.19(1H, d), 7.94-7.91(9H, m), 7.75(4H, d), 7.58-7.35(11H, m), 7.20-7.16(2H, m) |
| 3-23 | δ=8.55(1H, d), 8.21-8.19(2H, m), 7.94-7.91(5H, m), 7.75-7.35(18H, m), 7.20-7.16(2H, m) |
| 3-32 | δ=8.55(1H, d), 8.19(1H, d), 7.94-7.91(9H, m), 7.75(4H, d), 7.58-7.35(11H, m), 7.20-7.16(2H, m) |
| 3-35 | δ=8.55(1H, d), 8.21-8.19(2H, m), 7.94-7.91(5H, m), 7.68-7.35(18H, m), 7.20-7.16(2H, m) |
| 3-41 | δ=8.55(2H, d), 7.94-7.91(10H, m), 7.84(2H, d), 7.75(4H, d), 7.49-7.35(8H, m), 7.16(2H, t) |
| 3-61 | δ=8.55(2H, d), 7.94(2H, d), 7.42-7.35(4H, m), 7.16(2H, t) |
| 3-69 | δ=7.92-7.91(8H, m), 7.75(4H, d), 7.49-7.41(6H, m) |

**[Table 9]**

| Compound No. | FD-MS | Compound No. | FD-MS |
|---|---|---|---|
| 1-1 | m/z=564.30 (C39H12D14N4, 564.75) | 1-4 | m/z=723.41 (C51H13D21N4, 723.99) |
| 1-9 | m/z=726.43 (C51H10D24N4, 727.01) | 1-13 | m/z=735.40 (C51H13D20N5, 735.98) |
| 1-15 | m/z=734.37 (C51H14D18N4O, 734.95) | 1-25 | m/z=751.35 (C51H13D19N4S, 752.02) |
| 1-29 | m/z=638.32 (C45H18D12N4, 638.84) | 1-32 | m/z=712.34 (C51H24D10N4, 712.92) |
| 1-34 | m/z=715.36 (C51H21D13N4, 715.94) | 1-43 | m/z=744.31 (C51H20D12N4S, 744.98) |
| 1-45 | m/z=745.32 (C51H19D13N4S, 745.98) | 1-49 | m/z=746.32 (C51H18D14N4S, 746.99) |
| 1-56 | m/z=653.31 (C45H15D13N4O, 653.83) | 1-57 | m/z=729.34 (C51H19D13N4O, 729.92) |
| 1-59 | m/z=730.35 (C51H18D14N4O, 730.93) | 1-61 | m/z=729.34 (C51H19D13N4O, 729.92) |
| 1-62 | m/z=730.35 (C51H18D14N4O, 730.93) | 1-63 | m/z=731.35 (C51H17D15N4O, 731.94) |
| 1-69 | m/z=729.36 (C51H19D14N5, 729.95) | 1-76 | m/z=705.30 (C51H31D3N4, 705.88) |
| 1-77 | m/z=710.33 (C51H26D8N4, 710.91) | 1-78 | m/z=714.35 (C51H22D12N4, 714.93) |
| 1-86 | m/z=824.37 (C57H20D16N4S, 825.10) | 1-115 | m/z=732.36 (C51H16D16N4O, 732.94) |
| 1-121 | m/z=553.23 (C39H23D3N4, 553.68) | 1-124 | m/z=722.30 (C51H26D6N4O, 722.88) |
| 1-131 | m/z=586.30 (C41H22D8N4, 586.77) | 1-134 | m/z=614.33 (C43H26D8N4, 614.82) |
| 1-135 | m/z=638.3224 (C45H18D12N4, 638.8362) | 1-136 | m/z=646.3726 (C45H10D20N4, 646.8850) |
| 1-140 | m/z=812.4144 (C57H16D20N4O, 813.0640) | 1-146 | m/z=646.3726 (C45H10D20N4, 646.8850) |
| 1-147 | m/z=722.4039 (C51H14D20N4, 722.9830) | 1-148 | m/z=736.3831 (C51H12D20N4O, 736.9660) |
| 1-149 | m/z=811.4304 (C57H17D20N, 812.0800) | 1-151 | m/z=811.4304 (C57H17D20N, 812.0800) |
| 2-1 | m/z=484.59(C₃₆H₂₄N₂=484.19) | 2-2 | m/z=560.69(C₄₂H₂₈N₂=560.23) |
| 2-3 | m/z=560.69(C₄₂H₂₈N₂=560.23) | 2-4 | m/z=56069(C₄₂H₂₈N₂=560.23) |
| 2-5 | m/z=636.78(C₄₈H₃₂N₂=636.26) | 2-6 | m/z=636.78(C₄₈H₃₂N₂=636.26) |
| 2-7 | m/z=636.78(C₄₈H₃₂N₂=636.26) | 2-8 | m/z=543.65(C₄₀H₂₆N₂=543.21) |
| 2-9 | m/z=543.65(C₄₀H₂₆N₂=543.21) | 2-10 | m/z=600.75(C₄₅H₃₅N₂=600.26) |
| 2-11 | m/z=600. 75(C₄₅H₃₅N₂=600.26) | 2-12 | m/z=724.89(C₅₅H₃₆N₂=724.29) |
| 2-13 | m/z=724.89(C₅₅H₃₆N₂=724.29) | 2-14 | m/z=724.89(C₅₅H₃₆N₂=724.29) |
| 2-15 | m/z=724.89(C₅₅H₃₆N₂=724.29) | 2-16 | m/z=634.77 (C₄₈H₃₀N₂=634.24) |
| 2-17 | m/z=509.60(C₃₇H₂₃N₃=509.19) | 2-18 | m/z=742.98 (C₅₄H₃₈N₂Si=742.28) |
| 2-19 | m/z=636.78(C₄₈H₃₂N₂=636.26) | 2-20 | m/z=636.78 (C₄₈H₃₂N₂=636.26) |
| 2-21 | m/z=636.78(C₄₈H₃₂N₂=636.26) | 2-22 | m/z=712.88(C₅₄H₃₆N₂=712.29) |
| 2-23 | m/z=712.88(C₅₄H₃₆N₂=712.29) | 2-24 | m/z=712.88(C₅₄H₃₆N₂=712.29) |
| 2-25 | m/z=710.86(C₅₄H₃₄N₂=710.27) | 2-26 | m/z=712.88(C₅₄H₃₆N₂=712.29) |
| 2-27 | m/z=712.88(C₅₄H₃₆N₂=712.29) | 2-28 | m/z=712.88(C₅₄H₃₆N₂=712.29) |
| 2-29 | m/z=712.88(C₅₄H₃₆N₂=712.29) | 2-30 | m/z=712.88(C₅₄H₃₆N₂=712.29) |
| 2-31 | m/z=710.86(C₅₄H₃₄N₂=710.27) | 2-32 | m/z=636.78(C₄₈H₃₂N₂=636.26) |
| 2-33 | m/z=712.88(C₅₄H₃₆N₂=712.29) | 2-34 | m/z=712.88(C₅₄H₃₆N₂=712.29) |
| 2-35 | m/z=788.97(C₆₀H₄₀N₂=788.32) | 2-36 | m/z=686.84(C₅₂H₃₄N₂=686.27) |
| 2-37 | m/z=788.97(C₆₀H₄₀N₂=788.32) | 2-38 | m/z=788.97(C₆₀H₄₀N₂=788.32) |
| 2-39 | m/z=686.84(C₅₂H₃₄N₂=686.27) | 2-40 | m/z=686.84(C₅₂H₃₄N₂=686.27) |
| 2-53 | m/z=494.65(C₃₆H₁₄D₁₀N₂=494.26) | 2-54 | m/z=654.89(C₄₈H₁₄D₁₈N₂=654.37) |
| 2-55 | m/z=574.77(C₄₁H₁₄D₁₄N₂=574.31) | 2-56 | m/z=650.86(C₄₈H₁₄D₁₆N₂=650.34) |
| 2-57 | m/z=654.89(C₄₈H₁₄D₁₈N₂=654.37) | 2-58 | m/z=654.89(C₄₈H₁₄D₁₈N₂=654.37) |
| 2-59 | m/z=654.89(C₄₈H₁₄D₁₈N₂=654.37) | 2-60 | m/z=654.89(C₄₈H₁₄D₁₈N₂=654.37) |
| 2-61 | m/z=654.89(c₄₈H₁₄D₁₈N₂=654.37) | 2-72 | m/z:734.43(C₅₄H₁₄D₂₂N₂=735.03) |
| 2-63 | m/z=735.01(C₅₄H₁₄D₂₂N₂=734.43) | 2-64 | m/z=735.01(C₅₄H₁₄D₂₂N₂=734.43) |
| 2-65 | m/z=730.98(C₅₄H₁₄D₂₀N₂=730.40) | 2-66 | m/z=735.01(C₅₄H₁₄D₂₂N₂=734.43) |
| 2-67 | m/z=735.01(C₅₄H₁₄D₂₂N₂=734.43) | 2-68 | m/z=815.13(C₆₀H₁₄D₂₆N₂=814.48) |
| 2-69 | m/z=815.13(C₆₀H₁₄D₂₆N₂=814.48) | 2-70 | m/z=815.13(C₆₀H₁₄D₂₆N₂=814.48) |
| 2-71 | m/z=815.13(C₆₀H₁₄D₂₆N₂=814.48) | 2-72 | m/z=666.86(C₄₈H₁₄D₁₆N₂O=666.34) |
| 2-73 | m/z=498.68(C₃₆H₁₀D₁₄N₂=498.28) | 2-74 | m/z=650.87(C₄₈H₁₈D₁₄N₂=650.34) |
| 2-75 | m/z=574.77(C₄₁H₁₄D₁₄N₂=574.31) | 2-76 | m/z=648.85(C₄₈H₁₆D₁₄N₂=648.33) |
| 2-77 | m/z=650.87(C₄₈H₁₈D₁₄N₂=650.34) | 2-78 | m/z=650.87(C₄₈H₁₈D₁₄N₂=650.34) |
| 2-79 | m/z=650.87(C₄₈H₁₈D₁₄N₂=650.34) | 2-80 | m/z=650.87(C₄₈H₁₈D₁₄N₂=650.34) |
| 2-81 | m/z=650.87(C₄₈H₁₈D₁₄N₂=650.34) | 2-82 | m/z=726.38(C₅₄H₂₂D₁₄N₂=726.98) |
| 2-83 | m/z=726.96(C₅₄H₂₂D₁₄N₂=726.38) | 2-84 | m/z=726.96(C₅₄H₂₂D₁₄N₂=726.38) |
| 2-85 | m/z=724.95(C₅₄H₂₀D₁₄N₂=724.36) | 2-86 | m/z=726.96(C₅₄H₂₂D₁₄N₂=726.38) |
| 2-87 | m/z=726.96(C₅₄H₂₂D₁₄N₂=726.38) | 2-88 | m/z=803.06(C₆₀H₂₆D₁₄N₂=802.41) |
| 2-89 | m/z=803.06(C₆₀H₂₆D₁₄N₂=802.41) | 2-90 | m/z=803.06(C₆₀H₂₆D₁₄N₂=802.41) |
| 2-91 | m/z=803.06(C₆₀H₂₆D₁₄N₂=802.41) | 2-92 | m/z=803.06(C₆₀H₂₆D₁₄N₂=802.41) |
| 2-93 | m/z=508.74(C₃₆D₂₄N₂=508.34) | 2-94 | m/z=668.98(C₄₈D₃₂N₂=668.46) |
| 2-95 | m/z=588.86(C₄₂D₂₈N₂=588.40) | 2-96 | m/z=664.95(C₄₈D₃₀N₂=664.43) |
| 2-97 | m/z=668.98(C₄₈D₃₂N₂=668.46) | 2-98 | m/z=668.98(C₄₈D₃₂N₂=668.46) |
| 2-99 | m/z=668.98(C₄₈D₃₂N₂=668.46) | 2-100 | m/z=668.98(C₄₈D₃₂N₂=668.46) |
| 2-101 | m/z=668.98(C₄₈D₃₂N₂=668.46) | 2-102 | m/z=748.518(C₅₄D₃₆N₂=749.12) |
| 2-103 | m/z=749.10(C₅₄D₃₆N₂=748.51) | 2-104 | m/z=749.10(C₅₄D₃₆N₂=748.51) |
| 2-105 | m/z=745.07(C₅₄D₃₄N₂=744.49) | 2-106 | m/z=749.10(C₅₄D₃₆N₂=748.51) |
| 2-107 | m/z=749.10(C₅₄D₃₆N₂=748.51) | 2-108 | m/z=829.22(C₆₀D₄₀N₂=828.57) |
| 2-109 | m/z=829.22(C₆₀D₄₀N₂=828.57) | 2-110 | m/z=829.22(C₆₀D₄₀N₂=828.57) |
| 2-111 | m/z=829.22(C₆₀D₄₀N₂=828.57) | 2-112 | m/z=680.95(C₄₈D₃₀N₂O=680.42) |
| 2-113 | m/z=697.02(C₄₈D₃₀N₂S=696.40) | 2-114 | m/z=829.22(C₆₀D₄₀N₂=828.57) |
| 2-115 | m/z=632.95(C₄₅D₃₂N₂=632.46) | 2-116 | m/z=713.07(C₅₁D₃₆N₂=712.51) |
| 3-1 | m/z=408.16(C₃₀H₂₀N₂=408.50) | 3-4 | m/z=560.23(C₄₂H₂₈N₂=560.70) |
| 3-5 | m/z=560.23(C₄₂H₂₈N₂=560.70) | 3-22 | m/z=560.23(C₄₂H₂₈N₂=560.70) |
| 3-23 | m/z=560.23(C₄₂H₂₈N₂=560.70) | 3-32 | m/z=560.23(C₄₂H₂₈N₂=560.70) |
| 3-35 | m/z=560.23(C₄₂H₂₈N₂=560.70) | 3-41 | m/z=560.23(C₄₂H₂₈N₂=560.70) |
| 3-61 | m/z=578.34(C₄₂H₁₀D₁₈N₂=578.81) | 3-69 | m/z=570.29(C₄₂H₁₈D₁₀N₂=570.76) |
| 3-77 | m/z=588.40(C₄₂D₂₈N₂=588.87) | 3-96 | m/z=668.46(C₄₈D₃₂N₂, 668.99) |
| 3-99 | m/z=588.40(C₄₂D₂₈N₂, 588.87) | | |

### <Experimental Example 1>

### 1) Manufacture of Organic Light Emitting Device

A glass substrate on which ITO was coated as a thin film to thicknesses of 1,500 Å was ultrasonic cleaned with distilled water. When the cleaning with distilled water was finished, the substrate was ultrasonic cleaned with solvents such as acetone, methanol and isopropyl alcohol, then dried, and then subjected to UVO (ultraviolet/ozone) treatment for 5 minutes using UV in a UV cleaner. After that, the substrate was transferred to a plasma cleaner (PT), then subjected to plasma treatment under vacuum for ITO work function and residual film removal, and transferred to a thermal deposition apparatus for organic deposition.

On the transport ITO electrode (positive electrode), a hole injection layer 2-TNATA (4,4',4"-tris[2-naphthyl(phenyl)amino]triphenylamine) and a hole transport layer NPB (N,N'-di(1-naphthyl)-N,N'-diphenyl-(1,1'-biphenyl)-4,4'-diamine), which are common layers, were formed.

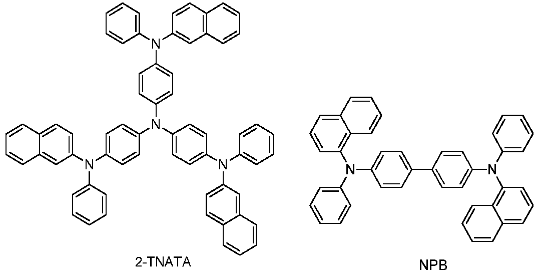

A light emitting layer was thermal vacuum deposited thereon as follows. The light emitting layer was deposited to 350 Å using a compound of the following Table 10 as a host and Ir(ppy)₃ (tris (2-phenylpyridine) iridium) as a green phosphorescent dopant, and by doping the Ir(ppy)₃ to the host by 6%. After that, BCP was deposited to 70 Å as a hole blocking layer, and Alq₃ was deposited to 250 Å thereon as an electron transport layer. Lastly, lithium fluoride (LiF) was deposited on the electron transport layer to a thickness of 10 Å to form an electron injection layer, and then an aluminum (Al) negative electrode was deposited on the electron injection layer to a thickness of 1,200 Å to form a negative electrode, and as a result, an organic electroluminescent device was manufactured.

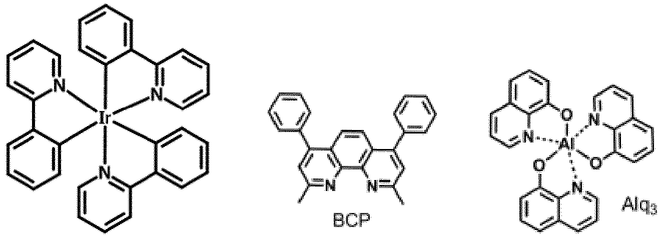

In the following Table 10, a green host was used in Examples and Comparative Examples. Meanwhile, all the organic compounds required to manufacture the OLED were vacuum sublimation purified under 10⁻⁸ torr to 10⁻⁶ torr for each material to be used in the manufacture of the OLED.

### (Comparative Example Compound]

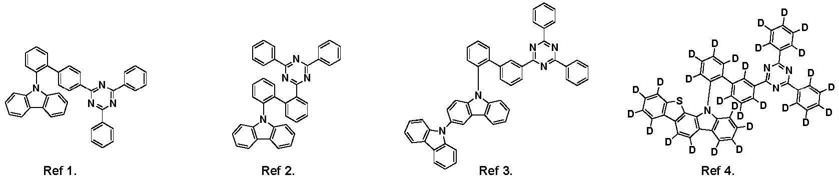

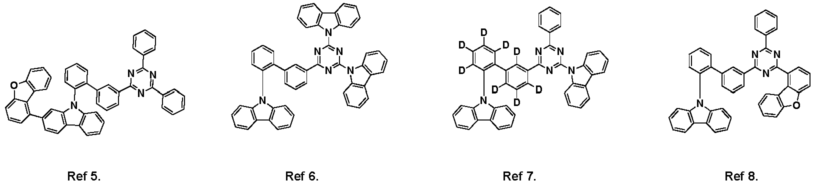

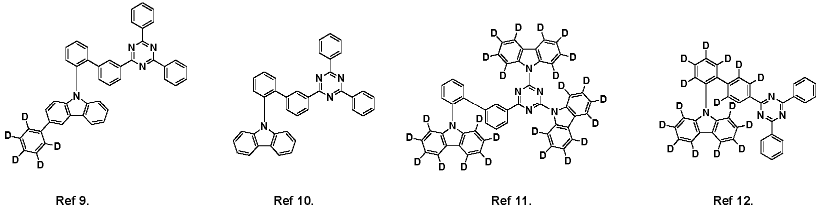

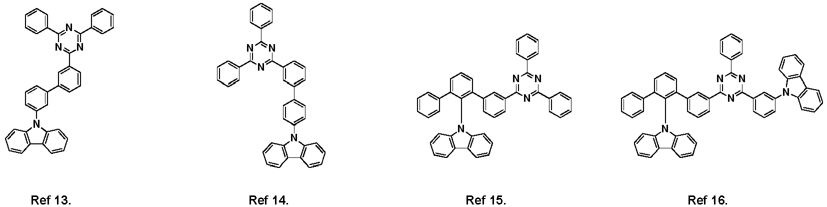

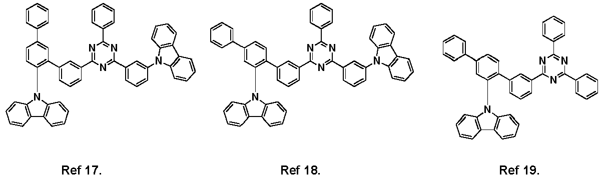

**[Table 10]**

| | Light Emitting Layer Compound | Driving Voltage (V) | Efficiency (cd/A) | Color EL Color | Lifetime (T₉₀) |
|---|---|---|---|---|---|
| Comparative Example 1 | Ref. 1 | 3.83 | 43.9 | Green | 58 |
| Comparative Example 2 | Ref. 2 | 4.26 | 54.3 | | 1 |
| Comparative Example 3 | Ref. 3 | 3.62 | 62.0 | | 76 |
| Comparative Example 4 | Ref. 4 | 3.82 | 55.2 | | 65 |
| Comparative Example 5 | Ref. 5 | 3.65 | 52.4 | | 78 |
| Comparative Example 6 | Ref. 6 | 4.40 | 49.8 | | 32 |
| Comparative Example 7 | Ref. 7 | 4.32 | 50.6 | | 38 |
| Comparative Example 8 | Ref. 8 | 3.96 | 49.1 | | 75 |
| Comparative Example 9 | Ref. 9 | 3.79 | 66.8 | | 72 |
| Comparative Example 10 | Ref. 10 | 3.83 | 67.6 | | 90 |
| Comparative Example 11 | Ref. 11 | 4.38 | 50.3 | | 36 |
| Comparative Example 12 | Ref. 12 | 3.81 | 42.7 | | 64 |
| Comparative Example 13 | Ref. 13 | 3.82 | 40.1 | | 52 |
| Comparative Example 14 | Ref. 14 | 3.64 | 38.5 | | 49 |
| Comparative Example 15 | Ref. 15 | 3.81 | 75.2 | | 51 |
| Comparative Example 16 | Ref. 16 | 3.92 | 71.5 | | 47 |
| Comparative Example 17 | Ref. 17 | 3.72 | 68.8 | | 52 |
| Comparative Example 18 | Ref. 18 | 3.56 | 42.7 | | 41 |
| Comparative Example 19 | Ref. 19 | 3.49 | 45.2 | | 44 |
| Example 1 | 1-1 | 3.74 | 72.1 | | 141 |
| Example 2 | 1-4 | 3.61 | 78.6 | | 128 |
| Example 3 | 1-9 | 3.58 | 80.4 | | 122 |
| Example 4 | 1-13 | 3.51 | 68.2 | | 119 |
| Example 5 | 1-15 | 3.62 | 75.8 | | 122 |
| Example 6 | 1-25 | 3.57 | 77.1 | | 131 |
| Example 7 | 1-29 | 3.65 | 61.7 | | 164 |
| Example 8 | 1-32 | 3.62 | 66.2 | | 160 |
| Example 9 | 1-34 | 3.59 | 67.5 | | 152 |
| Example 10 | 1-43 | 3.69 | 74.3 | | 138 |
| Example 11 | 1-45 | 3.65 | 72.5 | | 144 |
| Example 12 | 1-49 | 3.84 | 75.1 | | 129 |
| Example 13 | 1-56 | 3.70 | 69.9 | | 153 |
| Example 14 | 1-57 | 3.59 | 70.3 | | 168 |
| Example 15 | 1-59 | 3.82 | 84.4 | | 140 |
| Example 16 | 1-61 | 3.85 | 77.6 | | 132 |
| Example 17 | 1-62 | 3.55 | 66.3 | | 154 |
| Example 18 | 1-63 | 3.76 | 72.2 | | 134 |
| Example 19 | 1-69 | 3.44 | 65.1 | | 115 |
| Example 20 | 1-76 | 3.69 | 63.0 | | 146 |
| Example 21 | 1-77 | 3.64 | 64.9 | | 154 |
| Example 22 | 1-78 | 3.62 | 66.2 | | 158 |
| Example 23 | 1-86 | 3.68 | 74.2 | | 152 |
| Example 24 | 1-115 | 3.59 | 70.3 | | 168 |
| Example 25 | 1-121 | 3.79 | 70.0 | | 116 |
| Example 26 | 1-124 | 3.80 | 70.6 | | 125 |
| Example 27 | 1-131 | 3.56 | 73.4 | | 142 |
| Example 28 | 1-134 | 3.55 | 74.1 | | 138 |
| Example 29 | 1-135 | 3.81 | 74.9 | | 122 |
| Example 30 | 1-136 | 3.79 | 75.2 | | 135 |
| Example 31 | 1-140 | 3.58 | 72.4 | | 159 |
| Example 32 | 1-146 | 3.75 | 67.2 | | 121 |
| Example 33 | 1-147 | 3.69 | 65.6 | | 119 |
| Example 34 | 1-148 | 3.64 | 64.9 | | 113 |
| Example 35 | 1-149 | 3.83 | 75.3 | | 119 |
| Example 36 | 1-151 | 3.69 | 70.1 | | 129 |

As seen from the results of Table 10, it was identified that Examples 1 to 36, which are organic light emitting devices using the compound represented by Chemical Formula 1 of the present disclosure as a light emitting layer material (particularly, green phosphorescent host), had a reduced driving voltage, and significantly improved light emission efficiency and lifetime compared to Comparative Examples 1 to 19 that are organic light emitting devices not using the compound represented by Chemical Formula 1 of the present disclosure as a light emitting layer material.

When a strong electron donor and a strong electron acceptor directly bond in a molecule, a decrease in lifetime may occur due to the HOMO-LUMO overlap, and by introducing a biphenyl linker, the lifetime may be increased by offsetting the HOMO-LUMO overlap. Particularly, as the linker of the electron acceptor, the meta form has a structure capable of effectively offsetting the HOMO-LUMO overlap by further alleviating the resonance effect unlike the para or ortho form.

In addition, in the ortho form of the electron donor, the HOMO and the linking group face each other in space due to the spatial steric hindrance. This leads to a long lifetime by suppressing through bond charge transfer (TBCT), causes through space charge transfer (TSCT), exhibits thermally activated delayed fluorescence (TADF) properties, and may effectively exhibit high efficiency properties through spatial charge transfer between the electron donor and the electron acceptor.

A compound bonding with hydrogen and a compound substituted with deuterium are generally different in a thermodynamic behavior. This is due to the fact that the mass of deuterium atom is two times larger than the mass of hydrogen, and by the difference in the atomic mass, deuterium has lower vibration energy. In addition, carbon and deuterium have a shorter bond length and stronger dissociation energy used to break the bond compared to carbon and hydrogen. This is due to the fact that deuterium has a smaller Van der Waals radius compared to hydrogen, and the bond between carbon and deuterium has a narrower elongation amplitude.

When compared with a compound not substituted with deuterium, the compound of the present disclosure substituted with deuterium may have higher light emission efficiency by weakened intermolecular Van der Waals force caused by a shorter carbon-deuterium bond length than a carbon-hydrogen bond length. In addition, zero point energy, that is, ground state energy is lowered, and as the carbon-deuterium bond length decreases, a molecular hardcore volume decreases, and accordingly, electronical polarizability may be reduced, and by weakening intermolecular interactions, a volume of a thin film may be increased. Such properties create an amorphous state of the thin film, inducing an effect of lowering crystallinity. In conclusion, substitution with deuterium may be effective in improving heat resistance of an organic light emitting device, and lifetime and driving properties of the device may be improved thereby. In addition, the effect of improving device properties obtained from substitution with deuterium is improved as the deuterium substitution ratio increases in the molecule.

For materials used in an organic light emitting device, stability in the ground state and the excited state is an important factor for a lifetime of the device. A linker is at a position where the HOMO and the LUMO cross, and this is a form in which substantial structural changes occur in the molecular structure when the molecule becomes excited, which may significantly affect a lifetime of the device. Since the compound of the present disclosure has the linker substituted with deuterium having a larger molecular weight, a change in the vibrational frequency is reduced to lower molecular energy, and thus molecular stability increases. In addition, it may be identified that, since single bond dissociation energy between carbon and deuterium is higher than single bond dissociation energy between carbon and hydrogen, thermal stability of the molecule increases, thereby improving a lifetime of the device.

It may be identified that, when substituted with deuterium, charge mobility increases as the intermolecular distance decreases, thereby improving a lifetime of the device. As shown in Examples 20 to 22, it may be identified that device properties are improved as the deuterium substitution ratio increases in the molecule.

In Comparative Examples 1 to 3, 5, 6, 8, 10 and 13 to 19 not substituted with deuterium, electron mobility is faster than hole mobility in the molecule, and thus it is believed that the recombination zone leans toward the hole transport layer (HTL) side, and as a result, efficiency and lifetime of the device are reduced.

On the other hand, in Comparative Example 9, the phenyl group corresponding to the HOMO is substituted with deuterium, however, deuterium is not included in the carbazole and the linking group, and it was difficult to identify the effect since the proportion of deuterium in the stability of the ground state and the excited state is small.

This implies that, even having similar structures, compounds may have different properties depending on the substitution with deuterium.

On the other hand, in Comparative Examples 4, 6, 7 and 11, it was able to be identified that strong HT properties were obtained by adding a strong electron donor substituent in the molecule, and accordingly, electrons were not effectively stabilized, and the lifetime was reduced thereby.

On the other hand, in Comparative Examples 1 and 2, 12, 13 and 14, it was able to be identified that the strong HOMO-LUMO overlap was not effectively offset, and the lifetime was inferior when the compound was used in the device. In addition, in Comparative Example 2, the electron acceptor includes an ortho form, and in this case, it was able to be identified that the device exhibited very poor lifetime properties due to too strong steric hindrance.

On the other hand, in Comparative Examples 18 and 19, it was able to be identified that the linking group was included in a para terphenyl form, and efficiency was poor when used in the device. In general, para terphenyl has properties of increasing molecular stability and having high thermal stability due to the strong resonance effect. However, the strong resonance structure also has a property of lowering a triplet excited state (T1 state), and a host having such a low triplet excited state causes an energy back transfer of the dopant, which acts competitively with light emission of the dopant. As a result, it was able to be identified that inferior efficiency was obtained when a material having a linking group with such a strong resonance structure is used in the device.

### <Experimental Example 2>

### 1) Manufacture of Organic Light Emitting Device

A glass substrate on which ITO was coated as a thin film to thicknesses of 1,500 Å was ultrasonic cleaned with distilled water. When the cleaning with distilled water was finished, the substrate was ultrasonic cleaned with solvents such as acetone, methanol and isopropyl alcohol, then dried, and then subjected to UVO (ultraviolet/ozone) treatment for 5 minutes using UV in a UV cleaner. After that, the substrate was transferred to a plasma cleaner (PT), then subjected to plasma treatment under vacuum for ITO work function and residual film removal, and transferred to a thermal deposition apparatus for organic deposition.

On the transport ITO electrode (positive electrode), a hole injection layer 2-TNATA (4,4',4"-tris[2-naphthyl(phenyl)amino]triphenylamine) and a hole transport layer NPB (N,N'-di(1-naphthyl)-N,N'-diphenyl-(1,1'-biphenyl)-4,4'-diamine), which are common layers, were formed.

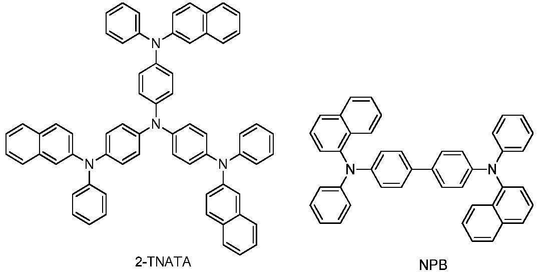

A light emitting layer was thermal vacuum deposited thereon as follows. As the light emitting layer, two types of compounds (N-host and P-host) described in the following Table 11 were premixed in a weight ratio described in the following Table 11 and then deposited to 350 Å in one source of supply as a host, and, as a green phosphorescent dopant, Ir (ppy)₃ was doped and deposited by 6% of the deposited thickness of the light emitting layer. After that, BCP was deposited to 70 Å as a hole blocking layer, and Alq₃ was deposited to 250 Å thereon as an electron transport layer. Lastly, lithium fluoride (LiF) was deposited on the electron transport layer to a thickness of 10 Å to form an electron injection layer, and then an aluminum (Al) negative electrode was deposited on the electron injection layer to a thickness of 1,200 Å to form a negative electrode, and as a result, an organic electroluminescent device was manufactured.

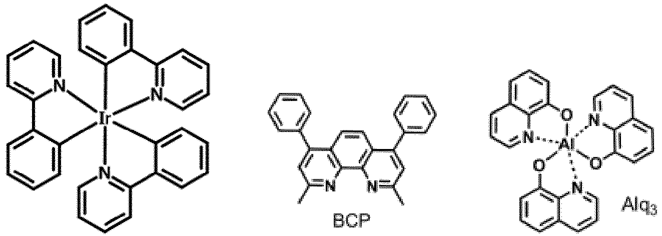

Meanwhile, all the organic compounds required to manufacture the OLED were vacuum sublimation purified under 10⁻⁸ torr to 10⁻⁶ torr for each material to be used in the manufacture of the OLED.

For each of the organic electroluminescent devices manufactured as above, electroluminescent (EL) properties were measured using M7000 manufactured by McScience Inc., and with the measurement results, T₉₀ was measured when standard luminance was 12,000 cd/m² through a lifetime measurement system (M6000) manufactured by McScience Inc.

Results of measuring driving voltage, light emission efficiency, color and lifetime of the organic light emitting devices manufactured according to the present disclosure are shown in the following Table 11.

**[Table 11]**

| | Light Emitting Layer Compound | Ratio | Driving Voltage (V) | Efficiency (cd/A) | Color EL Color | Lifetime (T₉₀) |
|---|---|---|---|---|---|---|
| Comparative Example 20 | [Ref.1]:2-93 | 1:1 | 3.64 | 49.9 | Green | 80 |
| Comparative Example 21 | | 1:2 | 3.69 | 48.8 | | 86 |
| Comparative Example 22 | | 1:3 | 3.73 | 47.2 | | 90 |
| Comparative Example 23 | [Ref.2]:2-93 | 1:1 | 4.01 | 60.0 | | 1 |
| Comparative Example 24 | | 1:2 | 4.09 | 59.5 | | 1 |
| Comparative Example 25 | | 1:3 | 4.13 | 58.4 | | 1 |
| Comparative Example 26 | [Ref.3]:2-93 | 1:1 | 3.41 | 69.9 | | 113 |
| Comparative Example 27 | | 1:2 | 3.46 | 68.2 | | 126 |
| Comparative Example 28 | | 1:3 | 3.50 | 67.0 | | 137 |
| Comparative Example 29 | [Ref.4]:2-93 | 1:1 | 3.61 | 62.1 | | 94 |
| Comparative Example 30 | | 1:2 | 3.64 | 60.5 | | 101 |
| Comparative Example 31 | | 1:3 | 3.69 | 59.2 | | 115 |
| Comparative Example 32 | [Ref.5]:2-93 | 1:1 | 3.39 | 60.6 | | 116 |
| Comparative Example 33 | | 1:2 | 3.46 | 59.4 | | 128 |
| Comparative Example 34 | | 1:3 | 3.53 | 57.3 | | 140 |
| Comparative Example 35 | [Ref.6]:2-93 | 1:1 | 4.22 | 58.7 | | 46 |
| Comparative Example 36 | | 1:2 | 4.27 | 56.8 | | 52 |
| Comparative Example 37 | | 1:3 | 4.32 | 54.6 | | 63 |
| Comparative Example 38 | [Ref.7]:2-93 | 1:1 | 4.02 | 59.3 | | 59 |
| Comparative Example 39 | | 1:2 | 4.07 | 57.9 | | 67 |
| Comparative Example 40 | | 1:3 | 4.18 | 55.5 | | 80 |
| Comparative Example 41 | [Ref.8]:2-93 | 1:1 | 3.66 | 58.1 | | 112 |
| Comparative Example 42 | | 1:2 | 3.72 | 56.7 | | 119 |
| Comparative Example 43 | | 1:3 | 3.78 | 54.4 | | 132 |
| Comparative Example 44 | [Ref.9]:2-93 | 1:1 | 3.60 | 75.4 | | 105 |
| Comparative Example 45 | | 1:2 | 3.65 | 74.5 | | 118 |
| Comparative Example 46 | | 1:3 | 3.69 | 72.3 | | 133 |
| Comparative Example 47 | [Ref.10]:2-93 | 1:1 | 3.38 | 80.6 | | 134 |
| Comparative Example 48 | | 1:2 | 3.51 | 79.3 | | 143 |
| Comparative Example 49 | | 1:3 | 3.64 | 77.8 | | 150 |
| Comparative Example 50 | [Ref.11]:2-93 | 1:1 | 4.08 | 56.3 | | 63 |
| Comparative Example 51 | | 1:2 | 4.15 | 54.1 | | 72 |
| Comparative Example 52 | | 1:3 | 4.19 | 52.4 | | 85 |
| Comparative Example 53 | [Ref.12]:2-93 | 1:1 | 3.50 | 49.3 | | 91 |
| Comparative Example 54 | | 1:2 | 3.61 | 47.1 | | 99 |
| Comparative Example 55 | | 1:3 | 3.69 | 45.6 | | 105 |
| Comparative Example 56 | [Ref.13]:2-93 | 1:1 | 3.55 | 46.5 | | 76 |
| Comparative Example 57 | | 1:2 | 3.62 | 44.0 | | 85 |
| Comparative Example 58 | | 1:3 | 3.71 | 42.1 | | 91 |
| Comparative Example 59 | [Ref.14]:2-93 | 1:1 | 3.49 | 43.2 | | 72 |
| Comparative Example 60 | | 1:2 | 3.53 | 41.9 | | 80 |
| Comparative Example 61 | | 1:3 | 3.58 | 40.8 | | 86 |
| Comparative Example 62 | [Ref.15]:2-93 | 1:1 | 3.62 | 82.4 | | 85 |
| Comparative Example 63 | | 1:2 | 3.65 | 81.8 | | 92 |
| Comparative Example 64 | | 1:3 | 3.71 | 80.6 | | 97 |
| Comparative Example 65 | [Ref.16]:2-93 | 1:1 | 3.71 | 78.9 | | 74 |
| Comparative Example 66 | | 1:2 | 3.75 | 78.2 | | 80 |
| Comparative Example 67 | | 1:3 | 3.80 | 77.4 | | 86 |
| Comparative Example 68 | [Ref.17]:2-93 | 1:1 | 3.51 | 74.7 | | 92 |
| Comparative Example 69 | | 1:2 | 3.55 | 74.0 | | 98 |
| Comparative Example 70 | | 1:3 | 3.59 | 73.4 | | 105 |
| Comparative Example 71 | [Ref.18] :2-93 | 1:1 | 3.39 | 48.1 | | 63 |
| Comparative Example 72 | | 1:2 | 3.42 | 47.5 | | 68 |
| Comparative Example 73 | | 1:3 | 3.46 | 46.3 | | 74 |
| Comparative Example 74 | [Ref.19]:2-93 | 1:1 | 3.28 | 51.3 | | 68 |
| Comparative Example 75 | | 1:2 | 3.33 | 50.9 | | 75 |
| Comparative Example 76 | | 1:3 | 3.37 | 50.3 | | 83 |
| Example 37 | 1-1:2-1 | 1:1 | 3.33 | 82.6 | | 183 |
| Example 38 | | 1:2 | 3.48 | 81.4 | | 199 |
| Example 39 | | 1:3 | 3.59 | 79.9 | | 211 |
| Example 40 | 1-1:2-93 | 1:1 | 3.29 | 84.1 | | 218 |
| Example 41 | | 1:2 | 3.44 | 82.7 | | 226 |
| Example 42 | | 1:3 | 3.56 | 80.3 | | 235 |
| Example 43 | 1-4:2-93 | 1:1 | 3.27 | 86.0 | | 192 |
| Example 44 | | 1:2 | 3.41 | 85.3 | | 199 |
| Example 45 | | 1:3 | 3.49 | 82.4 | | 208 |
| Example 46 | 1-9:2-100 | 1:1 | 3.34 | 90.4 | | 197 |
| Example 47 | | 1:2 | 3.38 | 89.1 | | 204 |
| Example 48 | | 1:3 | 3.43 | 87.2 | | 212 |
| Example 49 | 1-13:3-77 | 1:1 | 3.28 | 77.0 | | 189 |
| Example 50 | | 1:2 | 3.32 | 75.9 | | 194 |
| Example 51 | | 1:3 | 3.37 | 74.6 | | 207 |
| Example 52 | 1-29:2-97 | 1:1 | 3.38 | 72.2 | | 238 |
| Example 53 | | 1:2 | 3.45 | 70.9 | | 253 |
| Example 54 | | 1:3 | 3.51 | 69.3 | | 264 |
| Example 55 | 1-29:3-96 | 1:1 | 3.20 | 68.4 | | 256 |
| Example 56 | | 1:2 | 3.29 | 67.7 | | 271 |
| Example 57 | | 1:3 | 3.43 | 65.6 | | 283 |
| Example 58 | 1-32:2-100 | 1:1 | 3.39 | 75.5 | | 227 |
| Example 59 | | 1:2 | 3.43 | 74.9 | | 236 |
| Example 60 | | 1:3 | 3.47 | 73.6 | | 246 |
| Example 61 | 1-32:3-99 | 1:1 | 3.33 | 72.9 | | 250 |
| Example 62 | | 1:2 | 3.38 | 71.7 | | 262 |
| Example 63 | | 1:3 | 3.43 | 70.5 | | 271 |
| Example 64 | 1-34:2-100 | 1:1 | 3.35 | 74.3 | | 214 |
| Example 65 | | 1:2 | 3.39 | 72.1 | | 223 |
| Example 66 | | 1:3 | 3.45 | 70.6 | | 230 |
| Example 67 | 1-49:2-112 | 1:1 | 3.51 | 86.1 | | 186 |
| Example 68 | | 1:2 | 3.57 | 84.3 | | 201 |
| Example 69 | | 1:3 | 3.63 | 83.0 | | 215 |
| Example 70 | 1-49:3-77 | 1:1 | 3.38 | 80.6 | | 201 |
| Example 71 | | 1:2 | 3.44 | 79.9 | | 217 |
| Example 72 | | 1:3 | 3.52 | 78.1 | | 229 |
| Example 73 | 1-56:2-114 | 1:1 | 3.36 | 78.1 | | 239 |
| Example 74 | | 1:2 | 3.43 | 76.3 | | 251 |
| Example 75 | | 1:3 | 3.48 | 74.8 | | 263 |
| Example 76 | 1-57:2-101 | 1:1 | 3.34 | 79.5 | | 238 |
| Example 77 | | 1:2 | 3.39 | 78.4 | | 455 |
| Example 78 | | 1:3 | 3.44 | 76.3 | | 269 |
| Example 79 | 1-57:3-96 | 1:1 | 3.25 | 79.1 | | 262 |
| Example 80 | | 1:2 | 3.31 | 76.0 | | 280 |
| Example 81 | | 1:3 | 3.36 | 74.8 | | 293 |
| Example 82 | 1-59:2-93 | 1:1 | 3.57 | 93.4 | | 199 |
| Example 83 | | 1:2 | 3.64 | 91.5 | | 214 |
| Example 84 | | 1:3 | 3.69 | 90.3 | | 222 |
| Example 85 | 1-59:3-114 | 1:1 | 3.50 | 90.7 | | 218 |
| Example 86 | | 1:2 | 3.55 | 89.1 | | 231 |
| Example 87 | | 1:3 | 3.63 | 87.6 | | 249 |
| Example 88 | 1-61:2-100 | 1:1 | 3.59 | 85.1 | | 195 |
| Example 89 | | 1:2 | 3.63 | 84.2 | | 206 |
| Example 90 | | 1:3 | 3.70 | 83.0 | | 214 |
| Example 91 | 1-62:2-100 | 1:1 | 3.33 | 75.6 | | 226 |
| Example 92 | | 1:2 | 3.37 | 74.3 | | 240 |
| Example 93 | | 1:3 | 3.42 | 72.1 | | 255 |
| Example 94 | 1-63:2-100 | 1:1 | 3.46 | 79.4 | | 201 |
| Example 95 | | 1:2 | 3.54 | 78.0 | | 213 |
| Example 96 | | 1:3 | 3.61 | 76.2 | | 227 |
| Example 97 | 1-69:3-77 | 1:1 | 3.03 | 70.0 | | 179 |
| Example 98 | | 1:2 | 3.08 | 69.5 | | 186 |
| Example 99 | | 1:3 | 3.20 | 67.1 | | 192 |
| Example 100 | 1-77:2-100 | 1:1 | 3.40 | 74.4 | | 219 |
| Example 101 | | 1:2 | 3.45 | 74.1 | | 228 |
| Example 102 | | 1:3 | 3.49 | 73.4 | | 235 |
| Example 103 | 1-77:3-99 | 1:1 | 3.35 | 72.1 | | 242 |
| Example 104 | | 1:2 | 3.40 | 71.0 | | 254 |
| Example 105 | | 1:3 | 3.46 | 70.3 | | 262 |
| Example 106 | 1-86:2-101 | 1:1 | 3.44 | 81.6 | | 216 |
| Example 107 | | 1:2 | 3.52 | 80.1 | | 221 |
| Example 108 | | 1:3 | 3.57 | 78.6 | | 229 |
| Example 109 | 1-115:3-77 | 1:1 | 3.31 | 76.6 | | 251 |
| Example 110 | | 1:2 | 3.35 | 75.8 | | 258 |
| Example 111 | | 1:3 | 3.40 | 74.6 | | 266 |
| Example 112 | 1-135:2-100 | 1:1 | 3.47 | 83.4 | | 188 |
| Example 113 | | 1:2 | 3.54 | 82.3 | | 196 |
| Example 114 | | 1:3 | 3.61 | 80.1 | | 204 |
| Example 115 | 1-136:3-77 | 1:1 | 3.41 | 85.1 | | 207 |
| Example 116 | | 1:2 | 3.50 | 83.5 | | 216 |
| Example 117 | | 1:3 | 3.58 | 82.7 | | 221 |
| Example 118 | 1-140:3-99 | 1:1 | 3.29 | 82.2 | | 242 |
| Example 119 | | 1:2 | 3.35 | 80.5 | | 256 |
| Example 120 | | 1:3 | 3.39 | 79.6 | | 268 |
| Example 121 | 1-146:2-100 | 1:1 | 3.48 | 76.6 | | 175 |
| Example 122 | | 1:2 | 3.53 | 75.1 | | 183 |
| Example 123 | | 1:3 | 3.59 | 73.4 | | 198 |
| Example 124 | 1-147:3-99 | 1:1 | 3.38 | 73.9 | | 175 |
| Example 125 | | 1:2 | 3.44 | 72.6 | | 182 |
| Example 126 | | 1:3 | 3.50 | 71.8 | | 188 |
| Example 127 | 1-148:3-114 | 1:1 | 3.38 | 4.4 | | 170 |
| Example 128 | | 1:2 | 3.44 | 72.1 | | 175 |
| Example 129 | | 1:3 | 3.48 | 70.3 | | 188 |

The comparative compounds used in Table 11 are the same as the comparative compounds of Table 10.

Comparing the results of Table 11 with the results of Table 10, it may be identified that, when using the heterocyclic compound represented by Chemical Formula 1 and the heterocyclic compound represented by Chemical Formula 2 or 3 of the present application at the same time as a light emitting layer host, specifically, when using the heterocyclic compound represented by Chemical Formula 1 as an N-type host and the heterocyclic compound represented by Chemical Formula 2 or 3 as a P-type host, driving voltage, light emission efficiency and lifetime are all improved.

From this result, it may be expected that an exciplex phenomenon occurs when the two compounds are included at the same time.

The exciplex phenomenon is a phenomenon of releasing energy having a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transport ability and an acceptor (n-host) having a favorable electron transport ability are used as a host of a light emitting layer, holes are injected to the p-host and electrons are injected to the n-host, and thus a driving voltage may be lowered, which resultantly helps with enhancement in the lifetime. In the present disclosure, it was able to be identified that excellent device properties were obtained when the heterocyclic compound represented by Chemical Formula 1 and the heterocyclic compound represented by Chemical Formula 2 or 3 were used together as a light emitting layer host since the heterocyclic compound represented by Chemical Formula 1 serves as an acceptor and the heterocyclic compound represented by Chemical Formula 2 or 3 serves as a donor.

On the other hand, it may be seen that, when each of the comparative example compounds is used in combination with the compound of Chemical Formula 2 or 3 (Comparative Examples 20 to 76), performance in terms of driving voltage, light emission efficiency and lifetime declines compared to when the heterocyclic compound represented by Chemical Formula 1 according to the present disclosure is used.

In other words, it may be identified that, when the heterocyclic compound represented by Chemical Formula 1 and the heterocyclic compound represented by Chemical Formula 2 or 3 of the present disclosure are used at the same time as a host of a light emitting layer, significantly superior driving voltage, light emission efficiency and lifetime are obtained.

### [Reference Numeral]

- 100:: Substrate
- 200:: Positive Electrode
- 300:: Organic Material Layer
- 301:: Hole Injection Layer
- 302:: Hole Transport Layer
- 303:: Light Emitting Layer
- 304:: Hole Blocking Layer
- 305:: Electron Transport Layer
- 306:: Electron Injection Layer
- 400:: Negative Electrode

## Claims

1. A heterocyclic compound represented by the following Chemical Formula 1: 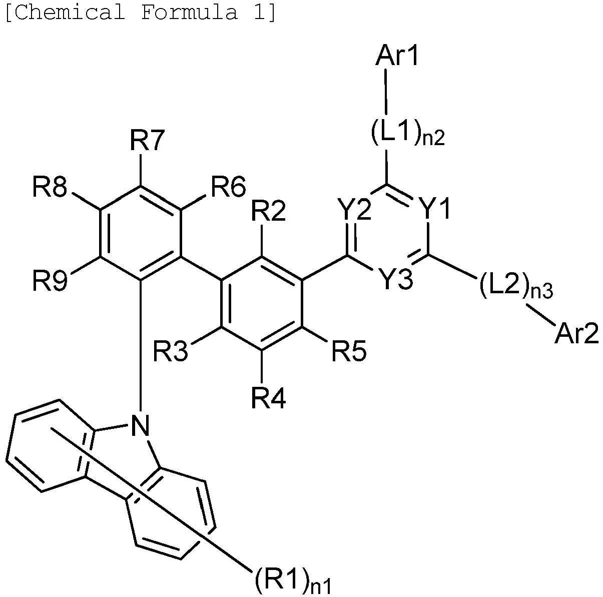 wherein, in Chemical Formula 1,
Y1 to Y3 are the same as or different from each other, and each independently CH; or N;
at least one of Y1 to Y3 is N;
Ar1 and Ar2 are the same as or different from each other, and each independently a substituted or unsubstituted C6 to C60 aryl group; or a substituted or unsubstituted C2 to C60 heteroaryl group;
L1 and L2 are the same as or different from each other, and each independently a single bond; a substituted or unsubstituted C6 to C60 arylene group; or a substituted or unsubstituted C2 to C60 heteroarylene group;
R1s are the same as or different from each other, and each independently hydrogen; deuterium; a halogen; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group;
R2 to R9 are the same as or different from each other, and each independently hydrogen; deuterium; a C1 to C60 alkyl group unsubstituted or substituted with deuterium; a C3 to C60 cycloalkyl group unsubstituted or substituted with deuterium; a C2 to C60 heterocycloalkyl group unsubstituted or substituted with deuterium; a C6 to C60 aryl group unsubstituted or substituted with deuterium; or a C2 to C60 heteroaryl group unsubstituted or substituted with deuterium;
at least one of R2 to R9 is deuterium or a C6 to C60 aryl group substituted with deuterium;
n1 is an integer of 0 to 8;
n2 and n3 are the same as or different from each other, and each independently an integer of 0 to 4; and
when n1, n2 and n3 are 2 or greater, R1s, L1s and L2s are the same as or different from each other.

2. The heterocyclic compound of claim 1, wherein Ar1 and Ar2 are represented by any one of the following Chemical Formula 1-1 to Chemical Formula 1-3: 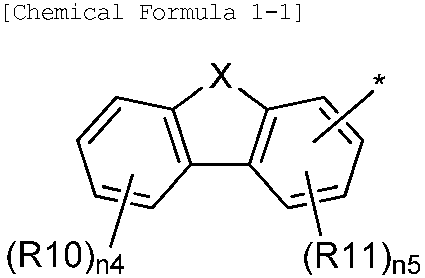 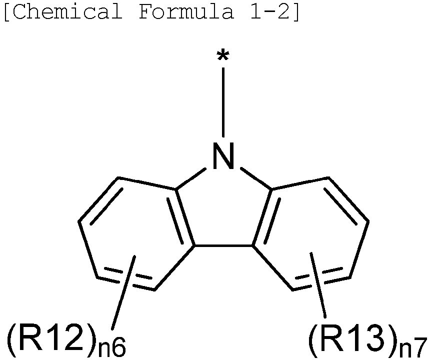
[Chemical Formula 1-3] Ar3-*
in Chemical Formula 1-1 to Chemical Formula 1-3,
X is O or S;
Ar3 is a substituted or unsubstituted C6 to C60 aryl group;
R10 to R13 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring; or a substituted or unsubstituted C2 to C60 heteroring;
* is a site linked to Chemical Formula 1;
n4, n6 and n7 are the same as or different from each other, and each independently an integer of 0 to 4;
n5 is an integer of 0 to 3; and
when n4 to n7 are 2 or greater, R10s to R13s are the same as or different from each other.

3. The heterocyclic compound of claim 2, wherein, when Ar1 or Ar2 of Chemical Formula 1 is represented by Chemical Formula 1-2, L1 and L2 of Chemical Formula 1 are the same as or different from each other, and each independently a substituted or unsubstituted C6 to C60 arylene group; or a substituted or unsubstituted C2 to C60 heteroarylene group.

4. The heterocyclic compound of claim 1, wherein R1s are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group.

5. The heterocyclic compound of claim 1, wherein the heterocyclic compound represented by Chemical Formula 1 has a deuterium content of 1% to 100% based on a total number of hydrogen atoms and deuterium atoms.

6. The heterocyclic compound of claim 1, wherein the heterocyclic compound represented by Chemical Formula 1 is represented by any one of the following compounds:

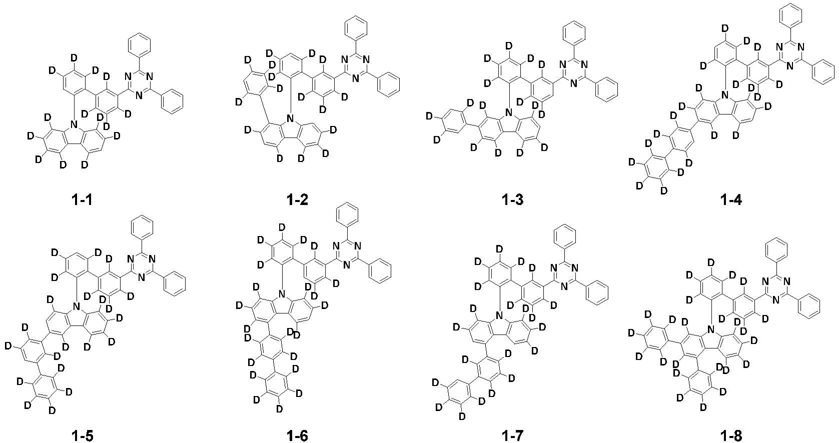

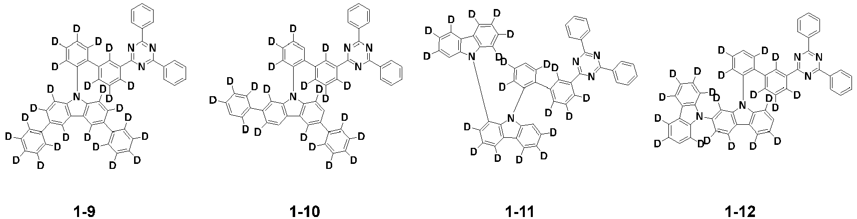

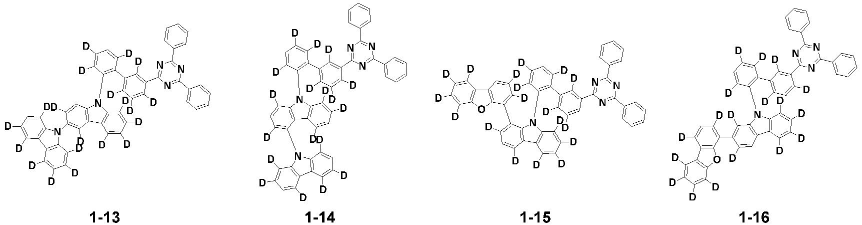

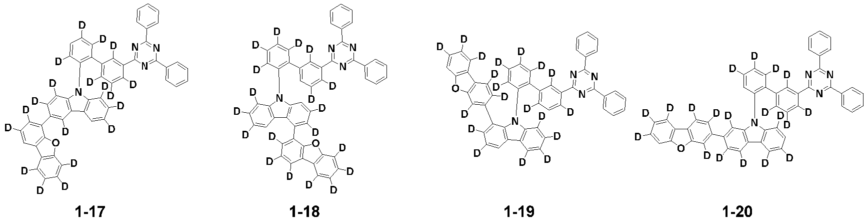

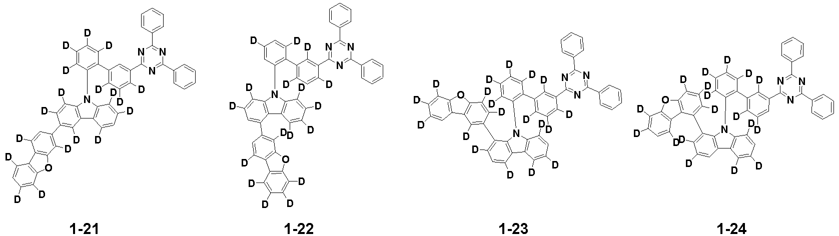

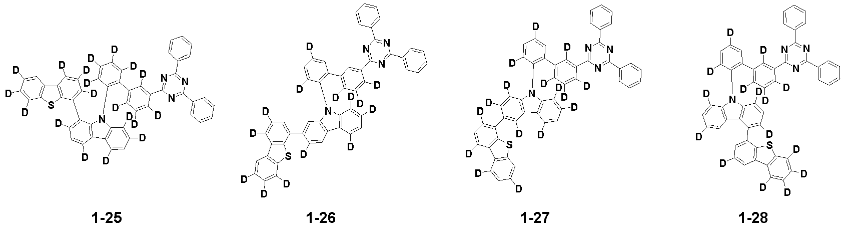

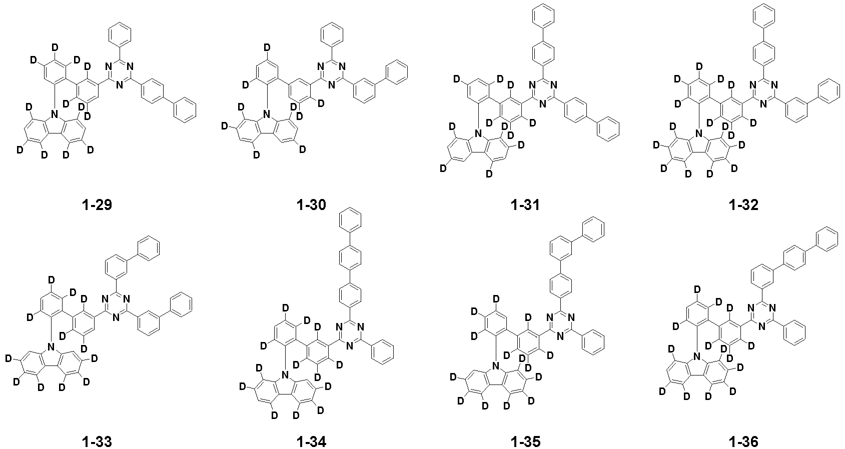

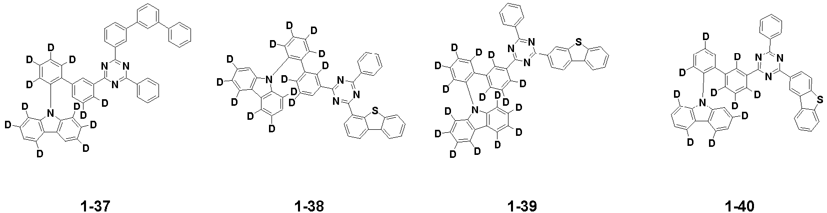

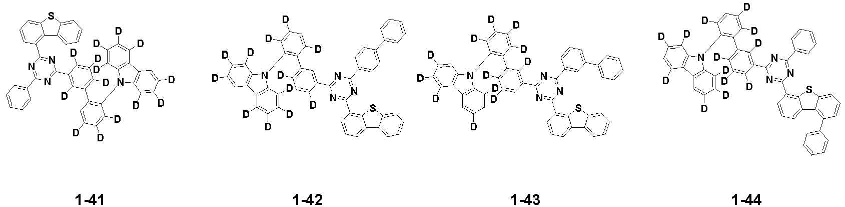

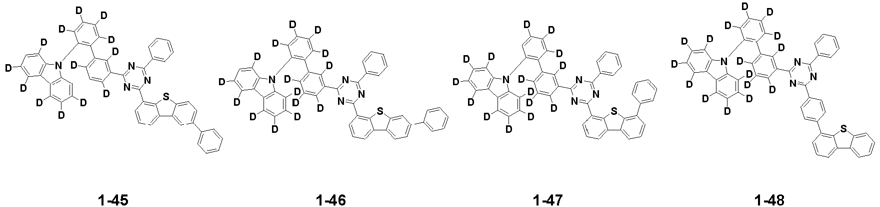

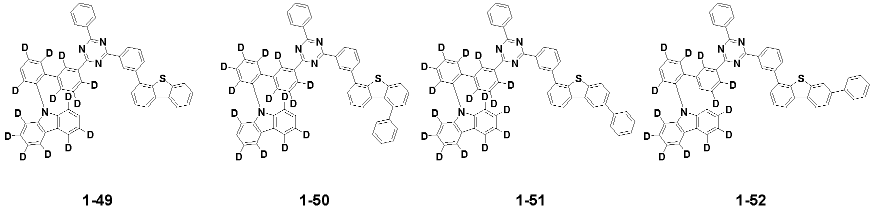

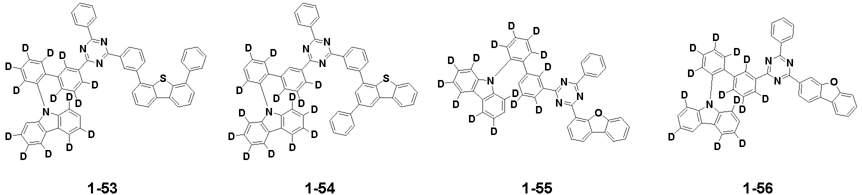

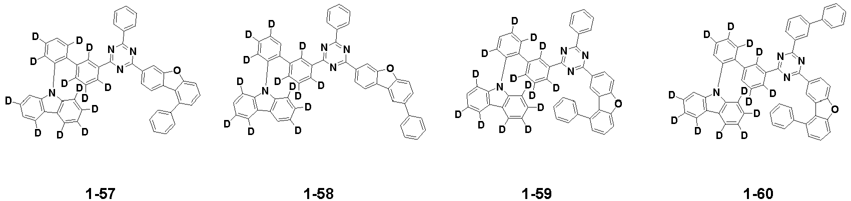

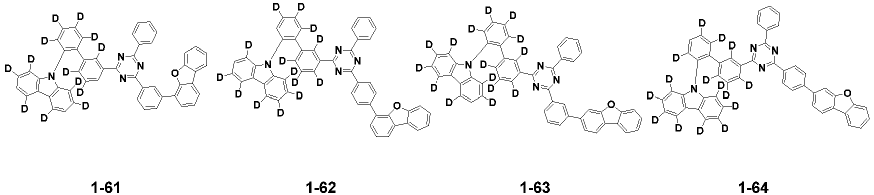

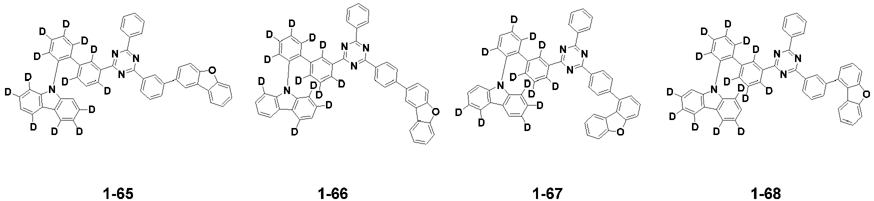

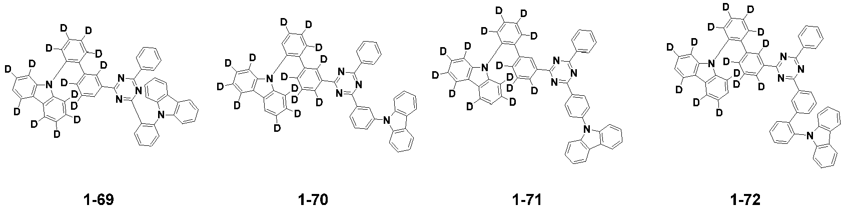

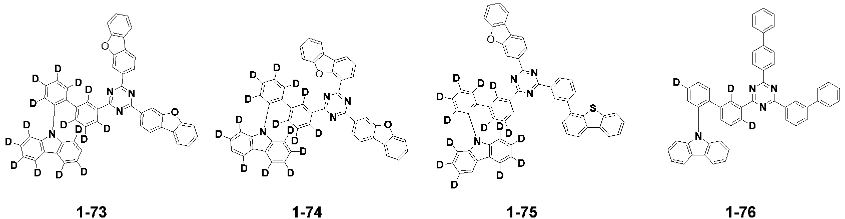

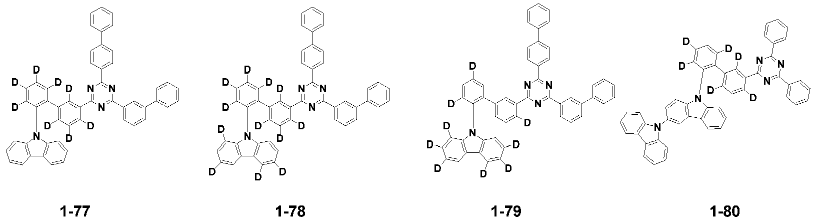

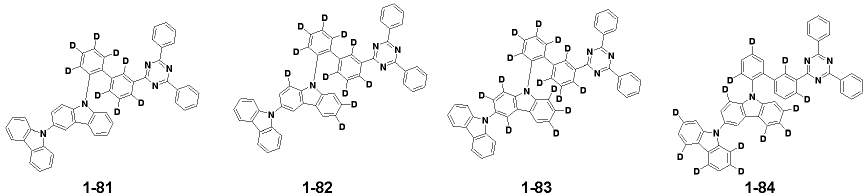

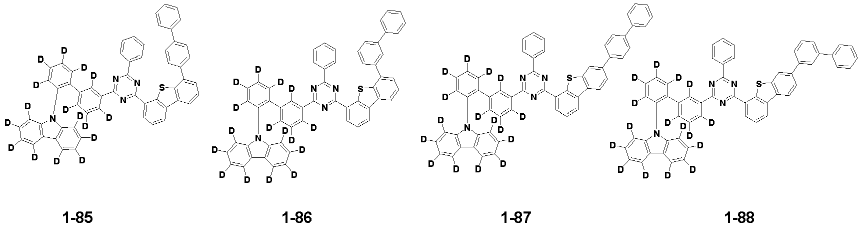

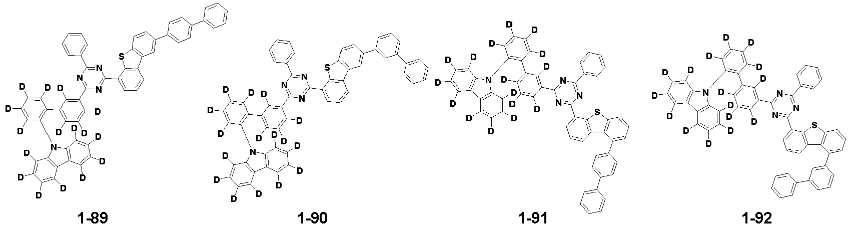

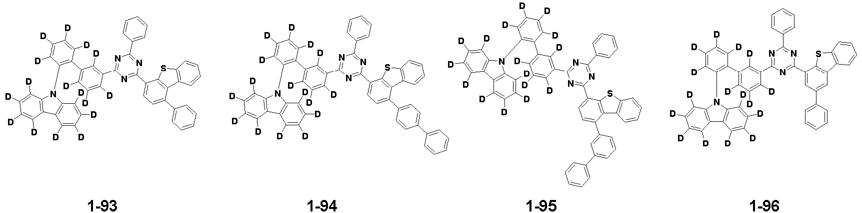

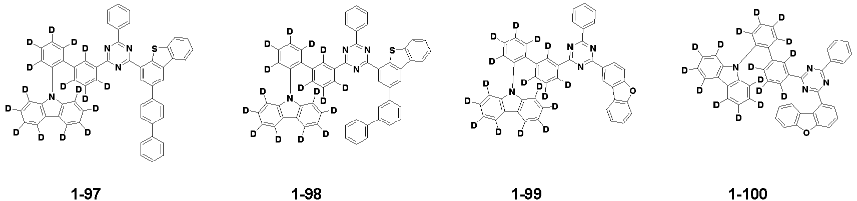

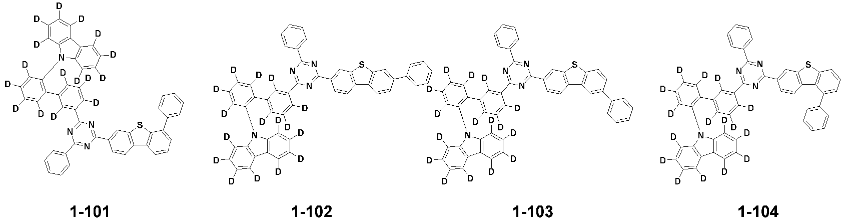

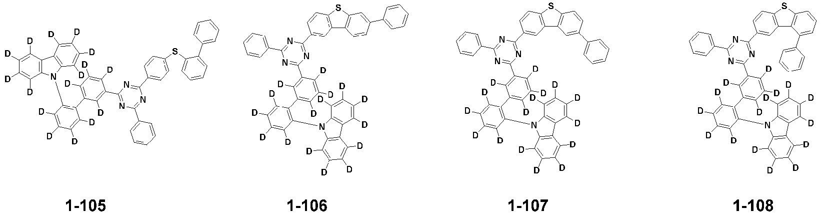

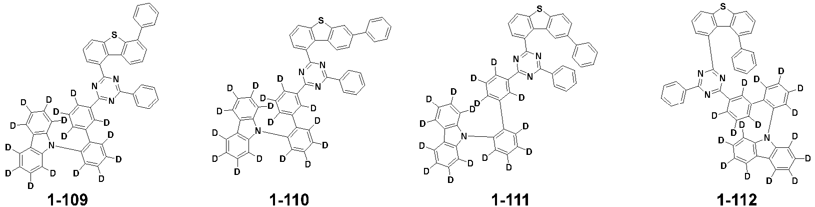

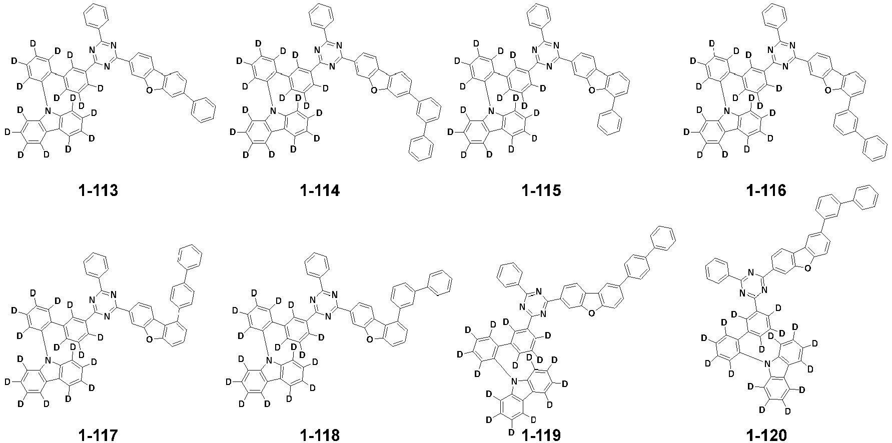

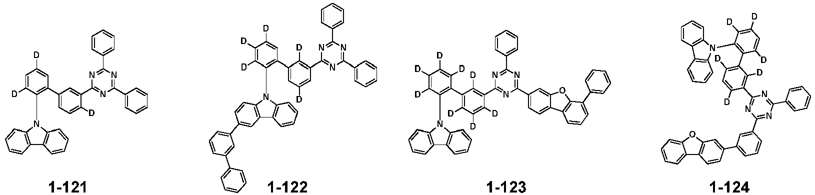

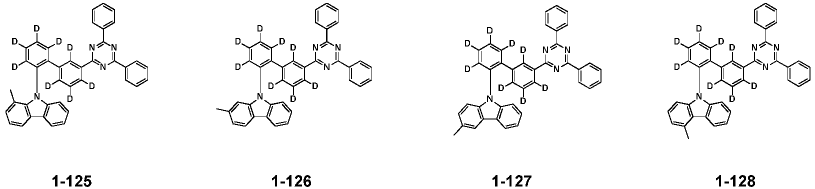

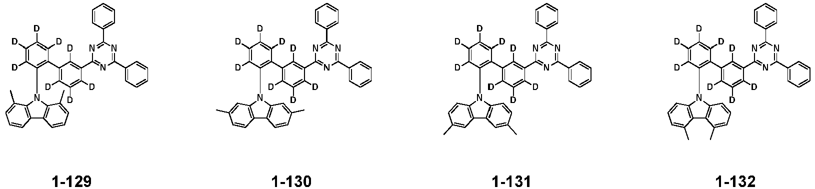

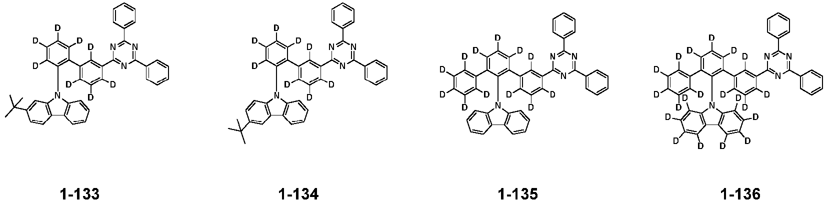

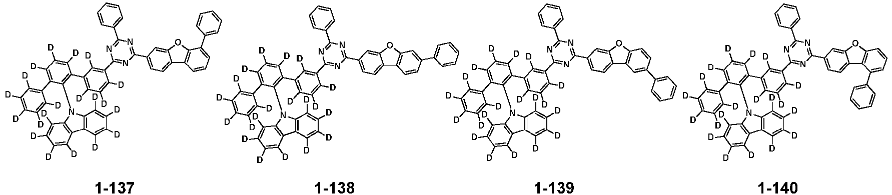

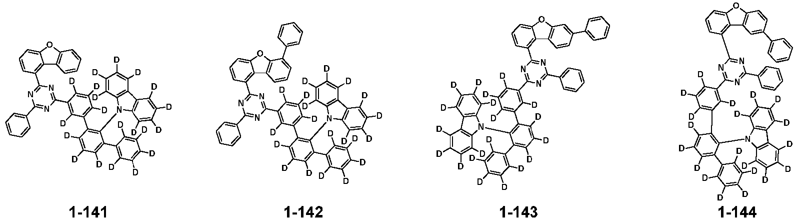

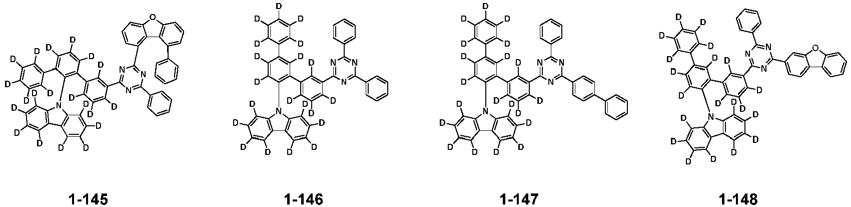

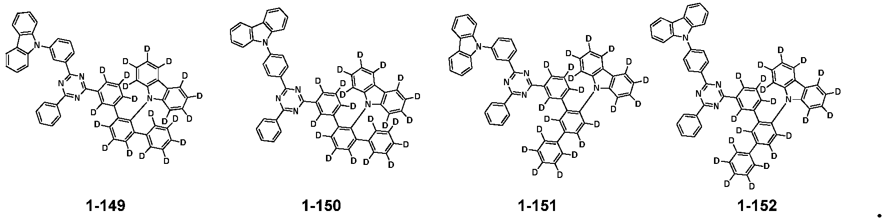

7. An organic light emitting device comprising:
a first electrode;
a second electrode provided opposite to the first electrode; and
one or more organic material layers provided between the first electrode and the second electrode,
wherein one or more layers of the organic material layers include the heterocyclic compound of any one of claims 1 to 6.

8. The organic light emitting device of claim 7, wherein the organic material layer includes a light emitting layer, and the light emitting layer includes the heterocyclic compound represented by Chemical Formula 1.

9. The organic light emitting device of claim 7, wherein the organic material layer includes a light emitting layer, the light emitting layer includes a host material, and the host material includes the heterocyclic compound represented by Chemical Formula 1.

10. The organic light emitting device of claim 7, wherein the organic material layer further includes a heterocyclic compound represented by the following Chemical Formula 2 or Chemical Formula 3: 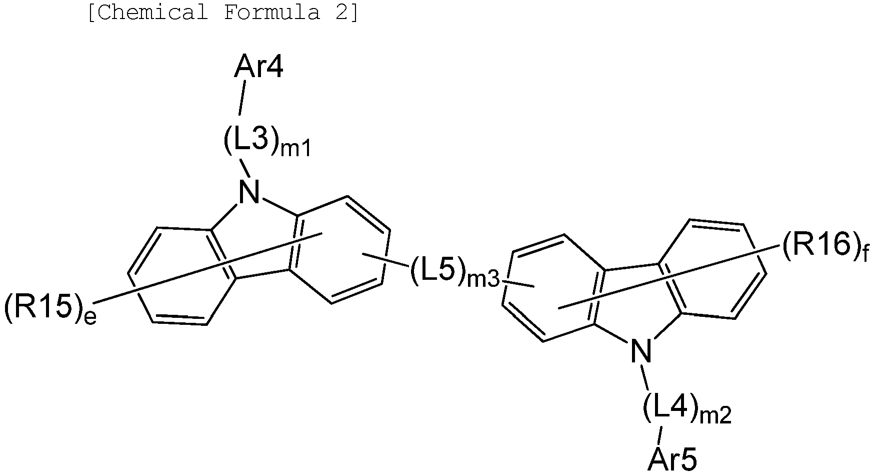 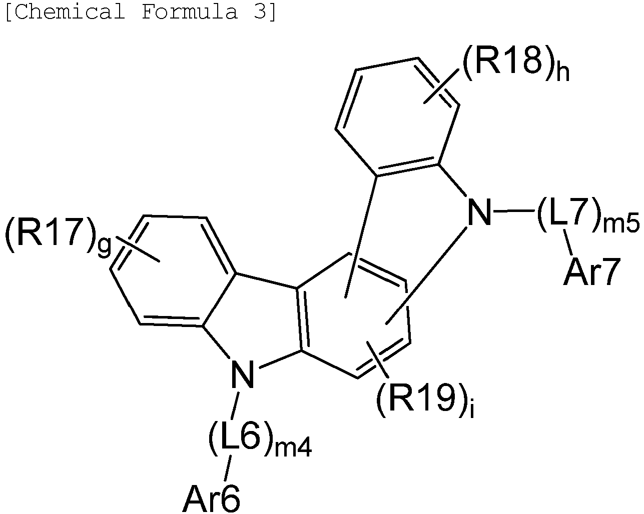 in Chemical Formula 2 and Chemical Formula 3,
L3 to L7 are the same as or different from each other, and each independently a single bond; a substituted or unsubstituted C6 to C60 arylene group; or a substituted or unsubstituted C2 to C60 heteroarylene group;
Ar4 to Ar7 are the same as or different from each other, and each independently a substituted or unsubstituted C6 to C60 aryl group; or a substituted or unsubstituted C2 to C60 heteroaryl group;
R15 to R19 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
m1 to m5 are the same as or different from each other, and each independently an integer of 0 to 3;
e and f are the same as or different from each other, and each independently an integer of 0 to 7;
g and h are the same as or different from each other, and each independently an integer of 0 to 4;
i is an integer of 0 to 2; and
when m1 to m5, e, f, g, h, and i are 2 or greater, L3s to L7s and R15s to R19s are the same as or different from each other.

11. The organic light emitting device of claim 10, wherein Ar4 to Ar7 are represented by any one of the following Chemical Formula 2-1 and Chemical Formula 2-2: 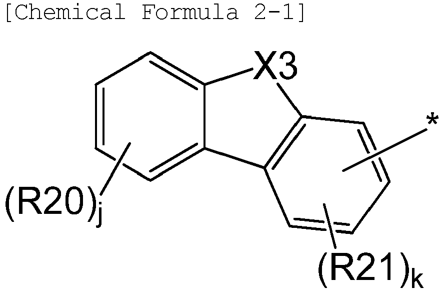
[Chemical Formula 2-2] Ar8-*
in Chemical Formula 2-1 and Chemical Formula 2-2,
X3 is O; S; or CRaRb;
Ar8 is a substituted or unsubstituted C6 to C60 aryl group,
R20, R21, Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
* is a site linked to Chemical Formula 2 or Chemical Formula 3;
j is an integer of 0 to 4;
k is an integer of 0 to 3; and
when j and k are 2 or greater, R20s and R21s are the same as or different from each other.

12. The organic light emitting device of claim 10, wherein the heterocyclic compound represented by Chemical Formula 2 or Chemical Formula 3 does not include deuterium as a substituent, or has a deuterium content of 1% to 100% based on a total number of hydrogen atoms and deuterium atoms.

13. The organic light emitting device of claim 10, wherein the heterocyclic compound represented by Chemical Formula 2 is represented by any one of the following compounds:

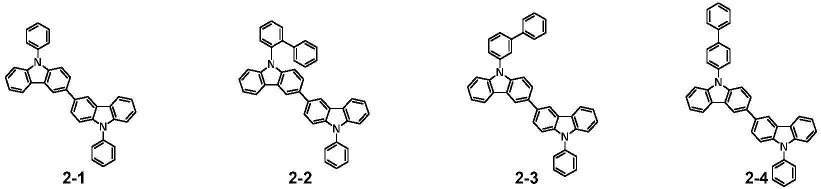

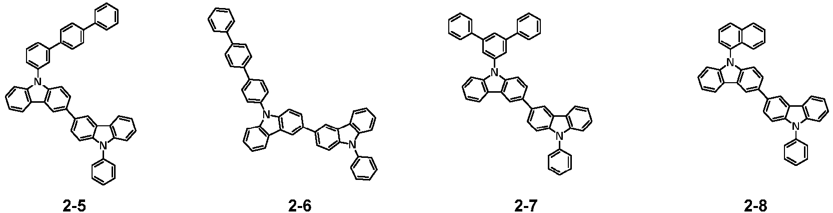

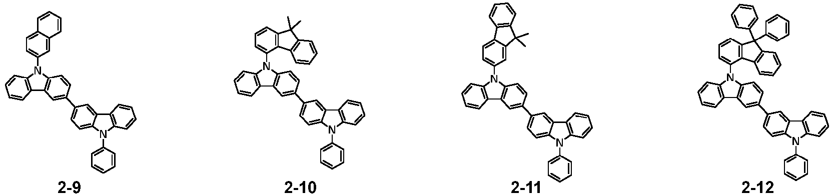

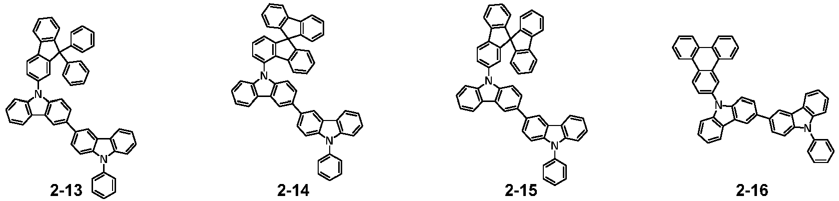

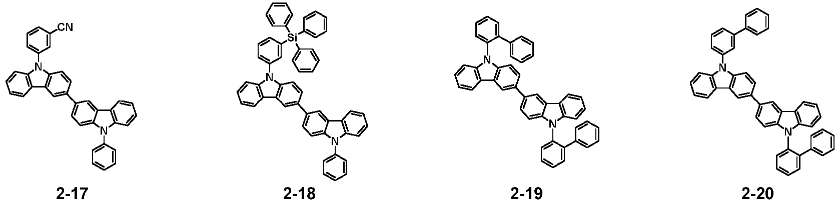

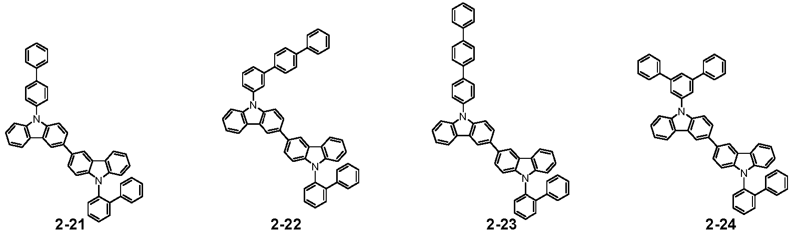

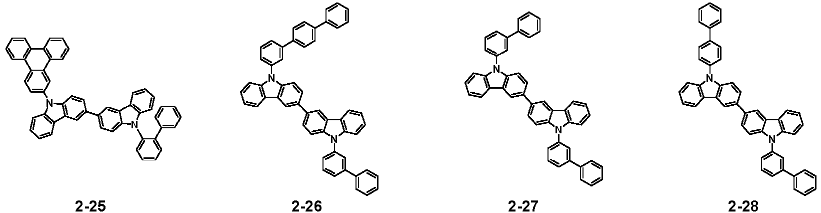

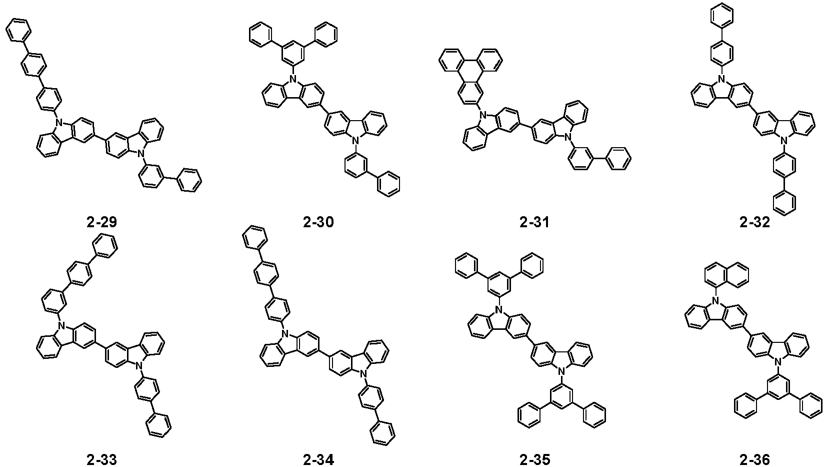

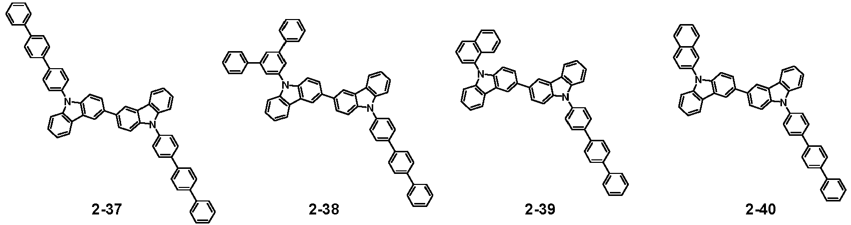

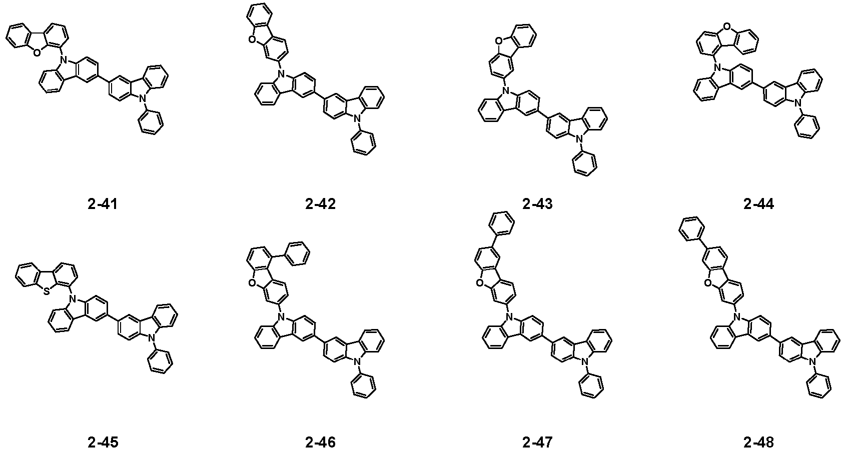

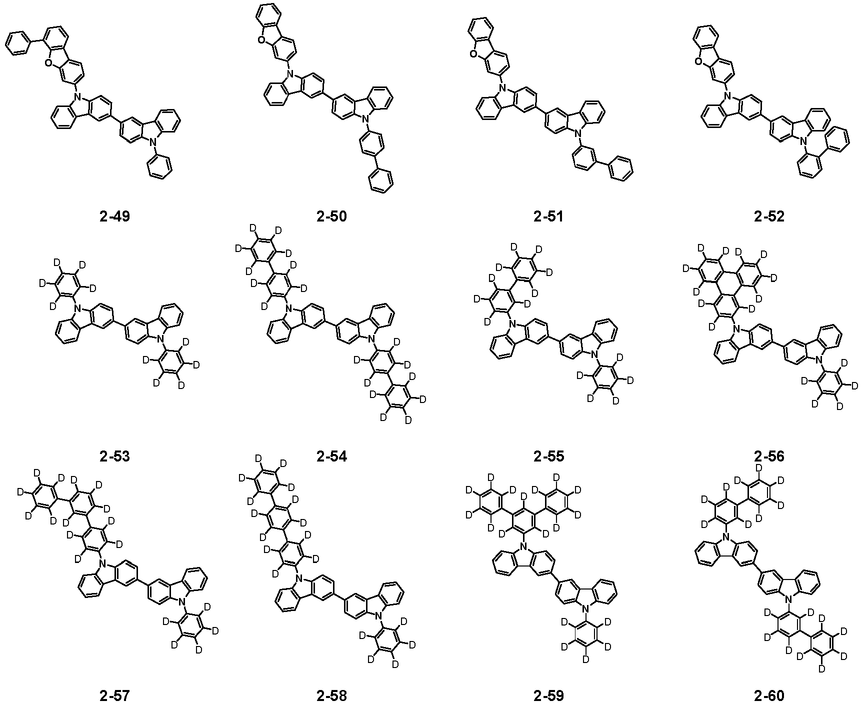

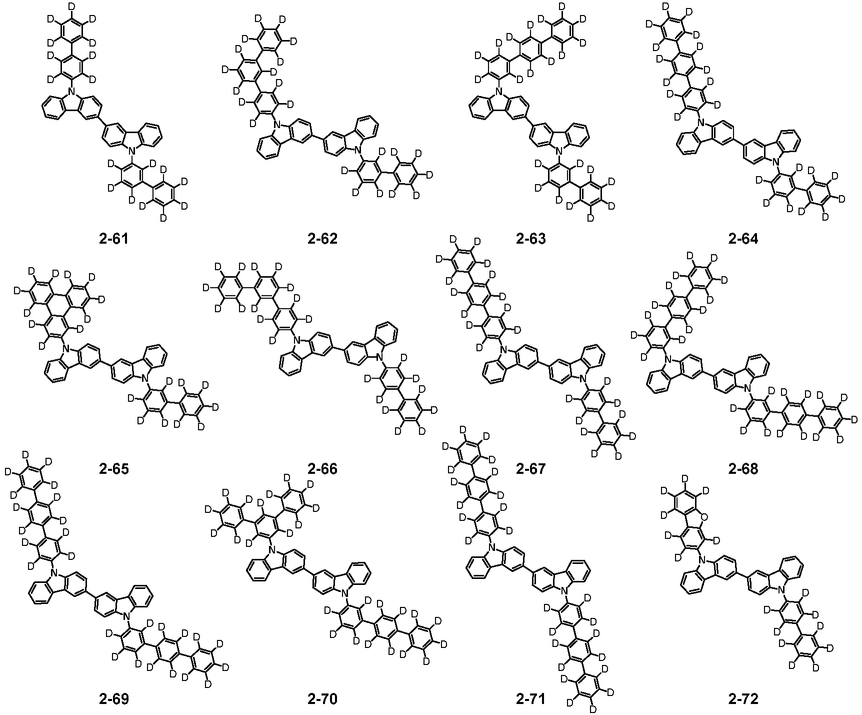

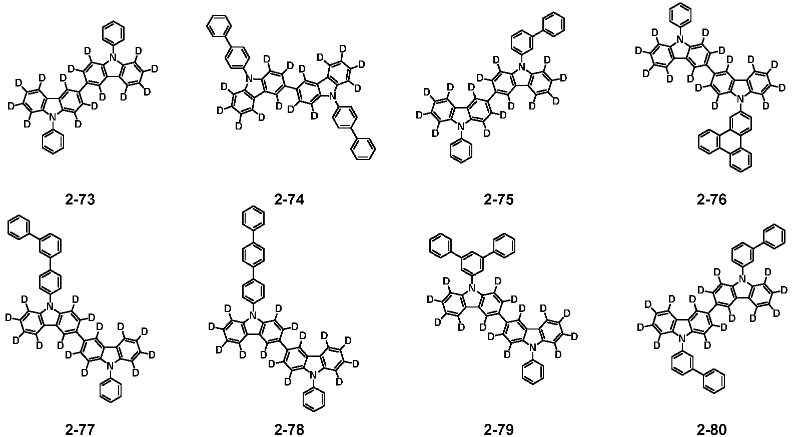

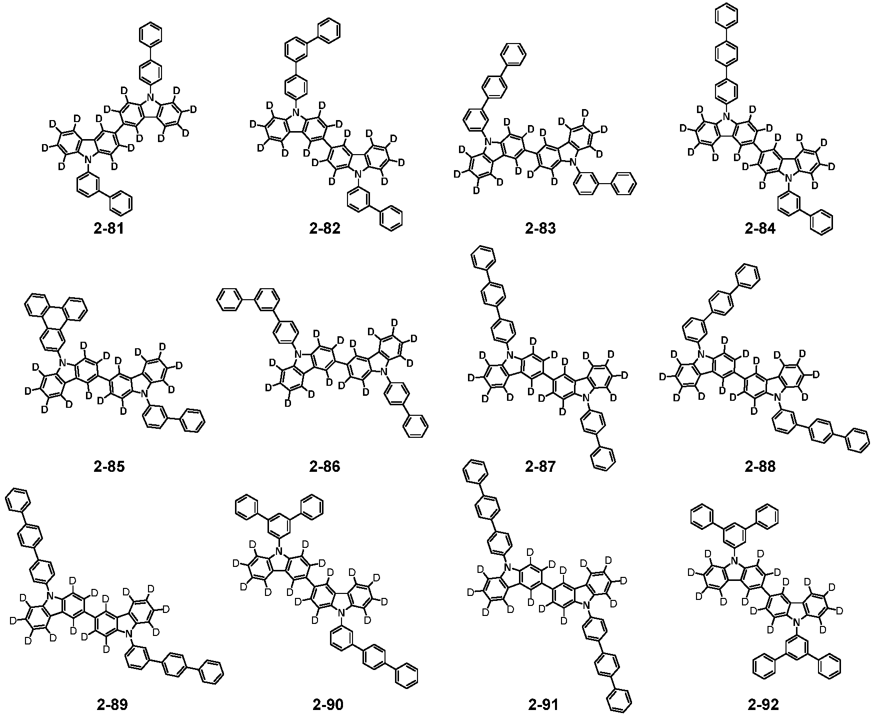

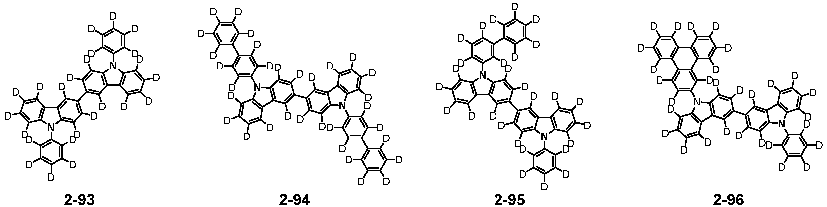

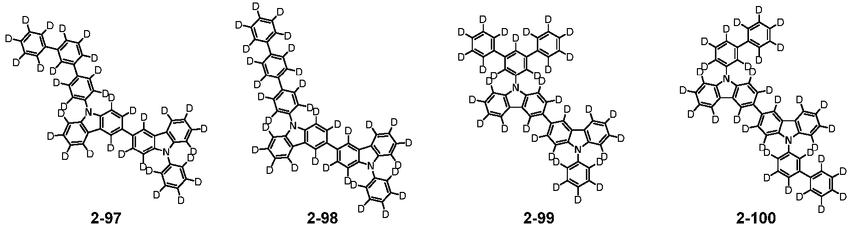

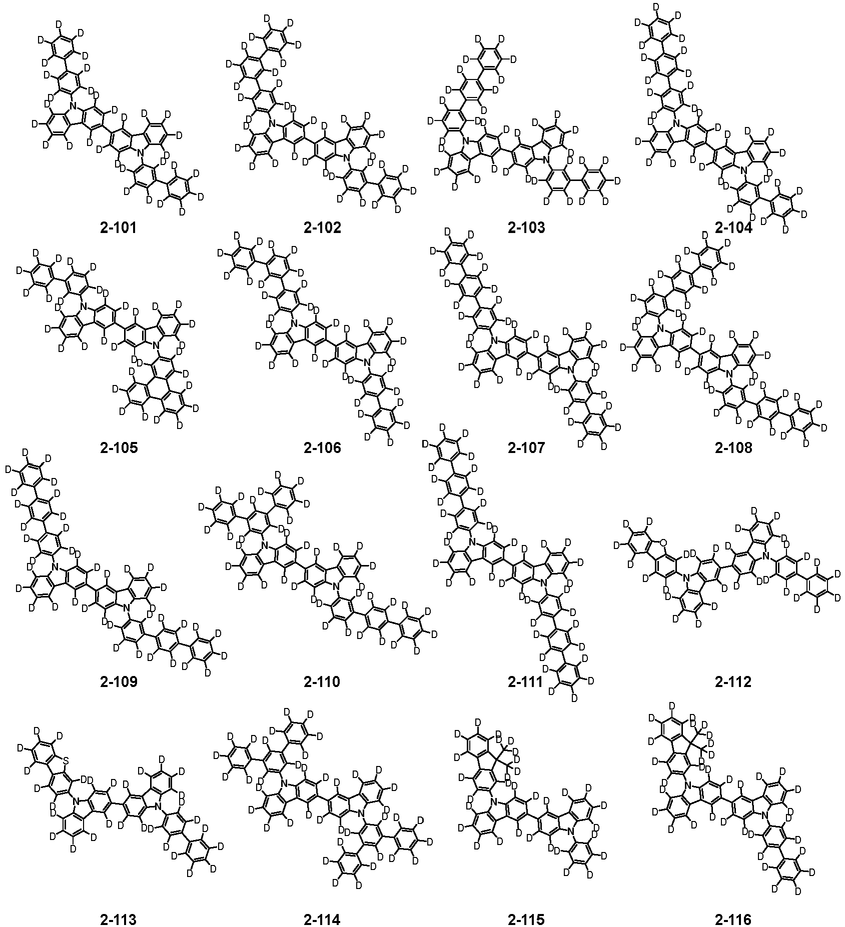

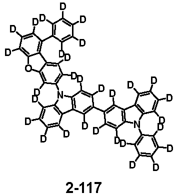

14. The organic light emitting device of claim 10, wherein the heterocyclic compound represented by Chemical Formula 3 is represented by any one of the following compounds:

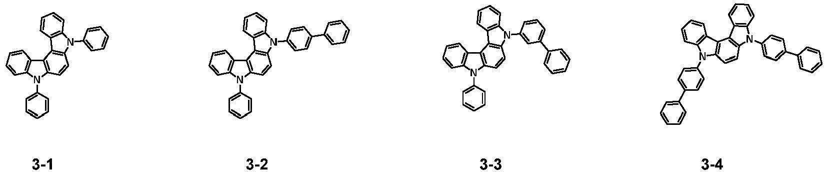

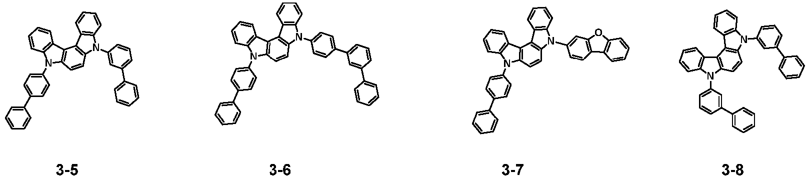

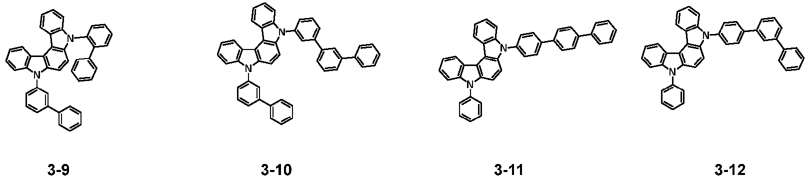

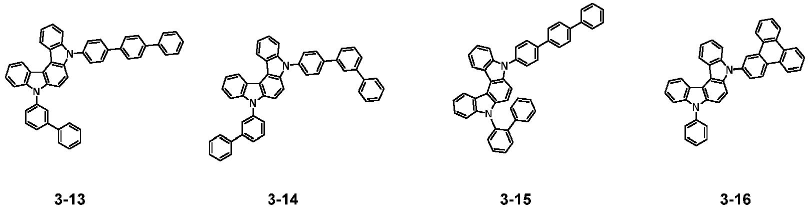

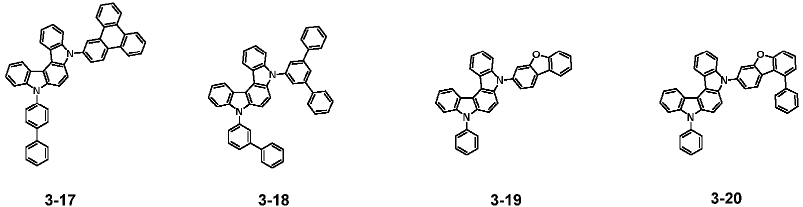

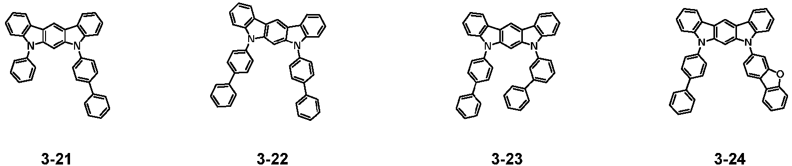

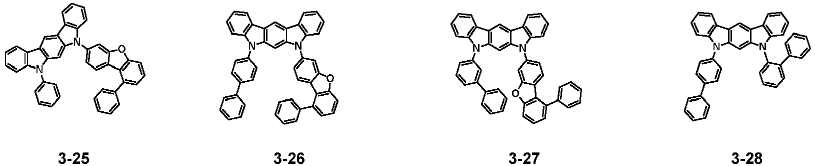

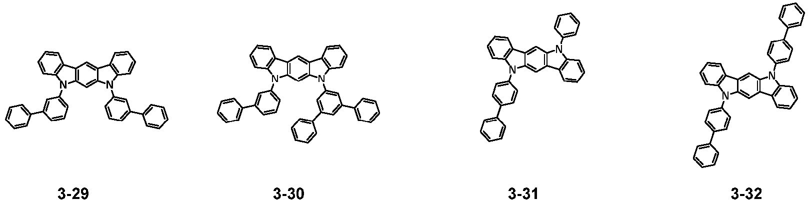

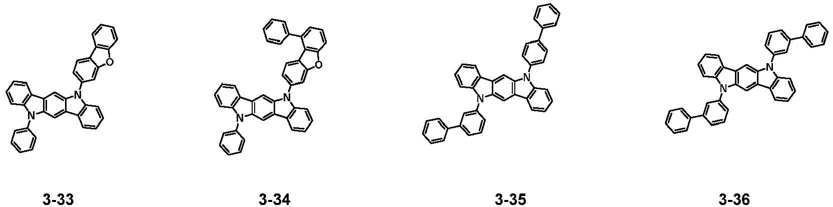

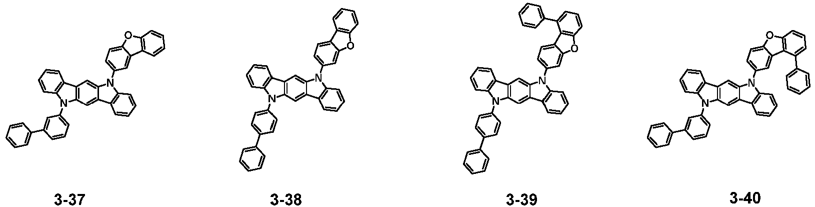

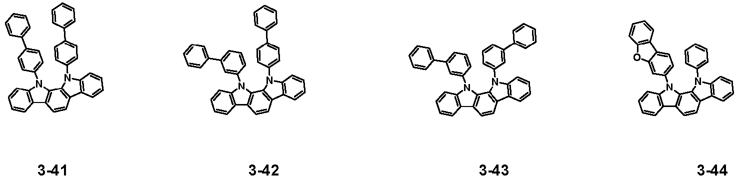

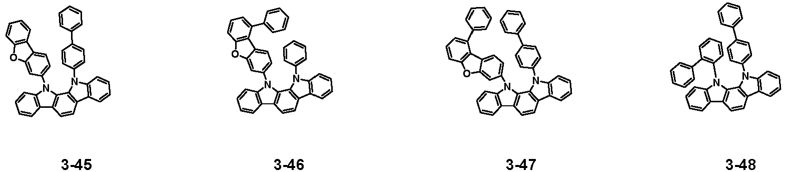

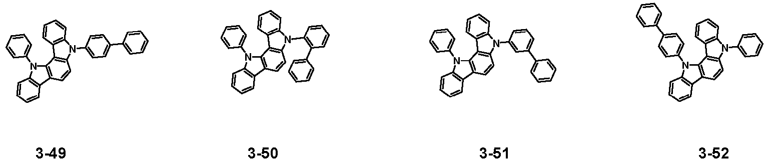

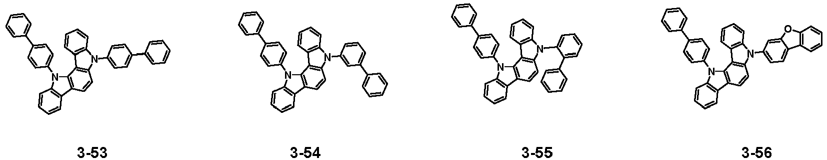

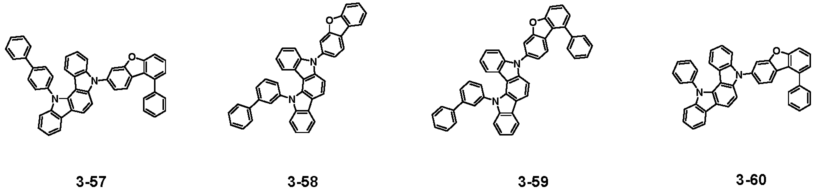

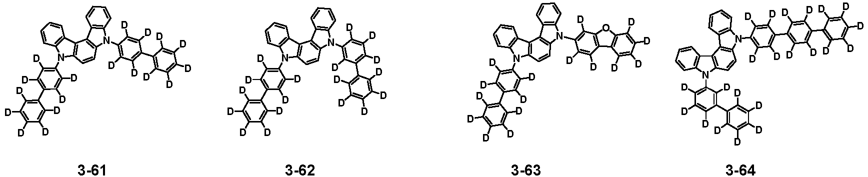

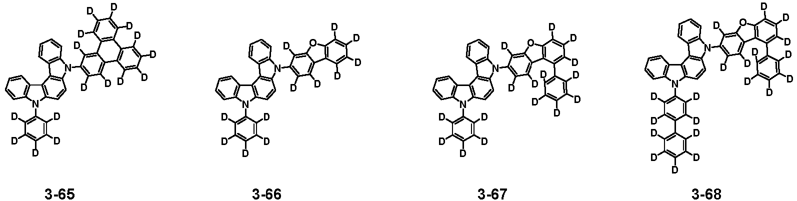

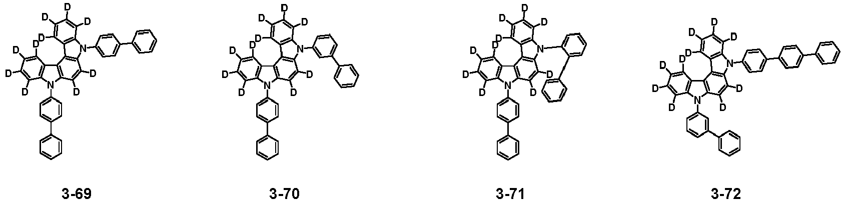

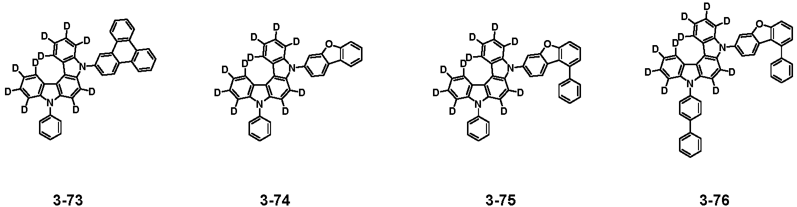

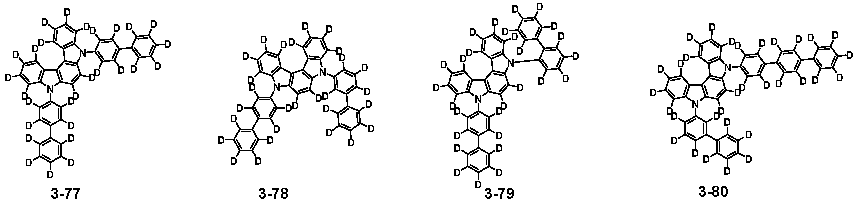

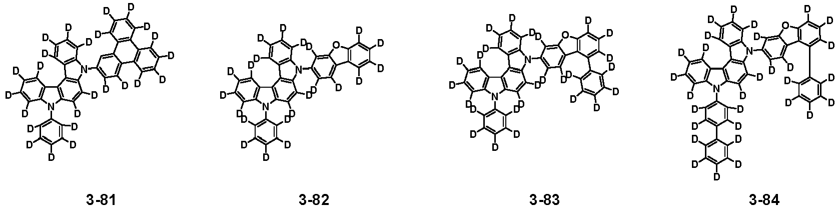

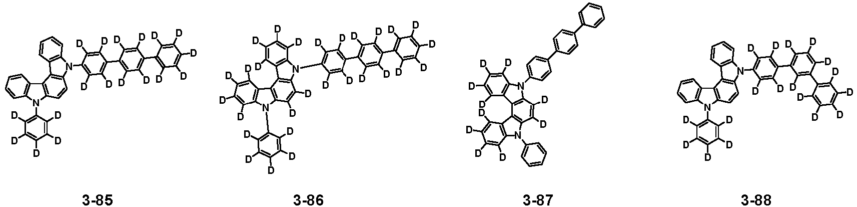

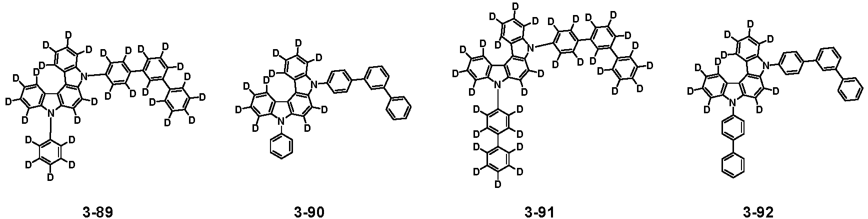

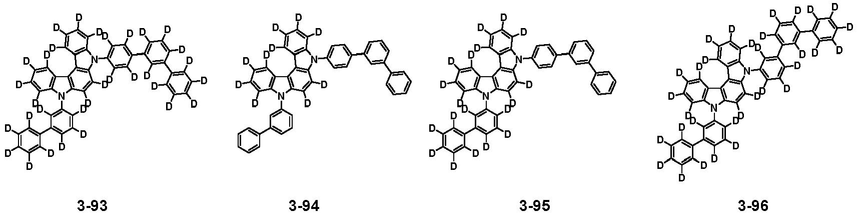

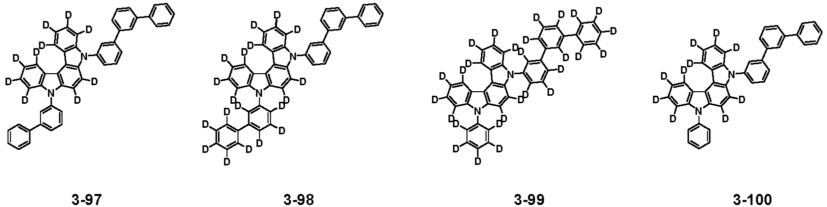

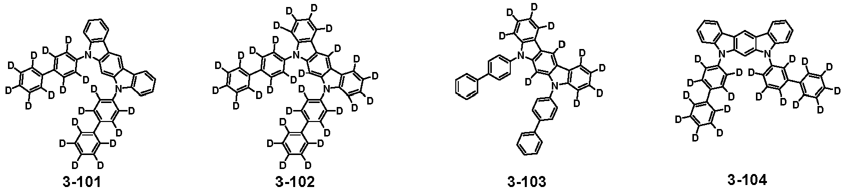

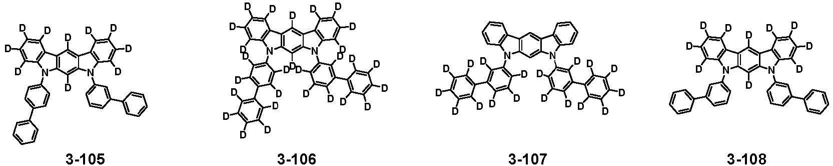

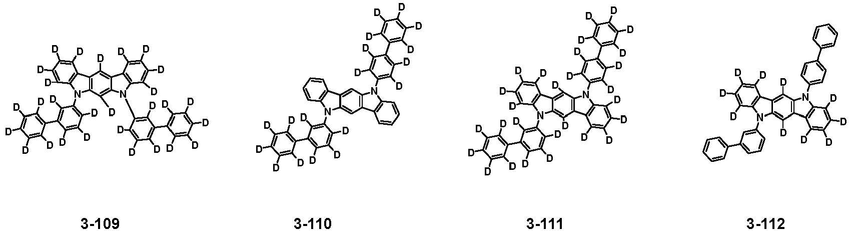

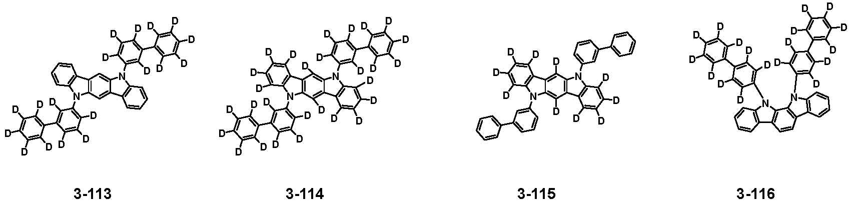

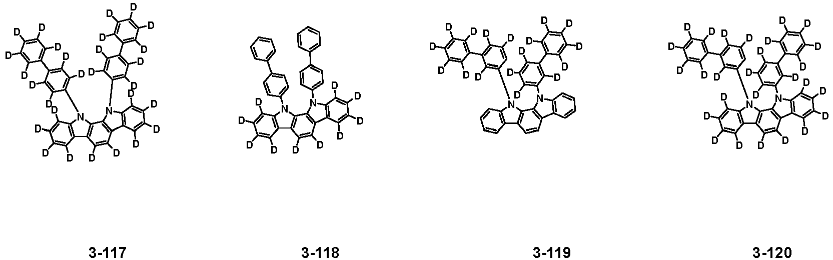

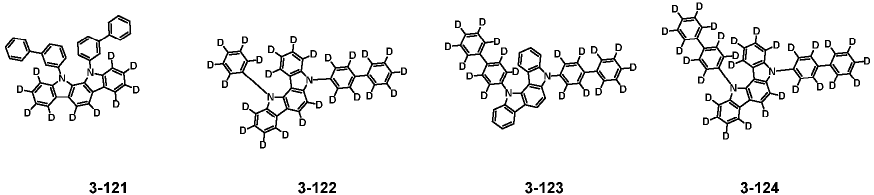

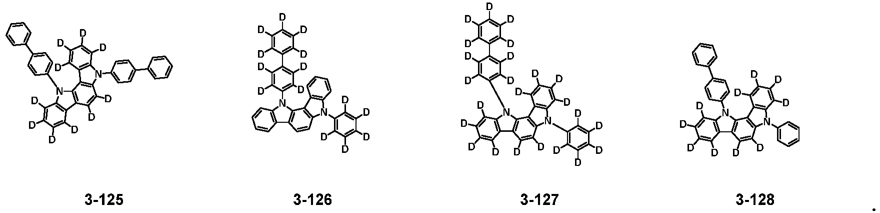

15. The organic light emitting device of claim 7, further comprising one, or two or more layers selected from the group consisting of a light emitting layer, a hole injection layer, a hole transport layer, a hole transport auxiliary layer, an electron injection layer, an electron transport layer, an electron blocking layer and a hole blocking layer.

16. A composition for an organic material layer, the composition comprising:
the heterocyclic compound of any one of claims 1 to 6; and
a heterocyclic compound represented by the following Chemical Formula 2 or Chemical Formula 3: 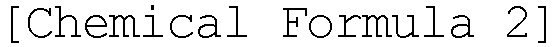 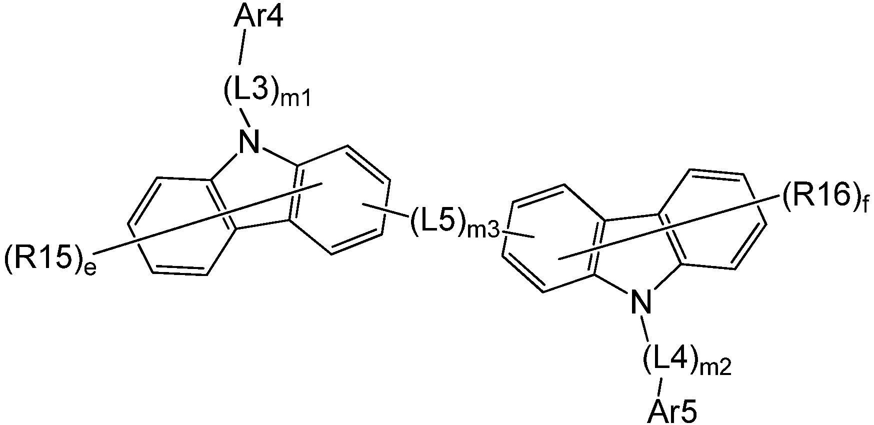 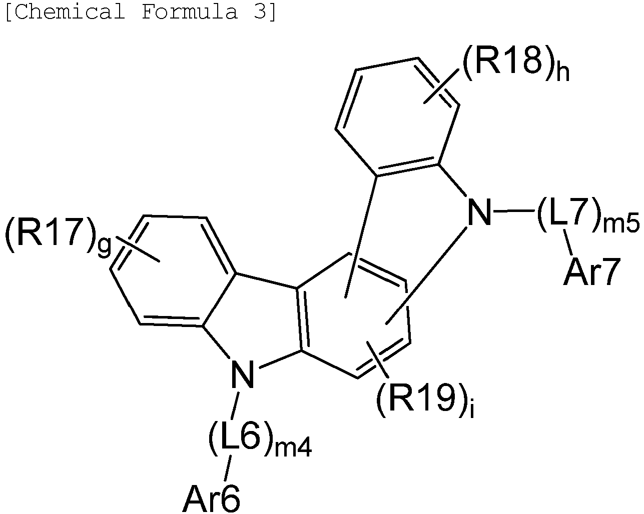 wherein, in Chemical Formula 2 and Chemical Formula 3,
L3 to L7 are the same as or different from each other, and each independently a single bond; a substituted or unsubstituted C6 to C60 arylene group; or a substituted or unsubstituted C2 to C60 heteroarylene group;
Ar4 to Ar7 are the same as or different from each other, and each independently a substituted or unsubstituted C6 to C60 aryl group; or a substituted or unsubstituted C2 to C60 heteroaryl group;
R15 to R19 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; and -SiR101R102R103, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
m1 to m5 are the same as or different from each other, and each independently an integer of 0 to 3;
e and f are the same as or different from each other, and each independently an integer of 0 to 7;
g and h are the same as or different from each other, and each independently an integer of 0 to 4;
i is an integer of 0 to 2; and
when m1 to m5, e, f, g, h, and i are 2 or greater, L3s to L7s and R15s to R19s are the same as or different from each other.

17. The composition of claim 16, wherein the heterocyclic compound represented by Chemical Formula 1 and the heterocyclic compound represented by Chemical Formula 2 or Chemical Formula 3 in the composition have a weight ratio of 1:10 to 10:1.
